(19) Europäisches Patentamt
European Patent Office
Office européen des brevets

(11) Publication number: **0 503 798 A1**

(12) # EUROPEAN PATENT APPLICATION

(21) Application number: **92301597.8**

(22) Date of filing: **26.02.92**

(51) Int. Cl.5: **C07D 263/46**, C07D 263/48, A01N 43/76, C07F 9/653, C07D 263/52, C07D 498/10, C07D 403/04, C07D 403/12

(30) Priority: **15.03.91 US 670539**

(43) Date of publication of application: **16.09.92 Bulletin 92/38**

(84) Designated Contracting States: **PT**

(71) Applicant: **E.I. DU PONT DE NEMOURS AND COMPANY**
**1007 Market Street**
**Wilmington Delaware 19898(US)**

(72) Inventor: **Sternberg, Jeffrey Arthur**
**27 Wenark, Apt. 10**
**Newark, Delaware 19713(US)**
Inventor: **Adams, John Benjamin**
**759 Norris Road**
**Hockessin, Delaware 19707(US)**

(74) Representative: **Hildyard, Edward Martin et al**
**Frank B. Dehn & Co. European Patent Attorneys Imperial House 15-19 Kingsway**
**London WC2B 6UZ(GB)**

(54) **Fungicidal 4-thixooxazolidin-2-ones and 4-iminooxazolindin-2-ones.**

(57) This invention pertains to compounds of Formula I including all geometric and stereoisomers, agriculturally suitable salts thereof, agricultural compositions containing them and their use as fungicides.

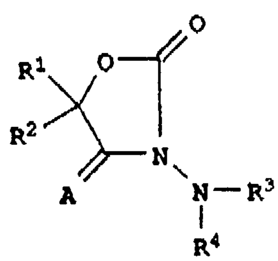

I

EP 0 503 798 A1

CROSS REFERENCE TO RELATED APPLICATION

This is a continuation-in-part of application U.S.S.N 07/670,539, filed March 15, 1991.

BACKGROUND OF THE INVENTION

New fungicides for controlling fungus growth on vegetation are in constant demand. In the most common situation, such compounds are sought to selectively control fungus growth on such useful crops as cotton, rice, corn, wheat and soybeans. Unchecked fungus growth in such crops can cause significant losses, reducing profit to the grower and increasing costs to the consumer.

A number of fungicides have been developed and employed. For example, WO 90/12791 pertains to the use of compounds of Formula i as fungicides, certain novel compounds of Formula i, compositions containing those compounds, and processes for preparing them:

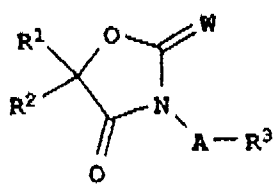

i

wherein:

A is O and $NR^4$; and

W is O and S.

The compounds of this invention differ from those of the current invention in the nature of $R^2$ and the lack of the A = S or N-J moiety shown in Formula I below.

The above reference does not specifically disclose the compounds of the instant invention or their particular fungicidal utility.

The compounds of the prior art, although useful as fungicides, tend to have drawbacks including poor efficacy, toxicity to non-target organisms and/or unacceptable environmental affects.

SUMMARY OF THE INVENTION

This invention pertains to compounds of Formula I including all geometric and stereoisomers, agriculturally suitable salts thereof, agricultural compositions containing them and their use as fungicides:

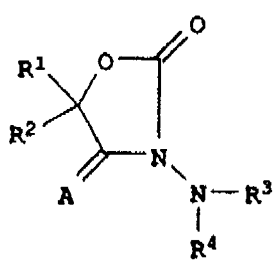

I

wherein:

| | |
|---|---|
| A | is S or N-J; |
| J | is $R^{15}$; $C(=O)R^{16}$; $C(=O)OR^{17}$; $C(=O)SR^{18}$; $C(=O)NR^{19}R^{20}$; P-$(=O)(C_1\text{-}C_4\ alkyl)_2$; or OG; |
| G | is H; $C_1$-$C_6$ alkyl; benzyl optionally substituted with $R^{34}$ on the phenyl ring; C(=O) ($C_1$-$C_4$ alkyl); C(=O)($C_1$-$C_4$ alkoxy); or C(=O)-$NHR^{36}$; |
| $R^1$ | in $C_1$-$C_4$ alkyl; $C_1$-$C_4$ haloalkyl) $C_3$-$C_6$ cycloalkyl; $C_2$-$C_4$ alkenyl; |

$C_2$-$C_4$ alkoxycarbonyl; or phenylmethyl optionally substituted with $R^6$ on the phenyl ring and with $R^8$ on the benzylic carbon;

$R^2$ is $C_1$-$C_{20}$ alkyl optionally substituted with $R^{22}$; $C_2$-$C_{20}$ alkoxyalkyl optionally substituted with $R^{35}$; $C_2$-$C_{20}$ alkenyl optionally substituted with $R^{42}$; $C_2$-$C_{20}$ alkynyl optionally substituted with $R^{41}$; $(CH_2CH_2OCH_2CH_2)CH$-; $(CH_2CH_2SCH_2CH_2)CH$-; $(CH_2CH_2SO_2CH_2CH_2)CH$-; $C_5$-$C_7$ cycloalkyl; $C_5$-$C_7$ cycloalkenyl; phenyl optionally substituted with $R^5$ and $R^7$; 2-naphthalenyl; thienyl optionally substituted with $R^5$ and $R^7$; furyl optionally substituted with $R^7$; or pyridyl optionally substituted with $R^5$ and $R^7$; or

$R^1$ and $R^2$ can be taken together to form structures selected from the group consisting of $-CH_2(CH_2)_2CH_2-$, $-CH_2(CH_2)_3CH_2-$, $-CH_2(CH_2)_4CH_2-$, $-CH_2CH_2OCH_2CH_2-$, $-CH_2CH_2SCH_2CH_2-$,

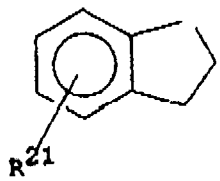

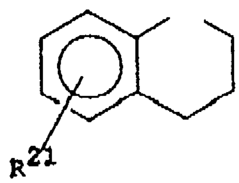

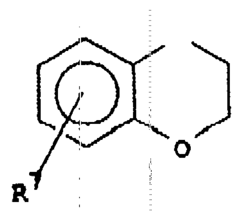

$R^3$ is phenyl, pyridyl, or pyrimidinyl each optionally substituted with $R^{10}$; or phenylmethyl;

$R^4$ is H or methyl;

$R^5$ is halogen; nitro; cyano; $C_1$-$C_6$ alkyl; $C_5$-$C_6$ cycloalkyl; $C_1$-$C_6$ haloalkyl; $C_1$-$C_6$ alkylthio; $C_1$-$C_6$ haloalkylthio; $C_1$-$C_6$ alkoxy; $C_1$-$C_6$ haloalkoxy; $C_5$-$C_6$ cycloalkyloxy; $C_2$-$C_6$ alkoxyalkyl; $C_2$-$C_6$ alxoxyalkoxy; $C_3$-$C_6$ alkenyl; $C_3$-$C_6$ haloalkenyl; $C_3$-$C_6$ alkenyloxy; $C_3$-$C_6$ alkynyl; $C_3$-$C_6$ haloalkynyl; $C_3$-$C_6$ alkynyloxy; $C_1$-$C_6$ alkylsulfonyl; $C_1$-$C_6$ haloalkylsulfonyl; phenyl or phenylthio each optionally substituted with $R^{24}$; phenylmethyl, phenoxymethyl, phenethyl, or styryl each optionally substituted with $R^{24}$ on the phenyl ring; phenoxy optionally substituted with $R^{27}$; benzyloxy optionally substituted with $R^{30}$ on the phenyl ring; $-OC(=O)NHR^{28}$, $-C(=O)-OR^{28}$; or $-OC(=O)R^{28}$;

$R^6$, $R^7$, $R^{12}$, $R^{13}$, $R^{24}$, $R^{26}$ and $R^{34}$ are independently 1-2 halogen; nitro; $C_1$-$C_4$ alkyl; trifluoromethyl; methylthio; or $C_1$-$C_4$ alkoxy;

$R^8$, $R^{14}$, $R^{20}$, $R^{40}$ and $R^{38}$ are independently H or $C_1$-$C_4$ alkyl;

$R^9$ is $C_1$-$C_{18}$ alkyl; or phenyl optionally substituted with $R^7$;

$R^{10}$, $R^{25}$ and $R^{33}$ are independently 1-2 substituents selected from the group consisting of halogen, nitro, cyano, $C_1$-$C_4$ alkyl, trifluoromethyl, $C_1$-$C_4$ alkylthio, $C_1$-$C_4$ alkoxy and trifluoromethoxy;

$R^{11}$ and $R^{36}$ are independently $C_1$-$C_6$ alkyl; or phenyl optionally substituted with $R^{12}$;

$R^{15}$ is H; $C_1$-$C_8$ alkyl optionally substituted with $C_1$-$C_2$ alkoxy; $C_3$-$C_6$ cycloalkyl; $C_3$-$C_8$ alkenyl; $C_3$-$C_8$ alkynyl; phenyl optionally substituted with $R^{13}$; benzyl optionally substituted with $R^{13}$ on the phenyl ring and with $R^{20}$ on the benzylic carbon; or pyridyl optionally substituted with $R^{13}$;

$R^{16}$ is H; $C_1$-$C_{17}$ alkyl optionally substituted with $R^{31}$; $C_2$-$C_{17}$ alkenyl optionally substituted with $R^{32}$; $C_2$-$C_7$ alkynyl; $C_3$-$C_8$ cycloalkyl; $C_5$-$C_6$ cycloalkenyl; $C_6$-$C_7$ alkylcyoloalkyl; $C_4$-$C_8$ cycloalkylalkyl; phenyl optionally substituted with $R^{33}$; naphthalenyl, furanyl, thienyl, benzoyl, or pyridyl each optionally substituted with $R^{34}$; or $C_2$-$C_5$ alkoxycarbonyl;

$R^{17}$ and $R^{18}$ are independently $C_1$-$C_{18}$ alkyl optionally substituted with $R^{23}$; $C_2$-

$C_{10}$ alkenyl optionally substituted with $R^{32}$; $C_3$-$C_8$ alkynyl; $C_3$-$C_{12}$ cycloalkyl; $C_5$-$C_6$ cycloalkenyl; $C_6$-$C_7$ alkylcycloalkyl; $C_6$-$C_7$ cycloalxylalkyl; or phenyl, naphthalenyl, or thienyl each optionally substituted with $R^{34}$;

$R^{19}$ is H; $C_1$-$C_{10}$ alkyl; $C_5$-$C_6$ cycloalkyl; or phenyl optionally substituted with $R^{34}$; or

$R^{19}$ and $R^{20}$ can be taken together to form structures selected from the group consisting of $-CH_2(CH_2)_2CH_2-$, $-CH_2(CH_2)_3CH_2-$, $-CH_2(CH_2)_4CH_2-$, $-CH_2CH_2OCH_2CH_2-$, $-CH_2CH(Me)CH_2CH(Me)CH_2-$, and $-CH_2CH(Me)OCH(Me)CH_2-$;

$R^{21}$ is 1-2 halogen; nitro; $C_1$-$C_4$ alkyl; trifluoromethyl; methylthio; or $C_1$-$C_4$ alkoxy; or phenoxy optionally substituted with $R^{26}$;

$R^{22}$ is cyano; nitro; $C_1$-$C_{19}$ alkylthio; $C_1$-$C_{19}$ alkylsulfinyl; $C_1$-$C_{19}$ haloalkoxy; $C_5$-$C_6$ cycloalkyloxy; $C_3$-$C_{19}$ alkenyloxy; $C_3$-$C_{19}$ alkynyloxy; $C_1$-$C_{19}$ alkylsulfonyl; $C_2$-$C_{19}$ alkoxycarbonyl; hydroxyl; hydroxycarbonyl; $R^{28}C(=O)O$; $R^{28}OC(=O)O$; $R^{28}R^{40}NC(=O)O$; $R^{39}R^{40}N$; $(C_1$-$C_4$ alkoxy$)_2P(=E)O$; $R^{11}SO_3$; $R^{40}R^{14}R^{38}N^+$;phenyl, phenylthio, phenoxy, phenylsulfonyl, phenylsulfinyl, pyridyl or pyridyloxy each optionally substituted with $R^{30}$; thienyl, pyrimidinyl, furanyl, naphthalenyl, pyrimidinyloxy, naphthalenyloxy each optionally substituted with $R^7$; tetrahydropyranyl; $C_3$-$C_6$ cycloalkyl; 2-tetrahydropyranyloxy; or $C(=Q)R^{40}$;

E is O or S;

Q is O or N-T-W;

T is O; $NR^{37}$; or a direct bond;

W is H; $C_1$-$C_8$ alkyl, $C_3$-$C_8$ alkenyl; phenylmethyl optionally substituted with $R^7$ on the phenyl ring and $R^{14}$ on the benzylic carbon; phenyl or pyridyl each optionally substituted with $R^7$; $C(=O)R^{28}$; $C(=O)OR^{28}$; or $C(=O)NR^{28}R^{14}$

$R^{23}$ is 1-3 halogen; $C_1$-$C_{12}$ alkoxy; $C_1$-$C_{12}$ alkylthio; phenyl or naphthalenyl each optionally substituted with $R^{34}$; or phenoxymethyl optionally substituted with $R^{34}$ on the phenyl ring;

$R^{27}$ is 1-2 halogen; nitro; cyano; $C_1$-$C_6$ alkyl; $C_1$-$C_6$ haloalkyl; $C_1$-$C_6$ alkoxy; $C_1$-$C_6$ haloalkoxy; $C_1$-$C_4$ alkylsulfonyl; $C_2$-$C_6$ alkoxyalkyl; $C_1$-$C_4$ alkylthio; $C_5$-$C_6$ cycloalkyl; $C_5$-$C_6$ cycloalkyloxy; $C_2$-$C_6$ alkenyl; $C_2$-$C_6$ haloalkenyl; $C_2$-$C_6$ alkynyl; hydroxycarbonyl; $C_2$-$C_4$ alkoxycarbonyl; or phenoxy optionally substituted with $R^{24}$;

$R^{28}$ is $C_1$-$C_8$ alkyl; or phenyl or pyridyl each optionally substituted with $R^{30}$;

$R^{30}$ is 1-2 substituents selected from the group consisting of halogen, nitro, cyano, $C_1$-$C_4$ alkyl, trifluoromethyl, $C_1$-$C_4$ alkoxy and trifluoromethoxy; or phenoxy optionally substituted with $R^{26}$;

$R^{31}$ is 1-3 halogen; $C_1$-$C_{18}$ alkoxy; allyloxy; $C_1$-$C_{18}$ alkylthio; phenyl, phenoxy, benzyloxy, or phenylthio each optionally substituted with $R^{34}$ on the phenyl ring; acetyl; or $C_2$-$C_5$ alkoxycarbonyl;

$R^{32}$ is 1-3 halogen; or $C_1$-$C_4$ alkoxy;

$R^{35}$ is cyano; nitro; $C_1$-$C_{17}$ alkylthio; $C_1$-$C_{17}$ alkylsulfinyl; $C_1$-$C_{17}$ haloalkoxy; $C_5$-$C_6$ cycloalkyloxy; $C_2$-$C_{17}$ haloalkenyl; $C_3$-$C_{17}$ alkenyloxy; $C_3$-$C_{17}$ haloalkynyl; $C_3$-$C_{17}$ alkynyloxy; $C_1$-$C_{17}$ alkylsulfonyl; $C_2$-$C_{17}$ alkoxycarbonyl; hydroxyl; hydroxycarbonyl; $R^{28}C(=O)O$; $R^{28}OC(=O)O$; $R^{28}R^{40}NC(=O)O$; $R^{40}R^{39}N$; $(C_1$-$C_4$ alkoxy$)_2P(=E)O$; $R^{11}SO_3$; $R^{40}R^{14}R^{38}N^+$;phenyl, phenoxy, phenylthio, phenylsulfonyl, phenylsulfinyl, pyridyl or pyridyloxy each optionally substituted with $R^{30}$; thienyl, pyrimidinyl, furanyl, naphthalenyl, pyrimidinyloxy, naphthalenyloxy each optionally substituted with $R^7$; tetrahydropyranyl; 2-tetrahydropyranyloxy; $C_1$-$C_{17}$ alkoxy; $C_2$-$C_{17}$ alkoxyalkoxy; $C_3$-$C_{17}$ alkynyl; $C_3$–$C_6$ cycloalkyl; or $C_2$-$C_{17}$

haloalkoxyalkoxy;

$R^{37}$ is H; $C_1$-$C_6$ alkyl; or phenyl optionally substituted with $R^7$;

$R^{39}$ is $C_1$-$C_{19}$ alkyl; $C_2$-$C_{19}$ alkylcarbonyl; $C_2$-$C_{19}$ alkoxycarbonyl; $(R^9R^{40}N)C=O$; phenyl optionally substituted with $R^{25}$; or phenoxycarbonyl optionally substituted with $R^7$;

$R^{41}$ is cyano; nitro; $C_1$-$C_{17}$ alkylthio; $C_1$-$C_{17}$ alkylsulfinyl; $C_1$-$C_{17}$ haloalkoxy; $C_5$-$C_6$ cycloalkyloxy; $C_3$-$C_{17}$ alkenyloxy; $C_3$-$C_{17}$ alkynyloxy; $C_1$-$C_{17}$ alkylsulfonyl; $C_2$-$C_{17}$ alkoxycarbonyl; hydroxyl; hydroxycarbonyl; $R^{28}C(=O)O$; $R^{28}OC(=O)O$; $R^{28}R^{40}NC(=O)O$; $R^{40}R^{39}N$; $(C_1$-$C_4$ alkoxy$)_2P(=E)O$; $R^{11}SO_3$; $R^{40}R^{14}R^{38}N^+$;phenyl, phenoxy, phenylsulfonyl, phenylsulfinyl, pyridyl or pyridyloxy each optionally substituted with $R^{30}$; thienyl, pyrimidinyl, furanyl, naphthalenyl, pyrimidinyloxy, naphthalanyloxy each optionally substituted with $R^7$; tetrahydropyranyl; 2-tetrahydropyranyloxy; $C_1$-$C_{17}$ alkoxy; 1-3 halogen; $C_2$-$C_{17}$ alkoxyalkoxy; or $C_3$-$C_6$ cycloalkyl;

$R^{42}$ is cyano; nitro; $C_1$-$C_{17}$ alkylthio; $C_1$-$C_{17}$ alkylsulfinyl; $C_1$-$C_{17}$ haloalkoxy; $C_5$-$C_6$ cycloalkyloxy; $C_3$-$C_{17}$ alkenyloxy; $C_3$-$C_{17}$ haloalkynyl; $C_3$-$C_{17}$ alkynyloxy; $C_1$-$C_{17}$ alkylsulfonyl; $C_2$-$C_{17}$ alkoxycarbonyl; hydroxyl; hydroxycarbonyl; $R^{28}C(=O)O$; $R^{28}OC(=O)O$; $R^{28}R^{40}NC(=O)O$; $R^{40}R^{39}N$; $(C_1$-$C_4$ alkoxy$)_2P(=E)O$; $R^{11}SO_3$; $R^{40}R^{14}R^{38}N^+$; phenyl, phenoxy, phenylthio, phenylsulfonyl, phenylsulfinyl, pyridyl or pyridyloxy each optionally substituted with $R^{30}$; thienyl, pyrimidinyl, furanyl, naphthalenyl, pyrimidinyloxy, naphthalenyloxy each optionally substituted with $R^7$; tetrahydropyranyl; 2-tetrahydropyranyloxy; $C_1$-$C_{17}$ alkoxy; 1-3 halogen; $C_2$-$C_{17}$ alkoxyalkoxy; $C_3$-$C_{17}$ alkynyl; or $C_3$-$C_6$ cycloalkyl;

provided that

i) when $R^2$ is a substituted phenyl or heterocyclic ring, then only H or F can be substituted on the carbon atom(s) of the phenyl or heterocyclic ring adjacent to the carbon bearing the oxazolidinone ring;

ii) when $R^3$ is a phenyl or heterocyclic ring disubstituted with two alkyl or alkoxy groups, or one alkyl and one alkoxy group, then at least one of the alkyl and alkoxy groups is methyl or methoxy; and

iii) the total number of carbons in $R^2$, $R^{16}$, $R^{17}$, and $R^{18}$ is each less than or equal to 20.

A further embodiment of the invention pertains to a fungicidally active compound of Formula I comprising

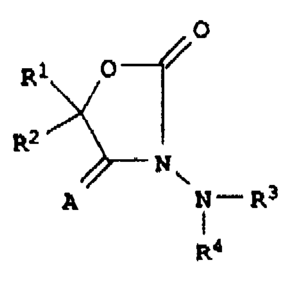

I

wherein:

A is S or N-OG;

G is H; $C_1$-$C_6$ alkyl; benzyl optionally substituted with $R^{34}$ on the phenyl ring; $C(=O)(C_1$-$C_4$ alkyl); $C(=O)(C_1$-$C_4$ alkoxy); or $C(=O)NHR^{36}$;

$R^1$ is $C_1$-$C_4$ alkyl; $C_1$-$C_4$ haloalkyl; $C_3$-$C_6$ cycloalkyl; $C_2$-$C_4$ alkenyl; $C_2$-$C_4$ alkoxycarbonyl; or phenylmethyl optionally substituted with $R^6$ on the phenyl ring and with $R^8$ on the benzylic carbon;

$R^2$ is $C_1$-$C_6$ alkyl; $C_5$-$C_7$ cycloalkyl; $C_2$-$C_6$ alkenyl; $C_5$-$C_7$ cycloalkenyl; $C_5$-$C_7$ cycloalkenyl; phenyl optionally substituted with $R^5$ and $R^7$; 2-naphthalenyl; thienyl optionally substituted with $R^5$ and $R^7$; furyl optionally substituted

with $R^7$; or pyridyl optionally substituted with $R^7$; or phenoxy or phenylthio each optionally substituted with $R^7$; or

$R^1$ and $R^2$ can be taken together to form structures selected from the group consisting of $-CH_2(CH_2)_2CH_2-$, $-CH_2(CH_2)_3CH_2-$, $-CH_2(CH_2)_4CH_2-$, $-CH_2CH_2OCH_2CH_2-$, $-CH_2CH_2SCH_2CH_2-$,

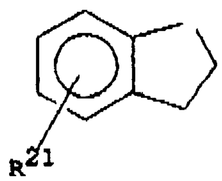 , 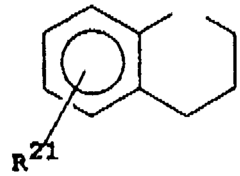 , and 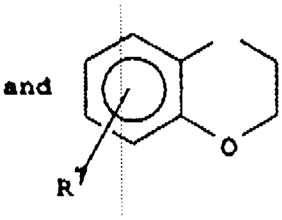 ;

$R^3$ is phenyl, pyridyl, or pyrimidinyl each optionally substituted with $R^{10}$; or phenylmethyl;

$R^4$ is H or methyl;

$R^5$ is halogen; nitro; cyano; $C_1$-$C_6$ alkyl; $C_5$-$C_6$ cycloalkyl; $C_1$-$C_6$ haloalkyl; $C_1$-$C_6$ alkylthio; $C_1$-$C_6$ haloalkylthio; $C_1$-$C_6$ alkoxy; $C_1$-$C_6$ haloalkoxy; $C_5$-$C_6$ cycloalkyloxy; $C_2$-$C_6$ alkoxyalkyl; $C_2$-$C_6$ alkoxyalkoxy; $C_3$-$C_6$ alkenyl; $C_3$-$C_6$ haloalkenyl; $C_3$-$C_6$ alkenyloxy; $C_3$-$C_6$ alkynyl; $C_3$-$C_6$ haloalkynyl; $C_3$-$C_6$ alkynyloxy, $C_1$-$C_6$ alkylsulfonyl; $C_1$-$C_6$ haloalkylsulfonyl; phenyl or phenylthio each optionally substituted with $R^{24}$; phenylmethyl, phenoxymethyl, phenethyl, or styryl each optionally substituted with $R^{24}$ on the phenyl ring; phenoxy optionally substituted with $R^{27}$; and $C_2$-$C_6$ alkoxycarbonyl;

$R^6$, $R^7$, $R^{24}$, $R^{26}$ and $R^{34}$ are independently 1-2 halogen; nitro; $C_1$-$C_4$ alkyl; trifluoromethyl; methylthio; or $C_1$-$C_4$ alkoxy;

$R^8$ is H or $C_1$-$C_4$ alkyl;

$R^{10}$ is 1-2 substituents selected from the group consisting of halogen, nitro, cyano, $C_1$-$C_4$ alkyl, trifluoromethyl, $C_1$-$C_4$ alkylthio, $C_1$-$C_4$ alkoxy and trifluoromethoxy;

$R^{21}$ is halogen; nitro; $C_1$-$C_4$ alkyl; trifluoromethyl; methylthio; or $C_1$-$C_4$ alkoxy; or phenoxy optionally substituted with $R^{26}$;

$R^{27}$ is 1-2 halogen; nitro; cyano; $C_1$-$C_6$ alkyl; $C_1$-$C_6$ haloalkyl; $C_1$-$C_6$ alkoxy; $C_1$-$C_6$ haloalkoxy; $C_1$-$C_4$ alkylsulfonyl; $C_2$-$C_6$ alkoxyalkyl; $C_1$-$C_4$ alkylthio; $C_5$-$C_6$ cycloalkyl; $C_5$-$C_6$ cycloalkyloxy; $C_2$-$C_6$ alkenyl; $C_2$-$C_6$ haloalkenyl; $C_2$-$C_6$ alkynyl; $C_2$-$C_4$ alkoxycarbonyl; or phenoxy; and

$R^{36}$ is $C_1$-$C_6$ alkyl; or phenyl optionally substituted with $R^{12}$.

A still further embodiment of the invention pertains to a fungicidally active composition comprising the compounds of Formula I.

Another embodiment of the invention pertains to a method of controlling disease in plants comprising applying to said plants a fungicidally active compound of Formula I.

## DETAILED DESCRIPTION OF THE INVENTION

In the above recitations, the term "alkyl", used either alone or in compound words such as "alkylthio" or "haloalkyl" denotes straight-chain or branched alkyl, e.g. methyl, ethyl, *n*-propyl, *i*-propyl, or the different butyl, pentyl or hexyl isomers.

"Alkenyl" denotes straight-chain or branched alkenes, e.g. 1-propenyl, 2-propenyl, 3-propenyl and the different butenyl, pentenyl and hexenyl isomers. "Alkenyl" also denotes polyenes such as 1,3-hexadiene and 2,4,6-heptatriene.

"Alkenyloxy" denotes straight-chain or branched alkenyloxy moieties. Examples of alkenyloxy include $H_2C=CHCH_2O$, $(CH_3)_2C=CHCH_2O$, $(CH_3)CH=CHCH_2O$, $(CH_3)CH=C(CH_3)CH_2O$ and $CH_2=CHCH_2CH_2O$.

"Alkynyl" denotes straight-chain or branched alkynes, e.g., ethynyl, 1-propynyl, 3-propynyl and the different butynyl, pentynyl and hexynyl isomers. "Alkynyl" can also denote moieties comprised of multiple triple bonds, e.g. 2,7-octadiyne and 2,5,8-decatriyne.

"Alkynyloxy" denotes straight-chain or branched alkynyloxy moieties. Examples include $HC\equiv CCH_2O$,

$CH_3C≡CCH_2O$ and $CH_3C≡CCH_2CH_2O$.

"Alkylthio" denotes branched or straight-chain alkylthio moieties, e.g. methylthio, ethylthio, and the different propylthio, butylthio, pentylthio and hexylthio isomers.

Examples of "alkylsulfonyl" include $CH_3SO_2$, $CH_3CH_2SO_2$, $CH_3CH_2CH_2SO_2$, $(CH_3)_2CHSO_2$ and the different butylsulfonyl, pentylsulfonyl and hexylsulfonyl isomers.

"Alkylsulfinyl" denotes both enantiomers of an alkylsulfinyl group. For example, $CH_3S(O)$, $CH_3CH_2S(O)$, $CH_3CH_2CH_2S(O)$, $(CH_3)_2CHS(O)$ and the different butylsulfinyl, pentylsulfinyl and hexylsufinyl isomers.

"Alkoxy" denotes, for example, methoxy, ethoxy, *n*-propyloxy, isopropyloxy and the different butoxy, pentoxy and hexyloxy isomers.

Examples of "alkoxyalkoxyalkoxy" include $CH_3OCH_2CH_2OCH_2O$, $CH_3CH_2OCH_2CH_2OCH_2O$, and $(CH_3)_2CHOCH_2CH_2OCH_2O$.

"Cycloalkyl" denotes, for example, cyclopropyl, cyclobutyl, cyclopentyl, and cyclohexyl. The term "cycloalkyloxy" denotes the same groups linked through an oxygen atom such as cyclopentyloxy and cyclohexyloxy. "Cycloalkenyl" denotes groups such as cyclopentenyl and cyclohexenyl.

Examples, of "cycloalkylalkyl" include cyclopropylmethyl, cyclohexylethyl, and other cycloalkyl moieties bonded to straight-chain or branched alkyl groups. "Alkylcycloalkyl" denotes alkyl substitution on a cycloalkyl moiety. Examples include 4-methylcyclohexyl and 3-isopropylcyclopentyl.

The term "halogen", either alone or in compound words such as "haloalkyl", denotes fluorine, chlorine, bromine or iodine. Further, when used in compound words such as "haloalkyl", said alkyl may be partially or fully substituted with halogen atoms which may be the same or different. Examples of "haloalkyl" indlude $F_3C$, $ClCH_2$, $CF_3CH_2$ and $CF_3CF_2$. Examples of "haloalkenyl" include $(Cl)_2C=CHCH_2$ and $CF_3CH_2CH=CHCH_2$. Examples of "haloalkynyl" include $HC≡CCHCl$, $CF_3C≡C$, $CCl_3C≡C$ and $FCH_2C≡CCH_2$. Examples of "haloalkoxy" include $CF_3O$, $CCl_3CH_2O$, $CF_2HCH_2CH_2O$ and $CF_3CH_2O$. Examples of "haloalkylthio" include $CCl_3S$, $CF_3S$, $CCl_3CH_2S$ and $CH_2ClCH_2CH_2S$. Examples of "haloalkylsulfonyl" include $CF_3SO_2$, $CCl_3SO_2$, $CF_3CH_2SO_2$ and $CF_3CF_2SO_2$. Examples of "haloalkoxyalkoxy" include $CF_3OCH_2O$, $ClCH_2CH_2OCH_2CH_2O$, $Cl_3CH_2OCH_2O$ as well as branched alkyl derivatives.

The total number of carbon atoms in a substituent group is indicated by the "$C_i$-$C_j$" prefix where i and j are numbers from 1 to 8. For example, $C_1$-$C_3$ alkylsulfonyl designates methylsulfonyl through propylsulfonyl; $C_2$ alkoxyalkoxy designates $CH_3OCH_2O$; $C_3$ alkoxyalkoxy designates, for example, $CH_3OCH_2CH_2O$ or $CH_3CH_2OCH_2O$; and $C_4$ alkoxyalkoxy designates the various isomers of an alkoxy group substituted with a second alkoxy group containing a total of 4 carbon atoms, examples including $CH_3CH_2CH_2OCH_2O$, and $CH_3CH_2OCH_2CH_2O$. Examples of "alkoxyalkyl" include $CH_3OCH_2$, $CH_3OCH_2CH_2$, $CH_3CH_2OCH_2$, $CH_3CH_2CH_2CH_2OCH_2$ and $CH_3CH_2OCH_2CH_2$. Examples of "alkoxycarbonyl" include $CH_3OC(=O)$, $CH_3CH_2OC(=O)$, $CH_3CH_2CH_2OC(=O)$, $(CH_3)_2CHOC(=O)$ and the different butoxy-, pentoxy- or hexyloxycarbonyl isomers.

In the above recitations, when a compound of Formula I comprises one or more pyridyl or pyrimidinyl rings, all bonds to these heterocycles are made through the carbon atom(s) of the moieties.

Preferred for greatest fungicidal activity and/or ease of synthesis are the compounds of Group 1 that are the compounds of Formula I wherein:

J is H; OH; $C(=O)R^{16}$; $C(=O)OR^{17}$; $C(=0)NHR^{19}$;

$R^1$ is $C_1$-$C_4$ alkyl; $C_1$-$C_4$ haloalkyl; or vinyl;

$R^2$ is $C_1$-$C_{20}$ alkyl; $C_2$-$C_{20}$ alkoxyalkyl; $C_2$-$C_{20}$ haloalkyl; $C_3$-$C_8$ alkyl substituted with phenoxy or phenylthio each optionally substituted with $R^{30}$; $C_5$-$C_7$ cycloalkyl; $C_2$-$C_{20}$ alkenyl; $C_5$-$C_7$ cycloalkenyl; phenyl optionally substituted with $R^5$ and $R^7$; 2-naphthalenyl; thienyl optionally substituted with $R^5$ and $R^7$; furyl optionally substituted with $R^7$; or pyridyl optionally substituted with $R^5$ and $R^7$;

$R^3$ is phenyl optionally substituted with $R^{10}$;

$R^{16}$ is H; $C_1$-$C_6$ alkyl; or phenyl optionally substituted with $R^{34}$

$R^{17}$ is $C_1$-$C_6$ alkyl;

$R^{19}$ is $C_1$-$C_6$ alkyl; or phenyl optionally substituted with $R^{34}$;

provided that when $R^2$ is phenyl and $R^5$ is not F, then

$R^5$ is attached to the para-position relative to the oxazolidinone ring.

More preferred are the compounds of Group 2 that are the compounds of Group 1 wherein:

J is H; $C(=O)R^{16}$;

$R^1$ is $C_1$-$C_2$ alkyl or vinyl;

$R^2$ is $C_1$-$C_{12}$ alkyl; $C_5$-$C_7$ cycloalkyl; phenyl optionally substituted with $R^5$ and $R^7$; thienyl optionally substituted with $R^7$; or pyridyl optionally substituted with $R^5$ and $R^7$;

| | |
|---|---|
| $R^3$ | is phenyl optionally substituted with F; Cl; or methyl; |
| $R^4$ | is H; |
| $R^5$ | is halogen; nitro; $C_1$-$C_6$ alkyl; $C_1$-$C_3$ haloalkyl;methylthio; $C_1$-$C_6$ alkoxy; $C_1$-$C_2$ haloalkoxy; $C_5$-$C_6$ cycloalkyloxy; phenoxy optionally substituted with $R^{27}$; phenylthio substituted with $R^{24}$; phenoxymethyl optionally substituted with $R^{24}$ on the phenyl ring; benzyloxy optionally substituted with $R^{30}$ on the phenyl ring; or -OC(=O)$R^{28}$; |
| $R^7$ and $R^{24}$ | are independently F; $C_1$-$C_2$ alkyl; methylthio; or $C_1$-$C_2$ alkoxy; |
| $R^{16}$ | is $C_1$-$C_6$ alkyl; |
| $R^{19}$ | is phenyl optionally substituted with $R^{34}$; |
| $R^{27}$ | is 1-2 halogen; cyano; $C_1$-$C_4$ alkyl; trifluoromethyl; $C_1$-$C_4$ alkoxy; $C_1$-$C_4$ haloalkoxy; $C_1$-$C_4$ alkylthio; $C_5$-$C_6$ cycloalkyloxy; or allyl; |
| $R^{30}$ | is 1-2 halogen; cyano; $C_1$-$C_4$ alkyl; trifluoromethyl; $C_1$-$C_4$ alkoxy; or trifluoromethoxy; |
| $R^{34}$ | is 1-2 halogen; nitro; $C_1$-$C_2$ alkyl or $C_1$-$C_2$ alkoxy. |

Even more preferred are the compounds of Group 3 that are the compounds of Group 2 wherein:

| | |
|---|---|
| J | is H; |
| $R^1$ | is methyl; |
| $R^2$ | is $C_1$-$C_{12}$ alkyl; phenyl optionally substituted with $R^5$ and $R^7$; pyridyl optionally substituted with $R^5$ and $R^7$; |
| $R^5$ | is F; Cl; Br; $C_1$-$C_6$ alkyl; trifluoromethyl; $C_1$-$C_6$ alkoxy; trifluoromethoxy; 2,2,2-trifluoroethoxy; $C_5$-$C_6$ cycloalkyloxy; methylthio; phenoxy optionally substituted with $R^{27}$; phenylthio optionally substituted with $R^{24}$; benzyloxy optionally substituted with $R^{30}$ on the phenyl ring; or -OC(=O)$R^{28}$; |

Specifically preferred for greatest fungicidal activity and/or ease of synthesis are the compounds of Formula I that are:

5-(2,4-Difluorophenyl)-5-methyl-3-(phenylamino)-4-thioxo-2-oxazolidinone;
4-Imino-5-methyl-5-(4-phenoxyphenyl)-3-(phenylamino)-2-oxazolidinone;
4-Imino-5-methyl-5-(4-phenoxyphenyl)-3-(phenylamino)-2-oxazolidinone hydrobromide; and
5-(2,4-Difluorophenyl)-4-imino-5-methyl-3-(phenylamino)-2-oxozolidinone.

## Synthesis

When $R^1$ and $R^2$ of the compounds of Formula I are different, the compounds of Formula I possess a chiral center. This invention therefore includes racemic mixtures as well as pure enantiomers. Although one enantiomer can have superior fungicidal activity for a given compound of Formula I, the other enantiomer is neither devoid of activity nor interferes with the activity of the more potent enantiomer. One also will notice that compounds of Formula I can exist as a mixture of E- and Z-imine isomers when A = N-J. This invention therefore pertains also to mixtures of geometric isomers as well as the individual isomers.

For clarity in discussion of their preparation, the compounds of Formula I can be subdivided into compounds of Formulas Ia, Ia.HX, Ib, Ic, Id and Ie:

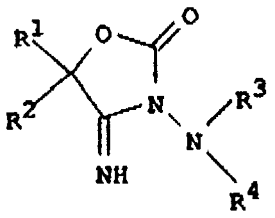

Ia

Compounds I wherein A = N-J and J = H.

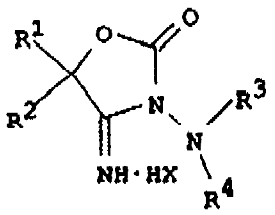

Ia.HX

Salts of compounds Ia with acids HX, wherein HX is a strong acid (such as HBr, HCl, HI, $HNO_3$, $H_2SO_4$, $H_3PO_4$, as well as organic acids such as alkyl- and arylsulfonic acids).

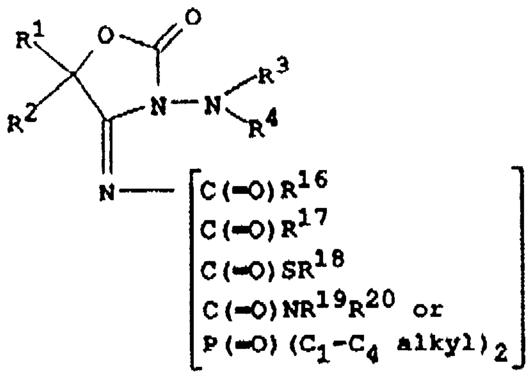

Ib

Compounds I wherein A is N-J, and J is as shown in brackets.

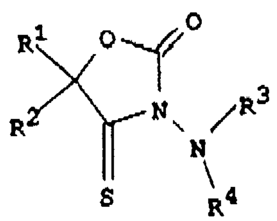

Ic

Compounds I wherein A is S.

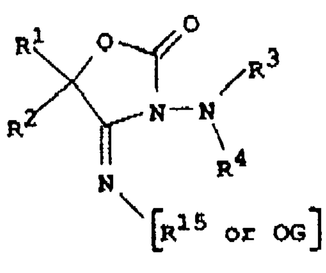

Id

Compounds I wherein A is N-J, J is $R^{15}$ or OG, and G is H; $C_1$-$C_4$ alkyl; or phenylmethyl optionally substituted with $R^{34}$ on the phenyl ring.

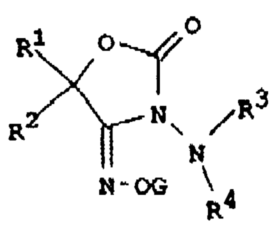

Ie

Compounds I wherein A is N-J, J is OG, and G is C(=O)($C_1$-$C_4$ alkyl); C(=O)($C_1$-$C_4$ alkoxy); or C(=O)NH$R^{36}$.

Preparation of the compounds Ia, Ia.HX, Ib, Ic, Id and Ie, all within structure I, is described schematically (Schematics 1 through 3) and in writing below.

Structure Ia: 4-Imino-2-oxazolidinones Ia can be prepared by sequential conversion of cyanohydrins II to their respective chloroformates III or *O*-(imidazolylcarbonyl) derivatives IV, treatment of a compound III or IV with a substituted hydrazine which produces the carbazates V, then cyclization of a carbazate V to the product Ia. This sequence is schematically illustrated in Schematic 1.

SCHEMATIC 1

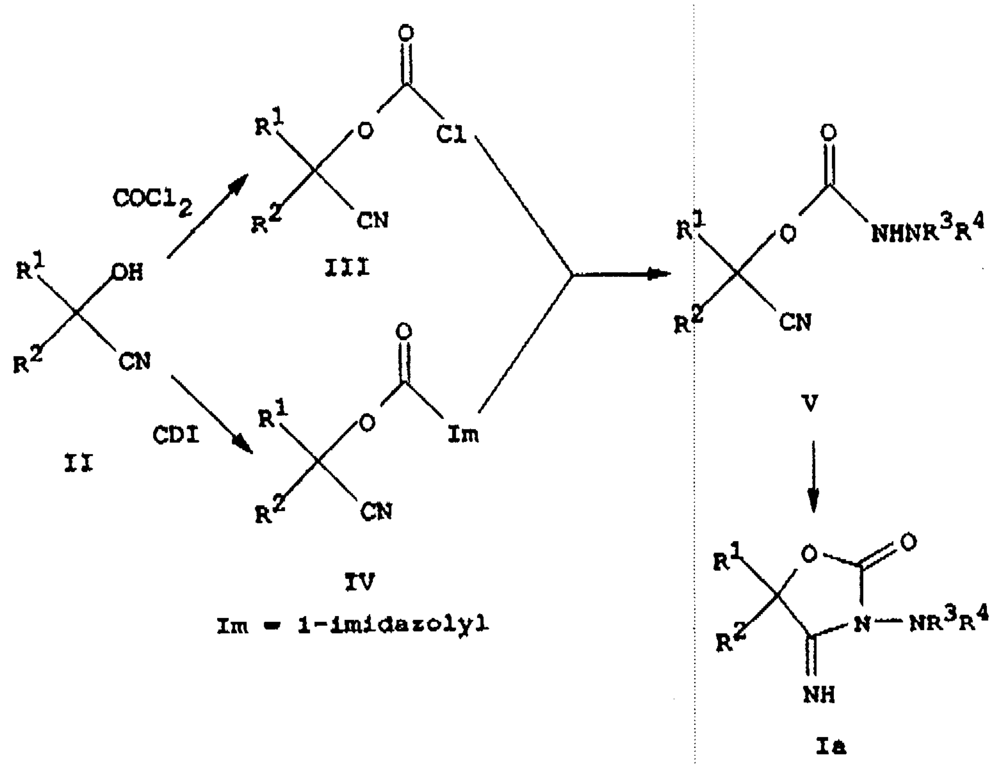


II, III, IV, V, Ia

The *O*-(imidazolylcarbonyl) derivatives IV will be the intermediates of choice over the chloroformates III for those cyanohydrins II which fail to react, or react only poorly, with phosgene. The cyanohydrins which react poorly with phosgene tend to be those derived from electron-rich-aryl alkyl ketones. Methodology for preparation of ketone cyanohydrins is well known to those skilled in the art. See, for example, *Org. Syntheses*, Coll. Vol. 4, 58, (1968) which teaches the preparation of acetophenone cyanohydrin, and Gassman et al. in *Tetrahedron Lett.* **1978**, 3773, which teaches a general method for preparation of trimethylsilyl cyanohydrins.

Conversion of cyanohydrins to chloroformates is generally accomplished by mixing the cyanohydrin with phosgene and an acid acceptor in an inert solvent. Typical acid acceptors include the tertiary-amine bases (such as pyridine, *N,N*-dimethylaniline, *N,N*-diethylaniline). Typical inert solvents include the aromatic hydrocarbons (such as benzene, toluene, xylenes), ethers (such as THF, dioxane, diethyl ether), and chlorinated hydrocarbons (such as methylene chloride, chloroform). Temperatures are typically -20° to +40°. The chloroformates can be used directly in solution or be isolated by filtration of the reaction mixture and evaporation of the solvent from the filtrate; further purification can be accomplished by dissolution of the chloroformate in an inert, water-immiscible solvent (such as ether, benzene, toluene, xylenes, ethyl acetate, methylene chloride, chloroform, 1-chlorobutane), washing the solution with cold, dilute mineral acid, cold water, drying the solution, and evaporation of the solvent. Conversion of cyanohydrins to their chloroformates is known in the literature (e.g., V. Kondratenko et al., *Probl. Poluch. Poluprod. Prom. Org. Sin., Akad, Nauk SSSR, Otd. Obshch. Tekh. Khim.* **1967**, 60-63; *Chem. Abs.*, **1968**, *68*, 12441m).

Conversion of cyanohydrins to their *O*-imidazolyl carbonates can be accomplished by mixing the cyanohydrin with 1,1'-carbonyldiimidazole (CDI) in an inert solvent until the reaction is substantially complete [optionally the CDI can be generated in situ from phosgene and imidazole as described by Staab and Wendel, Org. Synthesis Coll. Vol. 5, 201, (1973)]. The progress of the reaction can be conveniently followed by thin-layer chromatography, or by noting evolution of $CO_2$ (or lack thereof) on treatment of a sample of the reaction mixture with water, which reacts quickly with any unreacted CDI. Depending on the particular substrates, temperature, and concentration of reactants in solution, the reaction is generally over within a few minutes to a few days; typical times are about 2 h to an overnight period. Suitable inert solvents include those mentioned above for preparation of the chloroformates. Reaction temperatures can vary from about -20° up to the boiling point of the solvent, with ambient temperatures preferred for convenience. The reaction mixtures can be used directly for the next step (reaction with a substituted

hydrazine), or the intermediate *O*-(imidazolylcarbonyl) compounds can be purified and even isolated prior to use. For purification and isolation the solvent in the reaction mixture is evaporated (if water-miscible), and the residue is treated with cold water and water-immiscible solvent (e.g., diethyl ether, benzene, toluene, 1-chlorobutane, methylene chloride, chloroform, ethyl acetate); the layers are separated, and the organic layer is dried and evaporated to the *O*-(imidazolylcarbonyl) compound.

Conversion of the cyanohydrin chloroformates to the carbazates comprises mixing the chloroformate with a substituted hydrazine in an inert solvent, with either some of a substituted hydrazine or an added tertiaryamine base [e.g., pyridine, *N,N*-diethylaniline, *N,N*-dimethylaniline, triethylamine, (*N,N*-diisopropyl)-ethylamine] acting as an acid acceptor. Suitable solvents include those suitable for preparation of the chloroformate. The reaction is generally rapid and complete within a few minutes in the usual temperature range (about -20° to ambient). If desired, the intermediate carbazate can be isolated by evaporating the solvent in vacuo (if water-miscible) and washing a solution of the residue in a water-immiscible solvent [such as mentioned for the preparation of an O-(imidazolylcarbonyl) compound] with dilute mineral acid and water, drying, and evaporation of the solvent. If the carbazate does not spontaneously cyclize to the 4-imino-2-oxazolidinone within an acceptable time, cyclization can be effected by treatment of a solution of the carbazate in an inert solvent (e.g., toluene, benzene, 1-chlorobutane, THF, chloroform) with imidazole or a tertiary-amine base [e.g., triethylamine, (*N,N*-diisopropyl)ethylamine, pyridine] and optionally heating the mixture up to the boiling point of the solvent; for example, cyclization is typically effected by boiling a toluene solution of the carbazate in the presence of triethylamine at reflux for 2 h.

Conversion of an *O*-(imidazolylcarbonyl) derivative to a carbazate (which generally cyclizes spontaneously to the 4-imino-2-oxazolidinone under the influence of the liberated imidazole) is effected by mixing an *O*-(imidazolylcarbonyl) derivative with a substituted hydrazine in an inert solvent (such as those mentioned for preparation of the chloroformate). Reaction is generally complete within a few minutes to an hour. Purification of the products can be effected by washing the reaction mixtures (if a water-immiscible solvent was used) with water, drying, and evaporation of the solvent. If cyclization of a carbazate to a 4-imino-2-oxazolidinone is incomplete, it can be effected as described above. If cyclization is substantially complete, then the product has been obtained. As noted in the Examples, the infrared spectrum of the carbazate shows absorption around 1738 $cm^{-1}$.; for the 4-imino-2-oxazolidinone the corresponding figure is around 1800 $cm^{-1}$.

Substituted hydrazines like those illustrated in Schematic 1 can be prepared by literature methods. For example, see J. Timberlake; J. Stowell; *The Chemistry of* the *Hydrazo, Azo, and Azoxy Groups* (S. Patai, Ed.) John Wiley and Sons, Ltd., London (1975), p 69; and Demers, J. P.; Klaubert, D. J.; *Tetrahedron Lett.* 1987, 4933

Structure Ia.HX: Ease of isolation of the imino compound Ia can sometimes be enhanced by conversion of it to a salt, for example by addition of a strong acid HX (as earlier described) to a solution of compound Ia in an inert organic solvent (such as those mentioned for preparation of the chloroformate above). Crystallization of the salt Ia•HX can then often be effected from the same or different organic solvent, or the salt can be recovered by evaporation of the solvent. Some of the imine salts offer advantage of formulation or enhanced fungicidal activity over the free-base forms.

Additionally, compounds Ia•HX can be converted to the corresponding 2,4-oxazolidinedione ia by reaction with water; typically the salt (or Ia plus an acid) is mixed with water, optionally in the presence of a cosolvent (e.g., acetone, butanone, THF) and optionally heated until conversion to the 4-oxo compound ia is substantially complete. The 4-oxo compound can be isolated in the usual ways, such as by filtration or evaporation of the reaction mixture, or by extraction of the product into an organic solvent and evaporation of that solvent. Schematically:

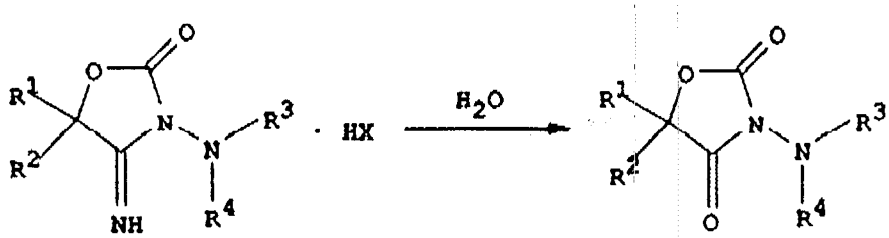


or

Ia + acid

ia

Structure Ib: Schematic 2 depicts preparation of compounds of Structure Ib.

SCHEMATIC 2

- Ia + Y[(C=O)$R^{16}$; (C=O)O$R^{17}$; (C=O)S$R^{18}$; or P(=O)($C_1$-$C_4$ alkyl)$_2$] → Ib, with J = (C=O)$R^{16}$; C(=O)O$R^{17}$; C(=O)S$R^{18}$; or P(=O)($C_1$-$C_4$ alkyl)$_2$.

- Ia or Ia.HX/base + (H or $R^{34}$)-Ph-NCO → Ib, with J = (C=O)NH-Ph-(H or $R^{34}$).

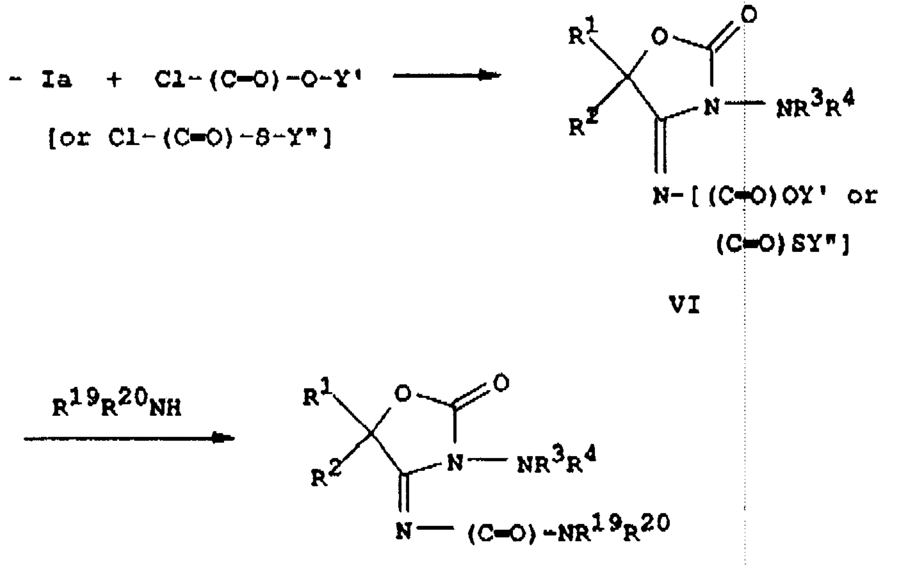


VI

Ib, with J = (C=O)-N$R^{19}R^{20}$

The *N*-acyl compounds Ib wherein J is (C=O)$R^{16}$, (C=O)O$R^{17}$, (C=O)S$R^{18}$, or P(=O) ($C_1$-$C_4$ alkyl)$_2$ can be prepared by reaction of compound Ia (or Ia•HX) with an appropriate acylating agent Y-[(C=O)$R^{16}$; (C=O)O$R^{17}$; (C=O)S$R^{18}$; or P(=O)($C_1$-$C_4$ alkyl)$_2$], wherein Y is a leaving group such as chloride, bromide, acetate, alkyloxycarbonyloxy (such as isobutoxycarbonyloxy), or other moiety commonly used as a leaving group in such acylations. When acylating agents with the most common leaving group (i.e., chloride) are used, the agents are acid chlorides, chloroformates, chlorothiolformates, and dialkylphosphinic chlorides. Operationally, the compound Ia (or Ia•HX) is mixed with an inert organic solvent (e.g., THF, ether, ethyl acetate, methylene chloride) and treated with a tertiary-amine base [such as *N,N*-diethylaniline, *N,N*-dimethylaniline, pyridine, triethylamine, (*N,N*-diisopropyl)ethylamine; with weak-acid-forming leaving groups such as acetate and alkoxycarbonyloxy, the tertiary-amino base can be omitted when the free base Ia is used] and the acylating agent. The reaction is allowed to proceed at about -20° to ambient temperature (for convenience) until it is substantially complete, which may require a few minutes to several days. The solvent (if water-miscible) is evaporated from the reaction mixture and treated with a water-immiscible solvent and water. The organic solution is washed with dilute mineral acid, dried, and evaporated to the product, which can be further purified, if desired, by recrystallization or chromatography.

For preparation of compounds Ib wherein J is C(=O)N$R^{19}R^{20}$ -- with $R^{19}$ as phenyl optionally substituted with $R^{34}$, and $R^{20}$ as H -- the compound Ia (as such, or free-based by a tertiary-amine base in situ from Ia•HX, as mentioned above) is allowed to react with an optionally-substituted-phenyl isocyanate

until the reaction is substantially complete. Isolation and purification of product can be accomplished as described above for the other *N*-acyl compounds.

For preparation of compounds Ib wherein J is $C(=O)NR^{19}R^{20}$, the compounds Ia are allowed to react sequentially with a chloroformate Cl-C(=O)O-Y' or chlorothiolformate Cl-C(=O)S-Y'' in substantially the same way as with the similar acylating agents above, followed by reaction of the intermediate VI with the appropriate amine $HNR^{19}R^{20}$. In these reactions,

Y'O is a labile leaving group less labile than halide; e.g., 4-nitrophenoxy, in which case the chloroformate is 4-nitrophenyl chloroformate, and

Y'' is, e.g. $C_1$-$C_4$ alkyl, phenyl, nitrophenyl or chlorophenyl in which case a chlorothiolformate could be methyl chlorothiolformate.

Structure Ic: The 4-thioxooxazolidin-2-ones Ic can be prepared by two Methods (Method 1 and Method 2) shown in Schematic 3 and discussed below.

SCHEMATIC 3

Method 1:

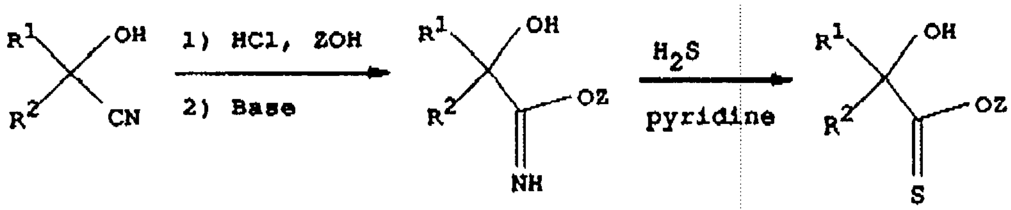


II VII VIII

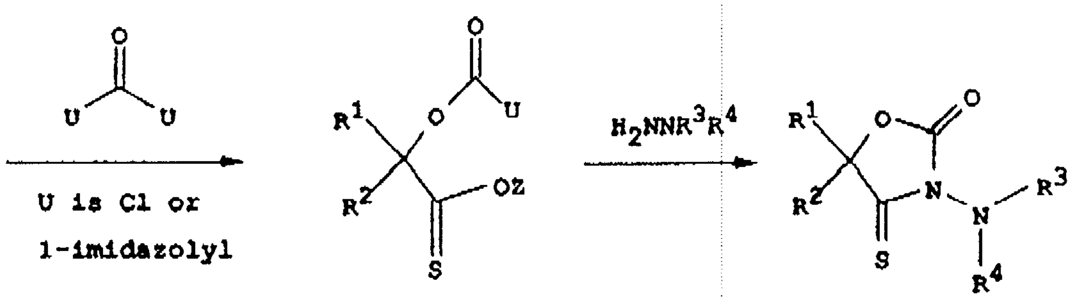


IXa: U = Cl
IXb: U = 1-imidazolyl

Ic

Z in ZOH and VII-IX is $C_1$-$C_4$ alkyl, $C_3$-$C_4$ alkenyl, $C_5$-$C_6$ cycloalkyl, or $PhCH_2$.

Method 2

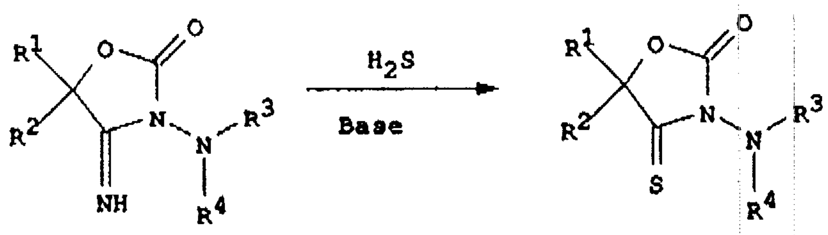


Ia Ic

Preparations of 2-hydroxy thionocarboxylic acid esters like VIII are known in the literature. It is known that cyanohydrins II or TMS cyanohydrins (previously referenced) can be converted to the 2-hydroxy iminoethers VII by treatment with HCl gas in an alcohol (or alcohol-containing) solvent; preferred for ease of synthesis, lower expense, or greater utility are the ethers in which Z is $C_1$-$C_4$ alkyl. This is followed by alkalinization. This two-step procedure is well known; see for example Schnur, R. C. et al., *J. Med. Chem.* 1982, *25*, 1451.

The imino ethers VII are converted to 2-hydroxy thionocarboxylic esters VIII with dry hydrogen sulfide in pyridine as taught, for example, by Janssen, M.J., "The Chemistry of Carboxylic Acids and Esters," Patai, S., Ed., Chap. 15, Interscience, New York, 1969; and Geffken, D., et al., *Arch. Pharm. (Weinheim)*, 1988, *321*, 45.

The 2-hydroxy thionocarboxylic esters VIII are dissolved in an inert solvent such as methylene chloride or 1-chlorobutane, and treated with a tertiary-amine base such as triethylamine, pyridine, and (*N,N*-diisopropyl)ethylamine, at a temperature of about -60° to +30°; to this mixture is added phosgene to provide products of Formula IXa. The phosgene can be added as a gas or liquid or dissolved in an inert solvent such as toluene and added in solution. When the reaction is complete, the resulting mixture is poured into a water-immiscible solvent and washed with mineral acid, water, and brine. The organic-liquid phase is dried and evaporated to yield products of Formula IXa.

The compounds IXb can be prepared with 1,1'-carbonyldiimidazole (CDI) and the hydroxy compounds VIII. The hydroxy compound VIII is dissolved in an inert solvent in which the CDI has sufficient solubility at the reaction temperature. Methylene chloride, 1-chlorobutane and toluene are three of many suitable inert solvents. The CDI is added as a solid or as a solution in an inert solvent at temperatures from 0° to 100°. When the synthesis reaction is complete, the resulting mixture is poured into a water-immiscible solvent and washed successively with dilute mineral acid, water, and brine. The organic-liquid phase of the mixture is separated, dried, and evaporated to isolate the product.

In some cases, isolation of the compounds IXa and IXb is unnecessary. For example, after their formation is complete, the compounds can be treated directly with the substituted hydrazine $H_2N-NR^3R^4$. Further, the use of pure, isolated CDI is unnecessary in the synthesis of compounds IXb. The CDI can be first prepared, for example, by treatment of a solution of imidazole in an inert solvent with phosgene as previously noted, and then treated directly with the hydroxy compounds VIII.

Compounds of Formula IXa or IXb can be dissolved in an inert solvent such as methylene chloride, 1-chlorobutane, or THF and treated with a substituted hydrazine $H_2N-NR^3R^4$ at a temperature from 0° to 80°. When compound IXa (U = Cl) is used, about one equivalent of a tertiaryamine base such as triethylamine, *N,N*-diethylaniline, (*N,N*-diisopropyl)ethylamine, or a second equivalent of the substituted hydrazine can be added. When compound IXb (U = 1-imidazolyl) is used, about one equivalent of a carboxylic acid can be added to accelerate the reaction; suitable carboxylic acids include acetic, pivalic, and benzoic acids. Upon completion of the reaction of IXa or IXb with the substituted hydrazine, the product of Formula Ic can be isolated by evaporation of the aforementioned inert solvent, and purified by dissolving the residue in a water-immiscible solvent such as ether or methylene chloride, washing with mineral acid, aqueous base, and water, drying, and evaporating the extraction solvent. Crystallization or chromatography can be utilized for additional purification if desired.

Preferred for reasons of higher yields and lower expense is the use of CDI [compound U-(C=O)-U with U = 1-imidazolyl] for preparation of compounds Ic.

As illustrated in Method 2 above, the 4-thiooxazolidin-2-ones Ic can be prepared by reaction of 4-iminooxazolidin-2-ones Ia (or Ia•HX) with hydrogen sulfide under basic conditions. For example, a solution of the imino compound Ia in pyridine can be treated with $H_2S$ (added as a gas or liquid) and kept at about

-20° to +40° until the conversion is substantially complete. Or, a solution of the imino compound in an alcohol (e.g., methanol, ethanol) can be treated with a tertiary-amine base (e.g., triethylamine, triethanolamine) and $H_2S$ and kept at about the same temperature until reaction is substantially complete. The product Ic can be isolated, and further purified as noted above.

Structure Id: These compounds can be prepared by alkylation of the 4-sulfur atom of compoundc Ic with iodomothane, dimethyl sulfate, trimethyloxonium tetrafluoroborate, or other alkylating agent and treatment of the resulting thionium salt with either the amino $H_2NR^{15}$ or hydroxylamine derivative $H_2N$-OG (with G defined as noted alongside of Formula Id). Reactions of this type have been described by Hunig, S., et al. (*Liebigs Ann. Chem.* **1962**, *651*, 89).

The amines and hydroxylamines employed in Method 2 above can be purchased from commercial sources or prepared by literature methods. The general preparation of hydroxylamines can also be accomplished by methods described in Castellino, A. J., et al. *J. Org. Chem.* **1984**, *49*, 1348; Murray, R. W., et al. *Synthetic Comm.* **1989**, *19*, 3509 and references cited therein; and Baldol, C., et al. *Synthesis* **1988**, 344.

Structure Ie: Those compounds can be prepared from those compounds of Id wherein OG is OH, through reaction with acylating agents Y-[C(=O)($C_1$-$C_4$ alkyl) or C(=O)($C_1$-$C_4$ alkoxy)] or isocyanates $R^{36}$-NCO. The nature of Group Y, reactions with the acylating agents and isocyanates, and isolation of the products are substantially as discussed earlier for preparation of the compounds Ib.

In addition to those disclosed above, other derivatives may be expected from reaction of imines of Formula Ia and 4-thioxo-2-oxazolidinones of Formula Ic with various nucleophiles and electrophiles.

Specific compounds of Formula I that can be made by the process at this invention are described in the Examples and Tables which follow. Those are intended to be only exemplary, and are not inclusive.

The following are a list of abbreviations and their definitions used in the Tables and Examples which follow. Unless indicated otherwise, alkyl, alkenyl, and alkynyl radicals are the normal isomers. For example, "pentyl" implies the *n*-pentyl isomer. The numbering system for heterocyclic groups is defined in the following way. First, hetero atoms contained in the ring are given the lowest possible numbers using the accepted rules of heterocycle numbering. Then, the point of attachment of the radical is the position assigned the highest priority. For example, the attached group at C-3 in 6-MeOC(=O)-3-pyridyl is higher priority that the MeOC(=O) substituent. Double bonds contained in compounds listed in the Tables are at the (E)-configuration unless indicated otherwise.

Ac = acetyl
Me = methyl
Et = ethyl
Pr = *n*-propyl
Bu = *n*-butyl
Bzl = benzyl
BzlO = benzyloxy
*c* = cyclo
OAc = acetate
CN = cyano
$NO_2$ = nitro
$SO_2$ = sulfonyl
S(O) = sulfinyl
PhO = phenoxy
BuO = n-butoxy PrO = *n*-propoxy

EXAMPLE 1

Preparation of *O*-Methyl 2-(2,4-diflourophenyl)thiolactate

A 10 g (64 mmol) portion of commercially-available 2,4-difluoroacetophenone was dissolved in dry methylene chloride (100 mL) and treated at room temperature with 10.2 mL (7.6 g, 77 mmol) trimethylsilyl cyanide and then 0.61 g (2 mmol) of zinc iodide. The mixture was stirred at room temperature overnight (14 h). The resulting yellow solution was then washed with saturated aq. $NaHCO_3$ (2 x 100 mL) and brine (100 mL), and each aqueous wash was back-extracted with fresh methylene chloride. The organic phases were combined, dried ($MgSO_4$), and evaporated to give a yellow oil. The resulting, crude trimethylsilyl cyanohydrin (TMSC) was used immediately in the following step.

All but three grams of the crude TMSC were dissolved in a mixture of ether (360 mL) and methanol (90

mL) and cooled to -78°. Hydrogen chloride gas was introduced while maintaining a temperature of -30°. When the solution became saturated as evidenced by a drop in temperature, the gas flow was halted. The solution was warmed to 0° and allowed to stand at 0° overnight (14 h). The reaction mixture was evaporated to a white solid, and the solid was washed with ether and dried. The free base of the iminoether hydrochloride was obtained by dissolving the solid in saturated aq. $NaHCO_3$ (150 mL) and extracting with ether (2 x 150 mL). The other layers were washed with brine (100 mL), combined, dried ($MgSO_4$), and evaporated. The resulting iminoether (8.29 g) was obtained as a viscous yellow oil, and was used without further purification in the following step.

The iminoether from above was dissolved in pyridine (100 mL) and cooled to 0°. Hydrogen sulfide gas was introduced for 1 h, and then the reaction mixture was gradually warmed to room temperature and outgassed for 1 h. The reaction mixture was poured into ice-cold 3N aqueous HCl (100 mL) and extracted with ether (2X). The ether phases were washed with 1N aq. HCl and brine, dried ($MgSO_4$), and evaporated to provide the crude product. Traces of pyridine that remained in the crude product were removed by redissolving the crude product in ether and washing again with 1N aq. HCl (2X), and brine. The ether extract was dried ($MgSO_4$) and evaporated. The crude product was further purified by bulb-to-bulb distillation to give 6.84 g of the title compound of this example as a yellow oil. $^1H$ NMR ($CDCl_3$, 200 MHz): $\delta$ 7.53 (m, 1H), 6.80 (m, 2H), 4.59 (s, 1H, OH), 4.13 (s, 3H), 1.84 (s, 3H).

## EXAMPLE 2

### Preparation of 5-(2,4-difluorophenyl)-5-methyl-3-(phenylamino)-4-thioxo-2-oxazolidinone

Ethyl 2-(2,4-difluorophenyl)thiolactate (3 g, 12.9 mmol) was dissolved in 1-chlorobutane (25 mL), treated with 1,1'-carbonyldiimidazole (3.15 g, 19.4 mmol), and heated at reflux for 6 h. After standing at room temperature overnight (14 h), the resulting mixture was poured into ether and washed with water and brine. The aqueous washes were reextracted with ether and then both ether layers were combined, dried ($MgSO_4$), and evaporated. The resulting crude product (4.5 g), was purified by chromatography over silica gel eluting with 3:1 hexanes:EtOAc to give 1.3 g of acyl imidazole. The acyl imidazole material was dissolved in 1-chlorobutane (75 mL) and treated sequentially with phenylhydrazine (0.435 mL, 4.4 mmol) and HOAc (0.275 mL, 4.8 mmol). The mixture was stirred overnight (14 h) at room temperature and then poured into ether and washed with 1N aq. HCl, water, saturated aq. $NaHCO_3$, and brine. The aqueous layers were extracted with fresh ether and the ether layers were combined, dried ($MgSO_4$), and evaporated to give 0.81 g of an orange oil. Chromatography over silica gel eluting with 5:1 hexanes:EtOAc afforded 0.57 mg of a yellow-brown oil. Further purification of the yellow-brown oil by recrystallization from 1-chlorobutane/hexanes provided the title compound of this example as a light green solid. $^1H$ NMR ($CDCl_3$, 200 MHz): $\delta$ 7.53 (m, 1H), 7.32 (m, 2H), 7.07 (m, 1H), 6.96 (m, 4H), 6.60 (s, 1H, NH), 2.14 (s, 3H); IR (mineral oil): 3352, 1783 $cm^{-1}$; mp 123-124°.

## EXAMPLE 3

### Preparation of 5-methyl-5-octyl-3-(phenylamino)-4-thioxo-2-oxazolidinone

A solution of 4-imino-5-methyl-5-octyl-3-(phenylamino)-2-oxazolidinone hydrobromide (0.92 g, 0.0023 mol) in pyridine (25 mL) was treated with liquid $H_2S$ (3 mL, 0.085 mol) with cooling in an ice bath, then stirred overnight at room temperature. The mixture was stripped to a yellow oil/solid residue, which was treated with 1-chlorobutane and water. The organic solution was washed with 1N HCl, water, and saturated brine, dried ($MgSO_4$), and stripped to a yellow oil comprising the title product of this example. IR (neat): 1806 $cm^{-1}$; TLC ($CHCl_3$): $R_f$ ~0.7 (major), trace component of slightly higher $R_f$; Anal. Calcd for $C_{18}H_{26}N_2O_2S$: C, 64.63; H, 7.84; N, 8.38; S, 9.59; Found: C, 62.93, 63.02; H, 7.67, 7.68; N, 8.01, 7.99; S, 12.35, 12.34.

## EXAMPLE 4

### Preparation or 4-imino-5-methyl-5-octyl-3-(phenylamino)-2-oxazolidinone hydrobromide

A solution of 2-decanone (16.62 g, 0.1064 mol) in methylene chloride was treated with trimethylsilyl cyanide (15.2 mL, 0.112 mol, 98% reagent) and a catalytic amount of zinc iodide. After 3.5 h, the solution was heated briefly to boiling. The cooled solution was washed with ice-cold water and saturated brine, dried

($MgSO_4$), and stripped to an oil (25.81 g), which was 2-decanone trimethylsilylcyanohydrin. The IR spectrum showed no evidence of ketone. The above oil dissolved in THF (150 mL) was treated with conc. HCl (16.7 mL, 0.2 mol), and the THF stripped off. The residue was extracted with ether (2X). The combined extracts were washed with water (2X) and saturated brine, dried ($MgSO_4$), and stripped to an oil (22.69 g) which contained 2-decanone cyanohydrin.

The above oil dissolved in ether (200 mL) and cooled to -10 +/-2°, was treated with liquid phosgene (10.4 mL, 0.15 mol), followed by addition during a 4-min period of a solution of pyridine (5 drops) in *N,N*-diethylaniline (17 mL, 0.107 mol). The mixture was stirred in the cold for 5 min, then for 2.5 h as the temperature was allowed to rise to 12°, and finally for 1 h without cooling. Filtration removed a white water-soluble solid, and the filtrate was stripped to an oil. A solution of the oil in ether (200 mL) was washed with cold 1N HCl (2X) and ice-water, dried ($MgSO_4$), and stripped to an orange oil. The oil was then dissolved in benzene and reevaporated, leaving an oil (26.55 g), which contained the chloroformate of 2-decanone cyanohydrin. IR (neat): 1786 $cm^{-1}$.

Into a stirred, ice-cooled solution of the above oil (9.96 g, 0.0405 mol assuming pure chloroformate) was pipetted phenylhydrazine (4.1 mL, 0.0404 mol), followed by pyridine (3.3 mL, 0.0410 mol). After 1.5 h without cooling the mixture was filtered. The filtrate was stripped to a yellow oil, and the oil was dissolved in ether. The ether solution was washed with 1N HCl (2X), water and saturated brine, dried ($MgSO_4$) and stripped to a light-orange oil (9.49 g), which contained the carbamate (*n*-octyl)(Me)C(CN)OC(=O)NHNHPh. IR (neat): 1738 $cm^{-1}$.

A solution of the above carbamate in toluene (100 mL), treated with triethylamine (3.5 mL), was heated at reflux for 2 h. The yellow supernatant solution was decanted from a little brown material, and stripped to a yellow oil, which contained the title product of this example in the free-base form. IR (neat): 1801 $cm^{-1}$.

The free base, dissolved in ether, was treated with a solution of 30% HBr in HOAc. The resulting solution was stripped of solvent, then dissolved in toluene and restripped (2X). The residual oil hardened to a soft solid under a nitrogen stream. The solid was stirred with ether, and the title product of this example was filtered off as a white solid, 2 g; m.p. 183-6°, dec.; IR (mineral oil): 1840 $cm^{-1}$; Anal. Calcd for $C_{18}H_{28}BrN_3O_2$: C, 54.27; H, 7.08; N, 10.55; Br, 20.06; Found: C, 53.82, 53.76; H, 7.05, 7.09; N, 10.52, 10.52; Br, 20.07, 20.16.

## EXAMPLE 5

### Preparation of 4-imino-5-methyl-5-(4-phenoxyphenyl)-3-(phenylamino)-2-oxazolidinone hydrobromide

To a solution of 4-phenoxyacetophenone trimethylsilylcyanohydrin (5.10 g, 0.0164 mol, prepared as described in Example 4 for 2-decanone trimethylsilylcyanohydrin) in THF (25 mL) was added conc. HCl (2.5 mL, 0.03 mol). The solution was stripped of THF, and the residue was extracted with ether. The ether solution was washed with water (2X) and saturated brine, dried ($MgSO_4$), and evaporated to an orange oil (4.20 g). The oil contained 4-phenoxyacetophenone cyanohydrin. TLC (silica gel, $CHCl_3$): $R_f$~0.4.

To a solution of the above cyanohydrin in THF (25 mL) was added 1,1'-carbonyldiimidazole (CDI, 3.20 g of 90% reagent; 0.0178 mol). Not all of the CDI dissolved. After 68 h, the clear, orange solution was stripped to an oil. The oil was dissolved in 1-chlorobutane, and the solution was washed with water (3X), dried ($MgSO_4$), and stripped to an orange oil (6.14 g). The oil contained the *O*-(imidazolylcarbonyl) derivative of 4-phenoxyacetophenone cyanohydrin. IR (neat): 1777 (s), 1679 (w) $cm^{-1}$; TLC ($CHCl_3$, $R_f$): ~0.27 (major), and 3 minor spots (2 lower- and 1 higher-$R_f$).

To a solution of the above oil in 1-chlorobutane (25 mL) was added phenylhydrazine (1.7 mL, ~0.0164 mol). After 20 h the IR spectrum of a sample, after evaporation to an oil, showed a strong absorption at ~1797 $cm^{-1}$ showing the formation of the oxazolidinone ring. The TLC ($CHCl_3$) of the reaction mixture, the same after 20 h as after 2.5 h, showed 2 major spots ($R_f$'s = 0.20, 0.60) and a minor spot ($R_f$ = 0.006). The reaction solution was stripped to a red oil, and then dissolved in chloroform. The chloroform solution was filtered on silica gel which filled a Buchner funnel. Elution with chloroform provided a fraction that was substantially homogeneous by TLC (9:1, $CHCl_3$/EtOAc, v/v; $R_f$ 0.4); evaporation of this fraction provided a red oil (3.87 g). The oil was the substantially pure 2-oxazolidinone of this example title, in the free-base form. IR (neat): ~1796 (s) cm-1.

To an ether solution of the above oil, cooled in an ice bath, was added a solution of 30% HBr in HOAc. A tacky off-white solid precipitated. The supernatant solution was decanted, and the solid was rinsed with ether and recrystallized from acetonitrile-ether to give 2.51 g of the title product of this example as a white solid. IR (mineral oil): ~1834 (s) $cm^{-1}$; Anal. Calcd for $C_{22}H_{20}BrN_3O_3$: C, 58.16; H, 4.44; N, 9.25; Br, 17.59; Found: C, 57.82; H, 4.35; N, 9.19; Br, 17.66; mp 161°, dec.

EXAMPLE 6

Preparation of 5-methyl-5-(4-phenoxyphenyl)-3-(phenylamino)-2,4-oxazolidinedione.

A sample of 4-imino-5-methyl-5-(4-phenoxyphenyl)-3-(phenylamino)-2-oxazolidinone hydrobromide, prepared as described in Example 5, was dissolved in aq. acetone. The solution was heated on a steam bath for 5 min. Evaporation of the mixture and extraction of the residue with 1-chlorobutane provided, on evaporation of the dried solution, a white solid identified as the title compound of this example. The IR spectrum and TLC behavior were identical to an authentic sample of 5-methyl-5-(4-phenoxyphenyl)-3-(phenylamino)-2,4-oxazolidinedione prepared as described in WO 90/12791.

EXAMPLE 7

Preparation of 4-imino-5-methyl-5-(4-phenoxyphenyl)-3-(phenylamino)-2-oxazolidinone from the hydrobromide

4-Imino-5-methyl-5-(4-phenoxyphenyl)-3-(phenylamino)-2-oxazolidinone hydrobromide, a saturated $NaHCO_3$ solution, and EtOAc were mixed well for 5 min, providing 2 clear layers. The EtOAc solution was dried ($MgSO_4$) and evaporated to a foamy grease which slowly solidified. Crystallization was effected from 1-chlorobutane/hexane, providing the title compound of this example as a white solid. mp 137-139°; Anal. Calcd for $C_{22}H_{19}N_3O_3$: C, 70.76; H, 5.13; N, 11.25; Found: C, 69.69; H, 5.24; N, 11.15.

EXAMPLE 8

Preparation of *N*-[5-methyl-2-oxo-5-(4-phenoxyphenyl)-3-(phenylamino)-4-oxazolidinylidenelacetamide

A solution of 4-imino-5-methyl-5-(4-phenoxyphenyl)-3-(phenylamino)-2-oxazolidinone (0.84 g, 0.0022 mol) in THF (25 mL) was treated with acetyl chloride (0.16 mL, 0.0022 mol), followed by *N,N*-diethylaniline (0.35 mL, 0.0022 mol) and pyridine (1 drop). After 3 h, the mixture was stripped to a clear grease. The grease was dissolved in 1-chlorobutane/water, and the organic layer washed with 1N HCl, water, and saturated brine, dried, and evaporated until the precipitation of white solid. The solid, the title compound of this example, was isolated by filtration. mp 160-162°; Anal. Calcd for $C_{24}H_{31}N_3O_4$: C, 69.38; H, 5.10; $N_1$, 10.11; Found: C, 69.32; H, 5.01; N, 9.96.

EXAMPLE 9

Preparation of *N*-[5-methyl-5-(4-phenoxyphenyl)-3-phenylamino-4-oxazolidinylidene]-*N'*-phenylurea

A solution of 4-imino-5-methyl-5-(4-phenoxyphenyl)-3-(phenylamino)-2-oxazolidinone (0.5 g, 0.0013 mol) in THF (25 mL) was treated with phenyl isocyanate (0.14 mL, 0.0013 mol). After stirring overnight, the mixture was stripped to an oil. The oil was crystallized from 1-chlorobutane/hexane, and then again from 1-chlorobutane to provide the title compound of this example as a white solid. mp 104-106°, dec.; Anal. Calcd for $C_{29}H_{24}N_4O_4$: C, 70.72; H, 4.91; N, 11.28; Found: C, 70.54; H, 5.04; N, 11.29.

EXAMPLE 10

Preparation of methyl *N*-[5-methyl-2-oxo-5-(4-phenoxyphenyl)-3-(phenylamino)-4-oxazolidinylidene]-carbamate

A solution or 4-imino-5-methyl-5-(4-phenoxyphenyl)-3-(phenylamino)-2-oxazolidinone (0.64 g, 0.0017 mol) in THF (25 mL) was treated with methyl chloroformate (0.13 mL, 0.0017 mol), followed by *N,N*-diethylaniline (0.27 mL, 0.0017 mol) and 1 drop of pyridine. After stirring overnight, the mixture was stripped to a clear grease, which was treated with water and 1-chlorobutane. The layers were separated and the organic layer was washed with 1N HCl and saturated brine, dried ($MgSO_4$), and evaporated until crystallization occurred. The title product of this example was isolated as a white solid. mp 147-149°, dec.; Anal. Calcd for $C_{24}H_{21}N_3O_5$: C, 66.81; H, 4.91; N, 9.74; Found: C, 66.71; H, 5.00; N, 9.54.

From the teaching above, one can prepare the compounds of this invention such as those exemplified in Tables 1-23.

Compounds referred to in Tables below:

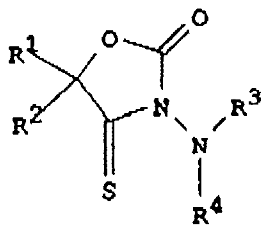

Ic

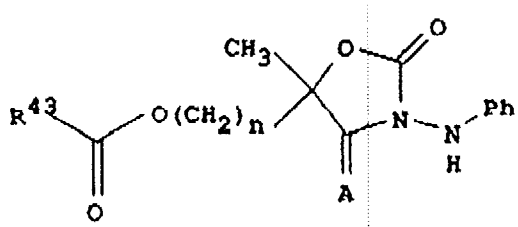

If

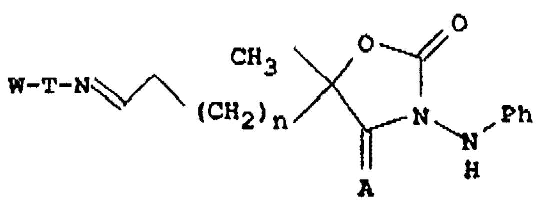

Ig

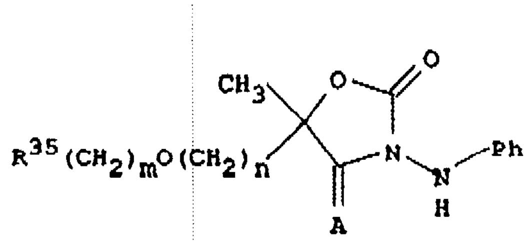

Ih

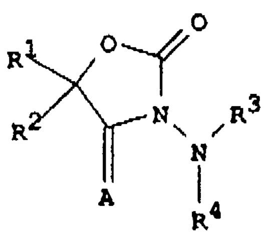

I

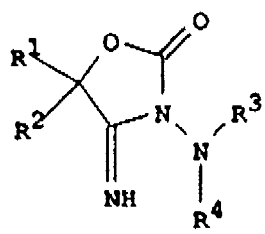

Ia

## TABLE 1

Compounds of Formula Ic wherein:

| $R^1$ | $R^2$ | $R^3$ | $R^4$ |
|---|---|---|---|
| Me | 2,4-diF-Ph | Ph | H |
| Et | 2,4-diF-Ph | Ph | H |
| *n*-butyl | 2,4-diF-Ph | Ph | H |
| $CF_3$ | 2,4-diF-Ph | Ph | H |
| $CF_3CH_2CH_2CH_2$ | 2,4-diF-Ph | Ph | H |
| cyclopropyl | 2,4-diF-Ph | Ph | H |
| cyclohexyl | 2,4-diF-Ph | Ph | H |
| vinyl | 2,4-diF-Ph | Ph | H |
| allyl | 2,4-diF-Ph | Ph | H |
| $CO_2CH_3$ | 2,4-diF-Ph | Ph | H |
| *n*-propyloxy-carbonyl | 2,4-diF-Ph | Ph | H |
| $PhCH_2$ | 2,4-diF-Ph | Ph | H |
| 4'-F-Bzl | 2,4-diF-Ph | Ph | H |
| 3'-Me-Bzl | 2,4-diF-Ph | Ph | H |
| 4'-$CF_3$-Bzl | 2,4-diF-Ph | Ph | H |
| 4'-MeO-Bzl | 2,4-diF-Ph | Ph | H |
| 4'-MeS-Bzl | 2,4-diF-Ph | Ph | H |
| 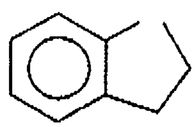 | | Ph | H |
| 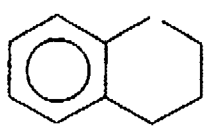 | | Ph | H |
| 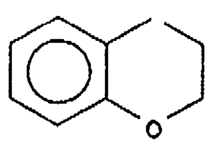 | | Ph | H |

| $R^1$ | $R^2$ | $R^3$ | $R^4$ |
|---|---|---|---|
| Me | 2,4-diF-Ph | $PhCH_2$ | H |
| Me | 2,4-diF-Ph | 4-F-Ph | H |
| Me | 2,4-diF-Ph | 3-F-Ph | H |
| Me | 2,4-diF-Ph | 3-Cl-Ph | H |
| Me | 2,4-diF-Ph | 3,4-diF-Ph | H |
| Me | 2,4-diF-Ph | 2-Me-Ph | H |
| Me | 2,4-diF-Ph | 4-Me-Ph | H |
| Me | 2,4-diF-Ph | 4-*n*-butyl-Ph | H |
| Me | 2,4-diF-Ph | 4-$NO_2$-Ph | H |
| Me | 2,4-diF-Ph | 2-cyano-Ph | H |
| Me | 2,4-diF-Ph | 3-$CF_3$-Ph | H |
| Me | 2,4-diF-Ph | 3-MeO-Ph | H |
| Me | 2,4-diF-Ph | 4-$CF_3O$-Ph | H |
| Me | 2,4-diF-Ph | 2-Me-3-Cl-Ph | H |
| Me | 2,4-diF-Ph | 2-Me-3-F-Ph | H |
| Me | 2,4-diF-Ph | 2-F-3-Cl-Ph | H |
| Me | 2,4-diF-Ph | 2,4-diMe-Ph | H |
| Me | 2,4-diF-Ph | 2-pyridyl | H |
| Me | 2,4-diF-Ph | 4-Cl-2-pyridyl | H |
| Me | 2,4-diF-Ph | Ph | Me |

TABLE 2

Compounds of Formula Ic wherein $R^1$=Me, $R^3$=Ph, $R^4$=H and:

| $R^2$ | $R^2$ |
|---|---|
| Et | 3-hydroxypropyl |
| heptyl | $HO_2C(CH_2)_7$ |
| octyl | $(MeO)_2P(=O)OCH_2(CH_2)_3CH_2$ |
| decyl | $(BuO)_2P(=S)OCH_2(CH_2)_3CH_2$ |
| undecyl | 8-($MeSO_3$)octyl |
| eicosanyl | 5-($BuSO_3$)pentyl |
| 3-Me-Bu | $(PhSO_3)CH_2CH_2$ |
| 3-Me-hexyl | 3-(4-F-$PhSO_3$)propyl |
| 2-Me-pentyl | (4-Et-$PhSO_3$)Bu$(CH_2)_4$ |
| 9,9,9-triF-nonyl | $(Me_3N^+)(CH_2)_4$ $I^-$ |
| 2,4-diCl-octyl | $(H_3N^+)(CH_2)_4$ $Cl^-$ |
| 4-(c-propyl)Bu | MeC(=O)NMe$(CH_2)_4$ |
| 8-(c-hexyl)octyl | 3-($PhSO_2$)propyl |
| 6-Ph-octyl | (2,6-diMe-$PhSO_2$)$(CH_2)_4$ |
| 4-(3-$CF_3$-Ph)Bu | 6-(2-EtO-$PhSO_2$)hexyl |
| 8-(4-$F_3$CO-Ph)octyl | 8-(PhS(O))octyl |
| 10-(2,4-diF-Ph)decyl | 7-(4-F-PhS(O))heptyl |
| 4-(4-Cl-Ph)Bu | (3-Me-PhS(O))$CH_2CH_2$ |
| 5-(4-Et-Ph)pentyl | 5-(3-BuO-PhS(O))pentyl |
| 8-(3-propyloxy-Ph)octyl | (4-pyridyl)$CH_2$ |
| 6-(4-(4-F-PhO)Ph)hexyl | $Me_2N(CH_2)_6$ |
| 7-(3-(4-Et-PhO)Ph)heptyl | 8-(2,6-diMe-4-pyridyl)octyl |
| 10-(4-(3-MeS-PhO)Ph)decyl | 10-(6-Cl-3-pyridyl)decyl |
| 2-CN-propyl | 6-(4-pyridyloxy)hexyl |
| 3-$NO_2$-propyl | 3-(2,6-diCl-4-pyridyloxy)propyl |
| 2-MeS-hexyl | (4-MeO-2-pyridyloxy)$(CH_2)_4$ |
| 2-(MeS(O))Et | 3-(2-thienyl)propyl |
| 2-($EtSO_2$)Et | 6-(3-Me-2-thienyl)hexyl |
| EtOC(=O)$(CH_2)_4$ | 8-(2-pyrimidinyl)octyl |

$R^2$

7-(4-MeO-2-pyrimidinyl)heptyl
10-(2-furanyl)decyl
(5-Et-2-furanyl) $(CH_2)_4$
3-(1-naphthalenyl)propyl
6-(6-F-2-naphthalenyl)hexyl
5-(2-pyrimidinyloxy)pentyl
8-(1-naphthalenyloxy)octyl
3-(2-tetrahydropyranyl)propyl
8-(2-tetrahydropyranyloxy) octyl
2-octenyl
2-decenyl
4-octenyl
3-EtS-2-propenyl
4-*c*-hexyloxy-2-butenyl
5-*c*-pentyloxy-3-pentenyl
6-hydroxy-4-hexenyl
3-(MeC(=O)O)-2-propenyl
3-(4-pyridyl)-2-propenyl
5-EtO-2-pentenyl
3-$CF_3$-2-propenyl
3-Ph-2-propenyl
4-PhO-2-butenyl
(Z)-(4-PhO-2-butenyl)
7-octynyl
2-hydroxy-4-butynyl
2-EtO-6-heptynyl
2,4-diF-7-octynyl
3-*c*-hexenyl

$R^2$

3-*c*-pentenyl
$(CH_2CH_2OCH_2CH_2)CH$
$(CH_2CH_2SCH_2CH_2)CH$
$(CH_2CH_2SO_2CH_2CH_2)CH$
$PhOCH_2CH_2CH_2$
PhO$(CH_2)_4$
PhO$(CH_2)_5$
PhO$(CH_2)_8$
(3-$CF_3$-PhO) $(CH_2)_3$
(3,5-diCl-PhO) $(CH_2)_6$
(2-F-PhO) $(CH_2)_3$
(2-Me-PhO) $(CH_2)_3$
(3,5-diMeO-PhO) $(CH_2)_8$
(4-(4-F-PhO)PhO) $(CH_2)_3$
(3-(3,5-diMe-PhO)PhO) $(CH_2)_6$
PhS$(CH_2)_3$
(3-$CF_3$-PhS) $(CH_2)_7$
(3-$F_3$CO-PhS) $(CH_2)_6$
(2,4-diF-PhS) $(CH_2)_5$
(4-Et-PhS) $(CH_2)_5$
(2-EtO-PhS) $(CH_2)_{10}$
(3-(4-Cl-PhO)PhS) $(CH_2)_7$
(3-(2,4-diMe-PhO)PhS) $(CH_2)_3$
(4-(4-EtO-PhO)PhS)$CH_2)_7$
(3-(2-MeS-PhO)PhS) $(CH_2)_3$
$FCH_2CH_2O(CH_2)_6$
(*c*-hexyloxy) $(CH_2)_6$
(*c*-pentyloxy) $(CH_2)_8$

$R^2$

$(CH_2=CHCH_2O)(CH_2)_7$

$CH≡CCH_2OCH_2CH_2$

$CH(=O)CH_2CH_2$

$MeC(=O)CH_2CH_2CH_2$

$BuC(=O)(CH_2)_7$

$MeC(=NMe)CH_2$

$MeC(=NOMe)CH_2CH_2CH_2$

$MeC(=NNHPh)CH_2CH_2CH_2$

hexyl

cyclopentyl

cyclohexyl

vinyl

3-hexenyl

2-cyclohexenyl

4-F-Ph

4-Cl-Ph

3-Cl-Ph

3-F-Ph

4-Br-Ph

3,4-diCl-Ph

2,3-diF-Ph

Ph

2,5-diF-Ph

3,5-diF-Ph

2,6-diF-Ph

3,4-diF-Ph

5-Cl-2-F-Ph

2-F-5-Me-Ph

$R^2$

2,5-diF-4-Me-Ph

2-F-Ph

4-Me-Ph

4-$NO_2$-Ph

4-hexyl-Ph

4-(cyclohexyl)Ph

4-$CF_3$-Ph

4-MeS-Ph

4-(hexylthio)Ph

3-MeO-Ph

4-(pentyloxy)Ph

4-$CF_3O$-Ph

4-(cyclohexyloxy)Ph

4-($BuOCH_2$)Ph

4-$MeOCH_2$-Ph

4-(2-propenyl)Ph

4-(3-pentenyl)Ph

4-($Cl_2C=CHCH_2$)Ph

4-(2-propenyloxy)Ph

4-(2-hexenyloxy)Ph

4-(propargyl)Ph

4-(2-butynyloxy)Ph

4-$BuSO_2$-Ph

4-PhO-Ph

4-(4-Cl-PhO)Ph

4-(3-Cl-PhO)Ph

4-(2,4-diF-PhO)Ph

4-(4-$NO_2$-PhO)Ph

$R^2$

4-(4-CN-PhO)Ph
4-(4-Me-PhO)Ph
4-(3-hexyl-PhO)Ph
4-(3-cyclohexyl-PhO)Ph
4-(3-$CF_3$-PhO)Ph
4-(4-MeS-PhO)Ph
4-(4-$MeSO_2$-PhO)Ph
4-(3-$BuSO_2$-PhO)Ph
4-(3-MeO-PhO)Ph
4-(3-hexyloxy-PhO)Ph
4-(4-$CF_3O$-PhO)Ph
4-(4-cyclohexyloxy-PhO)Ph
4-(4-MeOC(=O)-PhO)Ph
4-(4-allyl-PhO)Ph
4-(4-propargyl-PhO)Ph
4-((3-PhO)PhO)Ph
4-(3-(2-Cl-PhO)PhO)Ph
4-(3-(4-Me-PhO)PhO)Ph
4-(3-(3-MeO-PhO)PhO)Ph
4-PhCH=CH-Ph
4-(3-Br-Ph)CH=CH-Ph
4-(2-F-Ph)CH=CH-Ph
4-(3-Cl-Ph)CH=CH-Ph
4-(2,4-diF-Ph)CH=CH-Ph
4-(4-$NO_2$-Ph)CH=CH-Ph
4-(3-Me-Ph)CH=CH-Ph
4-(3-Bu-Ph)CH=CH-Ph
4-(4-$CF_3$-Ph)CH=CH-Ph

$R^2$

4-(2-MeS-Ph)CH=CH-Ph
4-(4-MeO-Ph)CH=CH-Ph
4-(4-BuO-Ph)CH=CH-Ph
4-$PhCH_2CH_2$-Ph
4-(3-Br-Ph)$CH_2CH_2$-Ph
4-(2-F-Ph)$CH_2CH_2$-Ph
4-(3-Cl-Ph)$CH_2CH_2$-Ph
4-(2,4-diF-Ph)$CH_2CH_2$-Ph
4-(4-$NO_2$-Ph)$CH_2CH_2$-Ph
4-(3-Me-Ph)$CH_2CH_2$-Ph
4-(3-Bu-Ph)$CH_2CH_2$-Ph
4-(4-$CF_3$-Ph)$CH_2CH_2$-Ph
4-(2-MeS-Ph)$CH_2CH_2$-Ph
4-(4-MeO-Ph)$CH_2CH_2$-Ph
4-(4-BuO-Ph)$CH_2CH_2$-Ph
4-$PhOCH_2$-Ph
4-(3-Br-Ph)$OCH_2$-Ph
4-(2-F-Ph)$OCH_2$-Ph
4-(3-Cl-Ph)$OCH_2$-Ph
4-(2,4-diF-Ph)$OCH_2$-Ph
4-(4-$NO_2$-Ph)$OCH_2$-Ph
4-(3-Me-Ph)$OCH_2$-Ph
4-(3-Bu-Ph)$OCH_2$-Ph
4-(4-$CF_3$-Ph)$OCH_2$-Ph
4-(2-MeS-Ph)$OCH_2$-Ph
4-(4-MeO-Ph)$OCH_2$-Ph
4-(4-BuO-Ph)$OCH_2$-Ph
4-$PhCH_2$-Ph

| $R^2$ | $R^2$ |
|---|---|
| 4-(3-Br-Ph)$CH_2$-Ph | 4-(4-$NO_2$-Ph)Ph |
| 4-(2-F-Ph)$CH_2$-Ph | 4-(3-Me-Ph)Ph |
| 4-(3-Cl-Ph)$CH_2$-Ph | 4-(3-Bu-Ph)Ph |
| 4-(2,4-diF-Ph)$CH_2$-Ph | 4-(4-$CF_3$-Ph)Ph |
| 4-(4-$NO_2$-Ph)$CH_2$-Ph | 4-(2-MeS-Ph)Ph |
| 4-(3-Me-Ph)$CH_2$-Ph | 4-(4-MeO-Ph)Ph |
| 4-(3-Bu-Ph)$CH_2$-Ph | 4-(4-BuO-Ph)Ph |
| 4-(4-$CF_3$-Ph)$CH_2$-Ph | 4-Ph$CH_2$O-Ph |
| 4-(2-MeS-Ph)$CH_2$-Ph | 4-(3-Br-Ph)$CH_2$O-Ph |
| 4-(4-MeO-Ph)$CH_2$-Ph | 4-(2-F-Ph)$CH_2$O-Ph |
| 4-(4-BuO-Ph)$CH_2$-Ph | 4-(3-Cl-Ph)$CH_2$O-Ph |
| 4-PhS-Ph | 4-(2,4-diF-Ph)$CH_2$O-Ph |
| 4-(3-Br-Ph)S-Ph | 4-(4-$NO_2$-Ph)$CH_2$O-Ph |
| 4-(2-F-Ph)S-Ph | 4-(3-Me-Ph)$CH_2$O-Ph |
| 4-(3-Cl-Ph)S-Ph | 4-(3-Bu-Ph)$CH_2$O-Ph |
| 4-(2,4-diF-Ph)S-Ph | 4-(4-$CF_3$-Ph)$CH_2$O-Ph |
| 4-(4-$NO_2$-Ph)S-Ph | 4-(2-MeS-Ph)$CH_2$O-Ph |
| 4-(3-Me-Ph)S-Ph | 4-(4-MeO-Ph)$CH_2$O-Ph |
| 4-(3-Bu-Ph)S-Ph | 4-(4-BuO-Ph)$CH_2$O-Ph |
| 4-(4-$CF_3$-Ph)S-Ph | 4-(4-CN-Ph)$CH_2$O-Ph |
| 4-(2-MeS-Ph)S-Ph | 4-(3-PhO-Ph)$CH_2$O-Ph |
| 4-(4-MeO-Ph)S-Ph | 4-[3-(3-F-PhO)Ph]$CH_2$O-Ph |
| 4-(4-BuO-Ph)S-Ph | 4-[4-(2-$NO_2$-PhO)Ph]$CH_2$O-Ph |
| 4-Ph-Ph | 4-[3-(4-Me-PhO)Ph]$CH_2$O-Ph |
| 4-(3-Br-Ph)Ph | 4-[4-(4-Bu-PhO)Ph]$CH_2$O-Ph |
| 4-(2-F-Ph)Ph | 4-[3-(3-$CF_3$-PhO)Ph]$CH_2$O-Ph |
| 4-(3-Cl-Ph)Ph | 4-[2-(3-MeS-PhO)Ph]$CH_2$O-Ph |
| 4-(2,4-diF-Ph)Ph | 4-[4-(4-MeO-PhO)Ph]$CH_2$O-Ph |

$B^2$

4-[3-(3-BuO-PhO)Ph]$CH_2$O-Ph
4-(2-F-4-MeO-Ph)$CH_2$O-Ph
3-(2-$CF_3$-4-Et-Ph)$CH_2$O-Ph
4-[MeNHC(=O)O]Ph
4-[octyl-NHC(=O)O]Ph
4-[PhNHC(=O)O]Ph
4-[(3-Br-Ph)NHC(=O)O]Ph
4-[(2-F-Ph)NHC(=O)O]Ph
4-[(3-Cl-Ph)NHC(=O)O]Ph
4-[(2,4-diF-Ph)NHC(=O)O]Ph
4-[(4-$NO_2$-Ph)NHC(=O)O]Ph
4-[(3-Me-Ph)NHC(=O)O]Ph
4-[(3-Bu-Ph)NHC(=O)O]Ph
4-[(4-$CF_3$-Ph)NHC(=O)O]Ph
4-[(2-MeS-Ph)NHC(=O)O]Ph
4-[(4-MeO-Ph)NHC(=O)O]Ph
4-[(4-BuO-Ph)NHC(=O)O]Ph
4-[(4-CN-Ph)NHC(=O)O]Ph
4-[(3-PhO-Ph)NHC(=O)O]Ph
4-[3-(3-F-PhO)Ph-NHC(=O)O]Ph
4-[4-(2-$NO_2$-PhO)Ph-NHC(=O)O]Ph
4-[2-(4-Me-PhO)Ph-NHC(=O)O]Ph
4-[4-(4-Bu-PhO)Ph-NHC(=O)O]Ph
4-[4-(3-$CF_3$-PhO)Ph-NHC(=O)O]Ph
4-[4-(3-MeS-PhO)Ph-NHC(=O)O]Ph
4-[3-(4-MeO-PhO)Ph-NHC(=O)O]Ph
4-[3-(3-BuO-PhO)Ph-NHC(=O)O]Ph
4-[(2-F-4-MeO-Ph)NHC(=O)O]Ph

$B^2$

3-[(2-$CF_3$-4-Et-Ph)NHC(=O)O]Ph
4-[(3-pyridyl)NHC(=O)O]Ph
4-[(3-Br-2-pyridyl)NHC(=O)O]Ph
4-[(2-F-3-pyridyl)NHC(=O)O]Ph
4-[(3-Cl-4-pyridyl)NHC(=O)O]Ph
4-[(2,4-diF-3-pyridyl)NHC(=O)O]Ph
4-[(4-$NO_2$-2-pyridyl)NHC(=O)O]Ph
4-[(3-Me-2-pyridyl)NHC(=O)O]Ph
4-[(3-Bu-2-pyridyl)NHC(=O)O]Ph
4-[(4-$CF_3$-3-pyridyl)NHC(=O)O]Ph
4-[(2-MeS-4-pyridyl)NHC(=O)O]Ph
4-[(4-MeO-3-pyridyl)NHC(=O)O]Ph
4-[(4-BuO-3-pyridyl)NHC(=O)O]Ph
4-[(4-CN-2-pyridyl)NHC(=O)O]Ph
4-[(3-PhO-2-pyridyl)NHC(=O)O]Ph
4-[3-(3-F-PhO)-2-pyridyl-NHC(=O)O]Ph
4-[4-(2-$NO_2$-PhO)-3-pyridyl-NHC(=O)O]Ph
4-[2-(4-Me-PhO)-4-pyridyl-NHC(=O)O]Ph
4-[4-(4-Bu-PhO)-3-pyridyl-NHC(=O)O]Ph
4-[4-(3-$CF_3$-PhO)-2-pyridyl-NHC(=O)O]Ph
4-[4-(3-MeS-PhO)-3-pyridyl-NHC(=O)O]Ph
4-[3-(4-MeO-PhO)-2-pyridyl-NHC(=O)O]Ph
4-[3-(3-BuO-PhO)-2-pyridyl-NHC(=O)O]Ph
4-[(2-F-4-MeO-3-pyridyl)NHC(=O)O]Ph
3-[(2-$CF_3$-4-Et-3-pyridyl)NHC(=O)O]Ph
4-[MeO-C(=O)]Ph
4-[octyloxy-C(=O)]Ph
4-[PhO-C(=O)]Ph

$R^2$

4-[3-Br-PhO-C(=O)]Ph

4-[2-F-PhO-C(=O)]Ph

4-[3-Cl-PhO-C(=O)]Ph

4-[2,4-diF-PhO-C(=O)]Ph

4-[4-$NO_2$-PhO-(=O)]Ph

4-[3-Me-PhO-C(=O)]Ph

4-[3-Bu-PhO-C(=O)]Ph

4-[4-$CF_3$-PhO-C(=O)]Ph

4-[2-MeS-PhO-C(=O)]Ph

4-[4-MeO-PhO-C(=O)]Ph

4-[4-BuO-PhO-C(=O)]Ph

4-[4-CN-PhO-C(=O)]Ph

4-[3-PhO-PhO-C(=O)]Ph

4-[3-(3-F-PhO)PhO-C(=O)]Ph

4-[4-(2-$NO_2$-PhO)PhO-C(=O)]Ph

4-[2-(4-Me-PhO)PhO-C(=O)]Ph

4-[4-(4-Bu-PhO)PhO-C(=O)]Ph

4-[4-(3-$CF_3$-PhO)PhO-C(=O)]Ph

4-[4-(3-MeS-PhO)PhO-C(=O)]Ph

4-[3-(4-MeO-PhO)PhO-C(=O)]Ph

4-[3-(3-BuO-PhO)PhO-C(=O)]Ph

4-[(2-F-4-MeO-PhO-C(=O)]Ph

3-[(2-$CF_3$-4-Et-PhO-C(=O)]Ph

4-[(3-pyridyloxy)C(=O)]Ph

4-[(3-Br-2-pyridyloxy)C(=O)]Ph

4-[(2-F-3-pyridyloxy)C(=O)]Ph

4-[(3-Cl-4-pyridyloxy)C(=O)]Ph

4-[(2,4-diF-3-pyridyloxy)C(=O)]Ph

$R^2$

4-[(4-$NO_2$-2-pyridyloxy)C(=O)]Ph
4-[(3-Me-2-pyridyloxy)C(=O)]Ph
4-[(3-Bu-2-pyridyloxy)C(=O)]Ph
4-[(4-$CF_3$-3-pyridyloxy)C(=O)]Ph
4-[(2-MeS-4-pyridyloxy)C(=O)]Ph
4-[(4-MeO-3-pyridyloxy)C(=O)]Ph
4-[(4-BuO-3-pyridyloxy)C(=O)]Ph
4-[(4-CN-2-pyridyloxy)C(=O)]Ph
4-[(3-PhO-2-pyridyloxy)C(=O)]Ph
4-[3-(3-F-PhO)-2-pyridyloxy-C(=O)]Ph
4-[4-(2-$NO_2$-PhO)-3-pyridyloxy-C(=O)]Ph
4-[2-(4-Me-PhO)-4-pyridyloxy-C(=O)]Ph
4-[4-(4-Bu-PhO)-3-pyridyloxy-C(=O)]Ph
4-[4-(3-$CF_3$-PhO)-2-pyridyloxy-C(=O)]Ph
4-[4-(3-MeS-PhO)-3-pyridyloxy-C(=O)]Ph
4-[3-(4-MeO-PhO)-2-pyridyloxy-C(=O)]Ph
4-[3-(3-BuO-PhO)-2-pyridyloxy-C(=O)]Ph
4-[(2-F-4-MeO-3-pyridyloxy)C(=O)]Ph
3-[(2-$CF_3$-4-Et-3-pyridyloxy)C(=O)]Ph
4-[MeC(=O)O]Ph
4-[octyl-C(=O)O]Ph
4-[PhC(=O)O]Ph
4-[3-Br-PhC(=O)O]Ph
4-[2-F-PhC(=O)O]Ph
4-[3-Cl-PhC(=O)O]Ph
4-[2,4-diF-PhC(=O)O]Ph
4-[4-$NO_2$-Ph(=O)O]Ph
4-[3-Me-PhC(=O)O]Ph

$R^2$

4-[3-Bu-PhC(=O)O]Ph
4-[4-$CF_3$-PhC(=O)O]Ph
4-[2-MeS-PhC(=O)O]Ph
4-[4-MeO-PhC(=O)O]Ph
4-[4-BuO-PhC(=O)O]Ph
4-[4-CN-PhC(=O)O]Ph
4-[3-PhO-PhC(=O)O]Ph
4-[3-(3-F-PhO)PhC(=O)O]Ph
4-[4-(2-$NO_2$-PhO)PhC(=O)O]Ph
4-[2-(4-Me-PhO)PhC(=O)O]Ph
4-[4-(4-Bu-PhO)PhC(=O)O]Ph
4-[4-(3-$CF_3$-PhO)PhC(=O)O]Ph
4-[4-(3-MeS-PhO)PhC(=O)O]Ph
4-[3-(4-MeO-PhO)PhC(=O)O]Ph
4-[3-(3-BuO-PhO)PhC(=O)O]Ph
4-[(2-F-4-MeO-Ph)C(=O)O]Ph
3-[(2-$CF_3$-4-Et-Ph)C(=O)O]Ph
4-[(3-pyridyl)C(=O)O]Ph
4-[(3-Br-2-pyridyl)C(=O)O]Ph
4-[(2-F-3-pyridyl)C(=O)O]Ph
4-[(3-Cl-4-pyridyl)C(=O)O]Ph
4-[(2,4-diF-3-pyridyl)C(=O)O]Ph
4-[(4-$NO_2$-2-pyridyl)C(=O)O]Ph
4-[(3-Me-2-pyridyl)C(=O)O]Ph
4-[(3-Bu-2-pyridyl)C(=O)O]Ph
4-[(4-$CF_3$-3-pyridyl)C(=O)O]Ph
4-[(2-MeS-4-pyridyl)C(=O)O]Ph
4-[(4-MeO-3-pyridyl)C(=O)O]Ph

$R^2$

4-[(4-BuO-3-pyridyl)C(=O)O]Ph
4-[(4-CN-2-pyridyl)C(=O)O]Ph
4-[(3-PhO-2-pyridyl)C(=O)O]Ph
4-[3-(3-F-PhO)-2-pyridyl-C(=O)O]Ph
4-[4-(2-NO$_2$-PhO)-3-pyridyl-C(=O)O]Ph
4-[2-(4-Me-PhO)-4-pyridyl-C(=O)O]Ph
4-[4-(4-Bu-PhO)-3-pyridyl-C(=O)O]Ph
4-[4-(3-CF$_3$-PhO)-2-pyridyl-C(=O)O]Ph
4-[4-(3-MeS-PhO)-3-pyridyl-C(=O)O]Ph
4-[3-(4-MeO-PhO)-2-pyridyl-C(=O)O]Ph
4-[3-(3-BuO-PhO)-2-pyridyl-C(=O)O]Ph
4-[(2-F-4-MeO-3-pyridyl)C(=O)O]Ph
3-[(2-CF$_3$-4-Et-3-pyridyl)C(=O)O]Ph
2-F-4-PhCH$_2$CH$_2$-Ph
3-Cl-4-(2-F-Ph)OCH$_2$-Ph
3-Me-4-(4-MeO-Ph)CH$_2$-Ph
2-F-4-PhS-Ph
3-BuO-4-Ph-Ph
3-MeS-4-PhO-Ph
2-F-4-PhCH$_2$O-Ph
2-F-4-[octyl-NHC(=O)O]Ph
4-F-3-thienyl
4-Cl-2-thienyl
5-Cl-2-thienyl
3-F-2-thienyl
4-Br-2-thienyl
3,4-diCl-2-thienyl
2,4-diF-3-thienyl

$R^2$

2-thienyl
3-thienyl
2,5-diF-3-thienyl
3,5-diF-2-thienyl
3,4-diF-2-thienyl
5-Cl-2-F-3-thienyl
2-F-5-Me-3-thienyl
2,5-diF-4-Me-3-thienyl
2-F-3-thienyl
4-Me-2-thienyl
4-NO$_2$-2-thienyl
4-hexyl-2-thienyl
5-cyclohexyl-3-thienyl
5-CF$_3$-3-thienyl
4-MeS-2-thienyl
4-hexylthio-2-thienyl
5-MeO-3-thienyl
4-pentyloxy-2-thienyl
4-CF$_3$O-2-thienyl
5-cyclohexyloxy-3-thienyl
4-BuOCH$_2$-2-thienyl
4-MeOCH$_2$-2-thienyl
5-(2-propenyl)-3-thienyl
5-(3-pentenyl)-3-thienyl
4-(Cl$_2$C=CHCH$_2$)-2-thienyl
4-(2-propenyloxy)-2-thienyl
4-(2-hexenyloxy)-2-thienyl
5-(propargyl)-3-thienyl

| $R^2$ | $R^2$ |
|---|---|
| 4-(2-butynyloxy)-2-thienyl | 4-(2-F-Ph)CH=CH-2-thienyl |
| 5-$BuSO_2$-3-thienyl | 4-(3-Cl-Ph)CH=CH-2-thienyl |
| 5-PhO-3-thienyl | 5-(2,4-diF-Ph)CH=CH-3-thienyl |
| 4-(4-Cl-PhO)-2-thienyl | 5-(4-$NO_2$-Ph)CH=CH-3-thienyl |
| 4-(3-Cl-PhO)-2-thienyl | 5-(3-Me-Ph)CH=CH-3-thienyl |
| 5-(2,4-diF-PhO)-3-thienyl | 5-(3-Bu-Ph)CH=CH-3-thienyl |
| 5-(4-$NO_2$-PhO)-3-thienyl | 4-(4-$CF_3$-Ph)CH=CH-2-thienyl |
| 5-(4-CN-PhO)-3-thienyl | 4-(2-MeS-Ph)CH=CH-2-thienyl |
| 5-(4-Me-PhO)-3-thienyl | 4-(4-MeO-Ph)CH=CH-2-thienyl |
| 4-(3-hexyl-PhO)-2-thienyl | 4-(4-BuO-Ph)CH=CH-2-thienyl |
| 4-(3-cyclohexyl-PhO)-2-thienyl | 5-$PhCH_2CH_2$-3-thienyl |
| 4-(3-$CF_3$-PhO)-2-thienyl | 5-(3-Br-Ph)$CH_2CH_2$-3-thienyl |
| 4-(4-MeS-PhO)-2-thienyl | 4-(2-F-Ph)$CH_2CH_2$-2-thienyl |
| 5-(4-$MeSO_2$-PhO)-3-thienyl | 4-(3-Cl-Ph)$CH_2CH_2$-2-thienyl |
| 5-(3-$BuSO_2$-PhO)-3-thienyl | 4-(2,4-diF-Ph)$CH_2CH_2$-2-thienyl |
| 5-(3-MeO-PhO)-3-thienyl | 5-(4-$NO_2$-Ph)$CH_2CH_2$-3-thienyl |
| 5-(3-hexyloxy-PhO)-3-thienyl | 5-(3-Me-Ph)$CH_2CH_2$-3-thienyl |
| 5-(4-$CF_3$O-PhO)-3-thienyl | 4-(3-Bu-Ph)$CH_2CH_2$-2-thienyl |
| 5-(4-cyclohexyloxy-PhO)-3-thienyl | 5-(4-$CF_3$-Ph)$CH_2CH_2$-3-thienyl |
| 4-(4-MeOC(=O)-PhO)-2-thienyl | 4-(2-MeS-Ph)$CH_2CH_2$-2-thienyl |
| 4-(4-allyl-PhO)-2-thienyl | 5-(4-MeO-Ph)$CH_2CH_2$-3-thienyl |
| 4-(4-propargyl-PhO)-2-thienyl | 4-(4-BuO-Ph)$CH_2CH_2$-2-thienyl |
| 5-((3-PhO)PhO)-3-thienyl | 4-$PhOCH_2$-2-thienyl |
| 5-(3-(2-Cl-PhO)PhO)-3-thienyl | 5-(3-Br-Ph)$OCH_2$-3-thienyl |
| 5-(3-(4-Me-PhO)PhO)-3-thienyl | 5-(2-F-Ph)$OCH_2$-3-thienyl |
| 5-(3-(3-MeO-PhO)PhO)-3-thienyl | 4-(3-Cl-Ph)$OCH_2$-2-thienyl |
| 4-PhCH=CH-2-thienyl | 5-(2,4-diF-Ph)$OCH_2$-3-thienyl |
| 4-(3-Br-Ph)CH=CH-2-thienyl | 5-(4-$NO_2$-Ph)$OCH_2$-3-thienyl |

| $R^2$ | $R^2$ |
|---|---|
| 4-(3-Me-Ph)$OCH_2$-2-thienyl | 5-(4-MeO-Ph)S-3-thienyl |
| 5-(3-Bu-Ph)$OCH_2$-3-thienyl | 5-(4-BuO-Ph)S-3-thienyl |
| 5-(4-$CF_3$-Ph)$OCH_2$-3-thienyl | 4-Ph-2-thienyl |
| 4-(2-MeS-Ph)$OCH_2$-2-thienyl | 4-(3-Br-Ph)-2-thienyl |
| 5-(4-MeO-Ph)$OCH_2$-3-thienyl | 4-(2-F-Ph)-2-thienyl |
| 4-(4-BuO-Ph)$OCH_2$-2-thienyl | 5-(3-Cl-Ph)-3-thienyl |
| 5-Ph$CH_2$-3-thienyl | 5-(2,4-diF-Ph)-3-thienyl |
| 5-(3-Br-Ph)$CH_2$-3-thienyl | 5-(4-$NO_2$-Ph)-3-thienyl |
| 5-(2-F-Ph)$CH_2$-3-thienyl | 4-(3-Me-Ph)-2-thienyl |
| 5-(3-Cl-Ph)$CH_2$-3-thienyl | 5-(3-Bu-Ph)-3-thienyl |
| 4-(2,4-diF-Ph)$CH_2$-2-thienyl | 4-(4-$CF_3$-Ph)-2-thienyl |
| 4-(4-$NO_2$-Ph)$CH_2$-2-thienyl | 5-(2-MeS-Ph)-3-thienyl |
| 5-(3-Me-Ph)$CH_2$-3-thienylPh | 5-(4-MeO-Ph)-3-thienyl |
| 4-(3-Bu-Ph)$CH_2$-2-thienyl | 4-(4-BuO-Ph)-2-thienyl |
| 4-(4-$CF_3$-Ph)$CH_2$-2-thienyl | 5-Ph$CH_2O$-3-thienyl |
| 5-(2-MeS-Ph)$CH_2$-3-thienyl | 4-(3-Br-Ph)$CH_2O$-2-thienyl |
| 4-(4-MeO-Ph)$CH_2$-2-thienyl | 5-(2-F-Ph)$CH_2O$-3-thienyl |
| 4-(4-BuO-Ph)$CH_2$-2-thienyl | 5-(3-Cl-Ph)$CH_2O$-3-thienyl |
| 5-PhS-3-thienyl | 5-(2,4-diF-Ph)$CH_2O$-3-thienyl |
| 4-(3-Br-Ph)S-2-thienyl | 4-(4-$NO_2$-Ph)$CH_2O$-2-thienyl |
| 5-(2-F-Ph)S-3-thienyl | 5-(3-Me-Ph)$CH_2O$-3-thienyl |
| 4-(3-Cl-Ph)S-2-thienyl | 4-(3-Bu-Ph)$CH_2O$-2-thienyl |
| 5-(2,4-diF-Ph)S-3-thienyl | 4-(4-$CF_3$-Ph)$CH_2O$-2-thienyl |
| 4-(4-$NO_2$-Ph)S-2-thienyl | 5-(2-MeS-Ph)$CH_2O$-3-thienyl |
| 5-(3-Me-Ph)S-3-thienyl | 4-(4-MeO-Ph)$CH_2O$-2-thienyl |
| 4-(3-Bu-Ph)S-2-thienyl | 5-(4-BuO-Ph)$CH_2O$-3-thienyl |
| 5-(4-$CF_3$-Ph)S-3-thienyl | 4-(4-CN-Ph)$CH_2O$-2-thienyl |
| 4-(2-MeS-Ph)S-2-thienyl | 5-(3-PhO-Ph)$CH_2O$-3-thienyl |

$B^2$

5-[3-(3-F-PhO)Ph]$CH_2$O-3-thienyl

4-[4-(2-$NO_2$-PhO)Ph]$CH_2$O-2-thienyl

4-[3-(4-Me-PhO)Ph]$CH_2$O-2-thienyl

4-[4-(4-Bu-PhO)Ph]$CH_2$O-2-thienyl

5-[3-(3-$CF_3$-PhO)Ph]$CH_2$O-3-thienyl

5-[2-(3-MeS-PhO)Ph]$CH_2$O-3-thienyl

4-[4-(4-MeO-PhO)Ph]$CH_2$O-2-thienyl

5-[3-(3-BuO-PhO)Ph]$CH_2$O-3-thienyl

4-(2-F-4-MeO-Ph)$CH_2$O-2-thienyl

5-(2-$CF_3$-4-Et-Ph)$CH_2$O-3-thienyl

4-[MeNHC(=O)O]-2-thienyl

4-[octyl-NHC(=O)O]-2-thienyl

5-[PhNHC(=O)O]-3-thienyl

5-[(3-Br-Ph)NHC(=O)O]-3-thienyl

4-[(2-F-Ph)NHC(=O)O]-2-thienyl

5-[(3-Cl-Ph)NHC(=O)O]-3-thienyl

5-[(2,4-diF-Ph)NHC(=O)O]-3-thienyl

5-[(4-$NO_2$-Ph)NHC(=O)O]-3-thienyl

4-[(3-Me-Ph)NHC(=O)O]-2-thienyl

5-[(3-Bu-Ph)NHC(=O)O]-3-thienyl

5-[(4-$CF_3$-Ph)NHC(=O)O]-3-thienyl

4-[(2-MeS-Ph)NHC(=O)O]-2-thienyl

4-[(4-MeO-Ph)NHC(=O)O]-2-thienyl

5-[(4-BuO-Ph)NHC(=O)O]-3-thienyl

5-[(4-CN-Ph)NHC(=O)O]-3-thienyl

4-[(3-PhO-Ph)NHC(=O)O]-2-thienyl

4-[3-(3-F-PhO)Ph-NHC(=O)O]-2-thienyl

4-[4-(2-$NO_2$-PhO)Ph-NHC(=O)O]-2-thienyl

$R^2$

4-[2-(4-Me-PhO)Ph-NHC(=O)O]-2-thienyl

5-[4-(4-Bu-PhO)Ph-NHC(=O)O]-3-thienyl

5-[4-(3-$CF_3$-PhO)Ph-NHC(=O)O]-3-thienyl

5-[4-(3-MeS-PhO)Ph-NHC(=O)O]-3-thienyl

4-[3-(4-MeO-PhO)Ph-NHC(=O)O]-2-thienyl

5-[3-(3-BuO-PhO)Ph-NHC(=O)O]-3-thienyl

4-[(2-F-4-MeO-Ph)NHC(=O)O]-2-thienyl

5-[(2-$CF_3$-4-Et-Ph)NHC(=O)O]-3-thienyl

5-[(3-pyridyl)NHC(=O)O]-3-thienyl

4-[(3-Br-2-pyridyl)NHC(=O)O]-2-thienyl

5-[(2-F-3-pyridyl)NHC(=O)O]-3-thienyl

5-[(3-Cl-4-pyridyl)NHC(=O)O]-3-thienyl

5-[(2,4-diF-3-pyridyl)NHC(=O)O]-3-thienyl

4-[(4-$NO_2$-2-pyridyl)NHC(=O)O]-2-thienyl

4-[(3-Me-2-pyridyl)NHC(=O)O]-2-thienyl

5-[(3-Bu-2-pyridyl)NHC(=O)O]-3-thienyl

5-[(4-$CF_3$-3-pyridyl)NHC(=O)O]-3-thienyl

4-[(2-MeS-4-pyridyl)NHC(=O)O]-2-thienyl

5-[(4-MeO-3-pyridyl)NHC(=O)O]-3-thienyl

4-[(4-BuO-3-pyridyl)NHC(=O)O]-2-thienyl

5-[(4-CN-2-pyridyl)NHC(=O)O]-3-thienyl

4-[(3-PhO-2-pyridyl)NHC(=O)O]-2-thienyl

4-[3-(3-F-PhO)-2-pyridyl-NHC(=O)O]-2-thienyl

5-[4-(2-$NO_2$-PhO)-3-pyridyl-NHC(=O)O]-3-thienyl

4-[2-(4-Me-PhO)-4-pyridyl-NHC(=O)O]-2-thienyl

5-[4-(4-Bu-PhO)-3-pyridyl-NHC(=O)O]-3-thienyl

4-[4-(3-$CF_3$-PhO)-2-pyridyl-NHC(=O)O]-2-thienyl

5-[4-(3-MeS-PhO)-3-pyridyl-NHC(=O)O]-3-thienyl

$R^2$

4-[3-(4-MeO-PhO)-2-pyridyl-NHC(=O)O]-2-thienyl

5-[3-(3-BuO-PhO)-2-pyridyl-NHC(=O)O]-3-thienyl

5-[(2-F-4-MeO-3-pyridyl)NHC(=O)O]-3-thienyl

4-[(2-$CF_3$-4-Et-3-pyridyl)NHC(=O)O]-2-thienyl

4-[MeO-C(=O)]-2-thienyl

4-[octyloxy-C(=O)]-2-thienyl

5-[PhO-C(=O)]-3-thienyl

5-[3-Br-PhO-C(=O)]-3-thienyl

4-[2-F-PhO-C(=O)]-2-thienyl

4-[3-Cl-PhO-C(=O)]-2-thienyl

5-[2,4-diF-PhO-C(=O)]-3-thienyl

5-[4-$NO_2$-PhO-(=O)]-3-thienyl

4-[3-Me-PhO-C(=O)]-2-thienyl

4-[3-Bu-PhO-C(=O)]-2-thienyl

5-[4-$CF_3$-PhO-C(=O)]-3-thienyl

5-[2-MeS-PhO-C(=O)]-3-thienyl

4-[4-MeO-PhO-C(=O)]-2-thienyl

4-[4-BuO-PhO-C(=O)]-2-thienyl

5-[4-CN-PhO-C(=O)]-3-thienyl

5-[3-PhO-PhO-C(=O)]-3-thienyl

4-[3-(3-F-PhO)PhO-C(=O)]-2-thienyl

5-[4-(2-$NO_2$-PhO)PhO-C(=O)]-3-thienyl

4-[2-(4-Me-PhO)PhO-C(=O)]-2-thienyl

5-[4-(4-Bu-PhO)PhO-C(=O)]-3-thienyl

4-[4-(3-$CF_3$-PhO)PhO-C(=O)]-2-thienyl

5-[4-(3-MeS-PhO)PhO-C(=O)]-3-thienyl

4-[3-(4-MeO-PhO)PhO-C(=O)]-2-thienyl

5-[3-(3-BuO-PhO)PhO-C(=O)]-3-thienyl

$R^2$

4-[(2-F-4-MeO-PhO-C(=O)]-2-thienyl

5-[(2-$CF_3$-4-Et-PhO-C(=O)]-3-thienyl

5-[(3-pyridyloxy)C(=O)]-3-thienyl

4-[(3-Br-2-pyridyloxy)C(=O)]-2-thienyl

5-[(2-F-3-pyridyloxy)C(=O)]-3-thienyl

5-[(3-Cl-4-pyridyloxy)C(=O)]-3-thienyl

4-[(2,4-diF-3-pyridyloxy)C(=O)]-2-thienyl

4-[(4-$NO_2$-2-pyridyloxy)C(=O)]-2-thienyl

5-[(3-Me-2-pyridyloxy)C(=O)]-3-thienyl

5-[(3-Bu-2-pyridyloxy)C(=O)]-3-thienyl

5-[(4-$CF_3$-3-pyridyloxy)C(=O)]-3-thienyl

5-[(2-MeS-4-pyridyloxy)C(=O)]-3-thienyl

5-[(4-MeO-3-pyridyloxy)C(=O)]-3-thienyl

4-[(4-BuO-3-pyridyloxy)C(=O)]-2-thienyl

4-[(4-CN-2-pyridyloxy)C(=O)]-2-thienyl

4-[(3-PhO-2-pyridyloxy)C(=O)]-2-thienyl

5-[3-(3-F-PhO)-2-pyridyloxy-C(=O)]-3-thienyl

5-[4-(2-$NO_2$-PhO)-3-pyridyloxy-C(=O)]-3-thienyl

5-[2-(4-Me-PhO)-4-pyridyloxy-C(=O)]-3-thienyl

5-[4-(4-Bu-PhO)-3-pyridyloxy-C(=O)]-3-thienyl

4-[4-(3-$CF_3$-PhO)-2-pyridyloxy-C(=O)]-2-thienyl

4-[4-(3-MeS-PhO)-3-pyridyloxy-C(=O)]-2-thienyl

4-[3-(4-MeO-PhO)-2-pyridyloxy-C(=O)]-2-thienylPh

5-[3-(3-BuO-PhO)-2-pyridyloxy-C(=O)]-3-thienyl

4-[(2-F-4-MeO-3-pyridyloxy)C(=O)]-2-thienyl

4-[(2-$CF_3$-4-Et-3-pyridyloxy)C(=O)]-2-thienyl

4-[MeC(=O)O]-2-thienyl

4-[octyl-C(=O)O]-2-thienyl

$R^2$

5-[PhC(=O)O]-3-thienyl

4-[3-Br-PhC(=O)O]-2-thienyl

5-[2-F-PhC(=O)O]-3-thienyl

5-[3-Cl-PhC(=O)O]-3-thienyl

4-[2,4-diF-PhC(=O)O]-2-thienyl

5-[4-$NO_2$-Ph(=O)O]-3-thienyl

4-[3-Me-PhC(=O)O]-2-thienyl

5-[3-Bu-PhC(=O)O]-3-thienyl

4-[4-$CF_3$-PhC(=O)O]-2-thienyl

4-[2-MeS-PhC(=O)O]-2-thienyl

5-[4-MeO-PhC(=O)O]-3-thienyl

5-[4-BuO-PhC(=O)O]-3-thienyl

4-[4-CN-PhC(=O)O]-2-thienyl

4-[3-PhO-PhC(=O)O]-2-thienyl

5-[3-(3-F-PhO)PhC(=O)O]-3-thienyl

4-[4-(2-$NO_2$-PhO)PhC(=O)O]-2-thienyl

5-[2-(4-Me-PhO)PhC(=O)O]-3-thienyl

4-[4-(4-Bu-PhO)PhC(=O)O]-2-thienyl

5-[4-(3-$CF_3$-PhO)PhC(=O)O]-3-thienyl

4-[4-(3-MeS-PhO)PhC(=O)O]-2-thienyl

5-[3-(4-MeO-PhO)PhC(=O)O]-3-thienyl

5-[3-(3-BuO-PhO)PhC(=O)O]-3-thienyl

4-[(2-F-4-MeO-Ph)C(=O)O]-2-thienyl

5-[(2-$CF_3$-4-Et-Ph)C(=O)O]-3-thienyl

5-[(3-pyridyl)C(=O)O]-3-thienyl

4-[(3-Br-2-pyridyl)C(=O)O]-2-thienyl

4-[(2-F-3-pyridyl)C(=O)O]-2-thienyl

5-[(3-Cl-4-pyridyl)C(=O)O]-3-thienyl

$R^2$

5-[(2,4-diF-3-pyridyl)C(=O)O]-3-thienyl

5-[(4-$NO_2$-2-pyridyl)C(=O)O]-3-thienyl

5-[(3-Me-2-pyridyl)C(=O)O]-3-thienyl

5-[(3-Bu-2-pyridyl)C(=O)O]-3-thienyl

4-[(4-$CF_3$-3-pyridyl)C(=O)O]-2-thienyl

5-[(2-MeS-4-pyridyl)C(=O)O]-3-thienyl

5-[(4-MeO-3-pyridyl)C(=O)O]-3-thienyl

4-[(4-BuO-3-pyridyl)C(=O)O]-2-thienyl

4-[(4-CN-2-pyridyl)C(=O)O]-2-thienyl

5-[(3-PhO-2-pyridyl)C(=O)O]-3-thienyl

5-[3-(3-F-PhO)-2-pyridyl-C(=O)O]-3-thienyl

5-[4-(2-$NO_2$-PhO)-3-pyridyl-C(=O)O]-3-thienyl

4-[2-(4-Me-PhO)-4-pyridyl-C(=O)O]-2-thienyl

5-[4-(4-Bu-PhO)-3-pyridyl-C(=O)O]-3-thienyl

5-[4-(3-$CF_3$-PhO)-2-pyridyl-C(=O)O]-3-thienyl

5-[4-(3-MeS-PhO)-3-pyridyl-C(=O)O]-3-thienyl

4-[3-(4-MeO-PhO)-2-pyridyl-C(=O)O]-2-thienyl

4-[3-(3-BuO-PhO)-2-pyridyl-C(=O)O]-2-thienyl

5-[(2-F-4-MeO-3-pyridyl)C(=O)O]-3-thienyl

4-[(2-$CF_3$-4-Et-3-pyridyl)C(=O)O]-2-thienyl

3-F-4-$PhCH_2CH_2$-2-thienyl

3-F-4-(2-F-Ph)$OCH_2$-2-thienyl

4-F-5-(4-MeO-Ph)$CH_2$-3-thienyl

2-F-5-PhS-3-thienyl

5-MeS-4-PhO-2-thienyl

2-F-5-$PhCH_2O$-3-thienyl

2-F-5-[octyl-NHC(=O)O]-3-thienyl

| $B^2$ | $B^2$ |
|---|---|
| 4-F-3-pyridyl | 6-(2-propenyl)-3-pyridyl |
| 4-Cl-3-pyridyl | 5-(3-pentenyl)-2-pyridyl |
| 3-F-2-pyridyl | 4-($Cl_2C$=$CHCH_2$)-2-pyridyl |
| 4-Br-3-pyridyl | 4-(2-propenyloxy)-2-pyridyl |
| 2,3-diF-4-pyridyl | 6-(2-hexenyloxy)-3-pyridyl |
| 2-pyridyl | 6-(propargyl)-3-pyridyl |
| 3-pyridyl | 2-(2-butynyloxy)-4-pyridyl |
| 4-pyridyl | 4-$BuSO_2$-2-pyridyl |
| 2,5-diF-3-pyridyl | 6-PhO-3-pyridyl |
| 3,5-diF-4-pyridyl | 6-(4-Cl-PhO)-3-pyridyl |
| 2,6-diF-3-pyridyl | 6-(3-Cl-PhO)-3-pyridyl |
| 3,4-diF-2-pyridyl | 5-(2,4-diF-PhO)-2-pyridyl |
| 5-Cl-2-F-3-pyridyl | 5-(4-$NO_2$-PhO)-3-pyridyl |
| 2-F-5-Me-3-pyridyl | 2-(4-CN-PhO)-4-pyridyl |
| 2-F-4-pyridyl | 5-(4-Me-PhO)-2-pyridyl |
| 4-Me-2-pyridyl | 6-(3-hexyl-PhO)-3-pyridyl |
| 4-$NO_2$-2-pyridyl | 6-(3-cyclohexyl-PhO)-3-pyridyl |
| 5-hexyl-3-pyridyl | 4-(3-$CF_3$-PhO)-2-pyridyl |
| 5-(cyclohexyl)-3-pyridyl | 4-(4-MeS-PhO)-2-pyridyl |
| 5-$CF_3$-3-pyridyl | 5-(4-$MeSO_2$-PhO)-2-pyridyl |
| 2-MeS-4-pyridyl | 6-(3-$BuSO_2$-PhO)-3-pyridyl |
| 4-hexylthio-2-pyridyl | 6-(3-MeO-PhO)-3-pyridyl |
| 5-MeO-4-pyridyl | 6-(3-hexyloxy-PhO)-3-pyridyl |
| 6-pentyloxy-3-pyridyl | 6-(4-$CF_3O$-PhO)-3-pyridyl |
| 6-$CF_3O$-3-pyridyl | 5-(4-cyclohexyloxy-PhO)-2-pyridyl |
| 6-cyclohexyloxy-3-pyridyl | 5-(4-MeOC(=O)-PhO)-2-pyridyl |
| 6-($BuOCH_2$)-3-pyridyl | 5-(4-allyl-PhO)-3-pyridyl |
| 5-$MeOCH_2$-2-pyridyl | 6-(4-propargyl-PhO)-3-pyridyl |

| $R^2$ |
|---|
| 6-((3-PhO)PhO)-3-pyridyl |
| 2-(3-(2-Cl-PhO)PhO)-4-pyridyl |
| 4-(3-(4-Me-PhO)PhO)-2-pyridyl |
| 5-(3-(3-MeO-PhO)PhO)-2-pyridyl |
| 6-PhCH=CH-3-pyridyl |
| 6-(3-Br-Ph)CH=CH-3-pyridyl |
| 5-(2-F-Ph)CH=CH-2-pyridyl |
| 2-(3-Cl-Ph)CH=CH-4-pyridyl |
| 5-(2,4-diF-Ph)CH=CH-3-pyridyl |
| 6-(4-$NO_2$-Ph)CH=CH-3-pyridyl |
| 4-(3-Me-Ph)CH=CH-2-pyridyl |
| 5-(3-Bu-Ph)CH=CH-2-pyridyl |
| 6-(4-$CF_3$-Ph)CH=CH-3-pyridyl |
| 6-(2-MeS-Ph)CH=CH-3-pyridyl |
| 5-(4-MeO-Ph)CH=CH-2-pyridyl |
| 6-(4-BuO-Ph)CH=CH-3-pyridyl |
| 6-$PhCH_2CH_2$-3-pyridyl |
| 6-(3-Br-Ph)$CH_2CH_2$-3-pyridyl |
| 5-(2-F-Ph)$CH_2CH_2$-2-pyridyl |
| 2-(3-Cl-Ph)$CH_2CH_2$-4-pyridyl |
| 5-(2,4-diF-Ph)$CH_2CH_2$-3-pyridyl |
| 6-(4-$NO_2$-Ph)$CH_2CH_2$-3-pyridyl |
| 4-(3-Me-Ph)$CH_2CH_2$-2-pyridyl |
| 5-(3-Bu-Ph)$CH_2CH_2$-2-pyridyl |
| 6-(4-$CF_3$-Ph)$CH_2CH_2$-3-pyridyl |
| 6-(2-MeS-Ph)$CH_2CH_2$-3-pyridyl |
| 5-(4-MeO-Ph)$CH_2CH_2$-2-pyridyl |
| 6-(4-BuO-Ph)$CH_2CH_2$-3-pyridyl |

| $R^2$ |
|---|
| 6-$PhOCH_2$-3-pyridyl |
| 6-(3-Br-Ph)$OCH_2$-3-pyridyl |
| 5-(2-F-Ph)$OCH_2$-2-pyridyl |
| 2-(3-Cl-Ph)$OCH_2$-4-pyridyl |
| 5-(2,4-diF-Ph)$OCH_2$-3-pyridyl |
| 6-(4-$NO_2$-Ph)$OCH_2$-3-pyridyl |
| 4-(3-Me-Ph)$OCH_2$-2-pyridyl |
| 5-(3-Bu-Ph)$OCH_2$-2-pyridyl |
| 6-(4-$CF_3$-Ph)$OCH_2$-3-pyridyl |
| 6-(2-MeS-Ph)$OCH_2$-3-pyridyl |
| 5-(4-MeO-Ph)$OCH_2$-2-pyridyl |
| 6-(4-BuO-Ph)$OCH_2$-3-pyridyl |
| 6-$PhCH_2$-3-pyridyl |
| 6-(3-Br-Ph)$CH_2$-3-pyridyl |
| 5-(2-F-Ph)$CH_2$-2-pyridyl |
| 2-(3-Cl-Ph)$CH_2$-4-pyridyl |
| 5-(2,4-diF-Ph)$CH_2$-3-pyridyl |
| 6-(4-$NO_2$-Ph)$CH_2$-3-pyridyl |
| 4-(3-Me-Ph)$CH_2$-2-pyridyl |
| 5-(3-Bu-Ph)$CH_2$-2-pyridyl |
| 6-(4-$CF_3$-Ph)$CH_2$-3-pyridyl |
| 6-(2-MeS-Ph)$CH_2$-3-pyridyl |
| 5-(4-MeO-Ph)$CH_2$-2-pyridyl |
| 6-(4-BuO-Ph)$CH_2$-3-pyridyl |
| 6-PhS-3-pyridyl |
| 6-(3-Br-Ph)S-3-pyridyl |
| 5-(2-F-Ph)S-2-pyridyl |
| 2-(3-Cl-Ph)S-4-pyridyl |

$B^2$

5-(2,4-diF-Ph)S-3-pyridyl
6-(4-$NO_2$-Ph)S-3-pyridyl
4-(3-Me-Ph)S-2-pyridyl
5-(3-Bu-Ph)S-2-pyridyl
6-(4-$CF_3$-Ph)S-3-pyridyl
6-(2-MeS-Ph)S-3-pyridyl
5-(4-MeO-Ph)S-2-pyridyl
6-(4-BuO-Ph)S-3-pyridyl
6-Ph-3-pyridyl
6-(3-Br-Ph)-3-pyridyl
5-(2-F-Ph)-2-pyridyl
2-(3-Cl-Ph)-4-pyridyl
5-(2,4-diF-Ph)-3-pyridyl
6-(4-$NO_2$-Ph)-3-pyridyl
4-(3-Me-Ph)-2-pyridyl
5-(3-Bu-Ph)-2-pyridyl
6-(4-$CF_3$-Ph)-3-pyridyl
6-(2-MeS-Ph)-3-pyridyl
5-(4-MeO-Ph)-2-pyridyl
6-(4-BuO-Ph)-3-pyridyl
6-Ph$CH_2$O-3-pyridyl
5-(3-Br-Ph)$CH_2$O-2-pyridyl
6-(2-F-Ph)$CH_2$O-3-pyridyl
4-(3-Cl-Ph)$CH_2$O-2-pyridyl
2-(2,4-diF-Ph)$CH_2$O-4-pyridyl
6-(4-$NO_2$-Ph)$CH_2$O-3-pyridyl
6-(3-Me-Ph)$CH_2$O-3-pyridyl
5-(3-Bu-Ph)$CH_2$O-2-pyridyl

$B^2$

2-(4-$CF_3$-Ph)$CH_2$O-4-pyridyl
5-(2-MeS-Ph)$CH_2$O-3-pyridyl
5-(4-MeO-Ph)$CH_2$O-2-pyridyl
6-(4-BuO-Ph)$CH_2$O-3-pyridyl
5-(4-CN-Ph)$CH_2$O-2-pyridyl
6-(3-PhO-Ph)$CH_2$O-3-pyridyl
6-[3-(3-F-PhO)Ph]$CH_2$O-3-pyridyl
6-[4-(2-$NO_2$-PhO)Ph]$CH_2$O-3-pyridyl
5-[3-(4-Me-PhO)Ph]$CH_2$O-2-pyridyl
5-[4-(4-Bu-PhO)Ph]$CH_2$O-2-pyridyl
6-[3-(3-$CF_3$-PhO)Ph]$CH_2$O-3-pyridyl
6-[2-(3-MeS-PhO)Ph]$CH_2$O-3-pyridyl
6-[4-(4-MeO-PhO)Ph]$CH_2$O-3-pyridyl
5-[3-(3-BuO-PhO)Ph]$CH_2$O-2-pyridyl
6-(2-F-4-MeO-Ph)$CH_2$O-3-pyridyl
6-(2-$CF_3$-4-Et-Ph)$CH_2$O-3-pyridyl
6-[(3-pyridyl)NHC(=O)O]-3-pyridyl
5-[(3-Br-2-pyridyl)NHC(=O)O]-2-pyridyl
6-[(2-F-3-pyridyl)NHC(=O)O]-3-pyridyl
4-[(3-Cl-4-pyridyl)NHC(=O)O]-2-pyridyl
2-[(2,4-diF-3-pyridyl)NHC(=O)O]-4-pyridyl
6-[(4-$NO_2$-2-pyridyl)NHC(=O)O]-3-pyridyl
6-[(3-Me-2-pyridyl)NHC(=O)O]-3-pyridyl
5-[(3-Bu-2-pyridyl)NHC(=O)O]-2-pyridyl
2-[(4-$CF_3$-3-pyridyl)NHC(=O)O]-4-pyridyl
5-[(2-MeS-4-pyridyl)NHC(=O)O]-3-pyridyl
5-[(4-MeO-3-pyridyl)NHC(=O)O]-2-pyridyl
2-[(4-BuO-3-pyridyl)NHC(=O)O]-4-pyridyl

$R^2$

5-[(4-CN-2-pyridyl)NHC(=O)O]-3-pyridyl
5-[(3-PhO-2-pyridyl)NHC(=O)O]-2-pyridyl
6-[3-(3-F-PhO)-2-pyridyl-NHC(=O)O]-3-pyridyl
5-[4-(2-$NO_2$-PhO)-3-pyridyl-NHC(=O)O]-2-pyridyl
6-[2-(4-Me-PhO)-4-pyridyl-NHC(=O)O]-3-pyridyl
6-[4-(4-Bu-PhO)-3-pyridyl-NHC(=O)O]-3-pyridyl
6-[4-(3-$CF_3$-PhO)-2-pyridyl-NHC(=O)O]-3-pyridyl
5-[4-(3-MeS-PhO)-3-pyridyl-NHC(=O)O]-2-pyridyl
5-[3-(4-MeO-PhO)-2-pyridyl-NHC(=O)O]-2-pyridyl
6-[3-(3-BuO-PhO)-2-pyridyl-NHC(=O)O]-3-pyridyl
6-[(2-F-4-MeO-3-pyridyl)NHC(=O)O]-3-pyridyl
6-[(2-$CF_3$-4-Et-3-pyridyl)NHC(=O)O]-3-pyridyl
6-[(3-pyridyloxy)C(=O)]-3-pyridyl
5-[(3-Br-2-pyridyloxy)C(=O)]-2-pyridyl
6-[(2-F-3-pyridyloxy)C(=O)]-3-pyridyl
4-[(3-Cl-4-pyridyloxy)C(=O)]-2-pyridyl
2-[(2,4-diF-3-pyridyloxy)C(=O)]-4-pyridyl
6-[(4-$NO_2$-2-pyridyloxy)C(=O)]-3-pyridyl
6-[(3-Me-2-pyridyloxy)C(=O)]-3-pyridyl
5-[(3-Bu-2-pyridyloxy)C(=O)]-2-pyridyl
2-[(4-$CF_3$-3-pyridyloxy)C(=O)]-4-pyridyl
5-[(2-MeS-4-pyridyloxy)C(=O)]-3-pyridyl
5-[(4-MeO-3-pyridyloxy)C(=O)]-2-pyridyl
4-[(4-BuO-3-pyridyloxy)C(=O)]-3-pyridyl
2-[(4-CN-2-pyridyloxy)C(=O)]-4-pyridyl
5-[(3-PhO-2-pyridyloxy)C(=O)]-2-pyridyl
5-[3-(3-F-PhO)-2-pyridyloxy-C(=O)]-3-pyridyl
6-[4-(2-$NO_2$-PhO)-3-pyridyloxy-C(=O)]-3-pyridyl

$R^2$

5-[2-(4-Me-PhO)-4-pyridyloxy-C(=O)]-2-pyridyl

6-[4-(4-Bu-PhO)-3-pyridyloxy-C(=O)]-3-pyridyl

6-[4-(3-$CF_3$-PhO)-2-pyridyloxy-C(=O)]-3-pyridyl

6-[4-(3-MeS-PhO)-3-pyridyloxy-C(=O)]-3-pyridyl

5-[3-(4-MeO-PhO)-2-pyridyloxy-C(=O)]-2-pyridyl

5-[3-(3-BuO-PhO)-2-pyridyloxy-C(=O)]-2-pyridyl

6-[(2-F-4-MeO-3-pyridyloxy)C(=O)]-3-pyridyl

6-[(2-$CF_3$-4-Et-3-pyridyloxy)C(=O)]-3-pyridyl

6-[(3-pyridyl)C(=O)O]-3-pyridyl

5-[(3-Br-2-pyridyl)C(=O)O]-2-pyridyl

6-[(2-F-3-pyridyl)C(=O)O]-3-pyridyl

4-[(3-Cl-4-pyridyl)C(=O)O]-2-pyridyl

2-[(2,4-diF-3-pyridyl)C(=O)O]-4-pyridyl

6-[(4-$NO_2$-2-pyridyl)C(=O)O]-3-pyridyl

6-[(3-Me-2-pyridyl)C(=O)O]-3-pyridyl

5-[(3-Bu-2-pyridyl)C(=O)O]-2-pyridyl

2-[(4-$CF_3$-3-pyridyl)C(=O)O]-4-pyridyl

5-[(2-MeS-4-pyridyl)C(=O)O]-3-pyridyl

5-[(4-MeO-3-pyridyl)C(=O)O]-2-pyridyl

2-[(4-BuO-3-pyridyl)C(=O)O]-4-pyridyl

5-[(4-CN-2-pyridyl)C(=O)O]-3-pyridyl

5-[(3-PhO-2-pyridyl)C(=O)O]-2-pyridyl

6-[3-(3-F-PhO)-2-pyridyl-C(=O)O]-3-pyridyl

5-[4-(2-$NO_2$-PhO)-3-pyridyl-C(=O)O]-2-pyridyl

6-[2-(4-Me-PhO)-4-pyridyl-C(=O)O]-3-pyridyl

6-[4-(4-Bu-PhO)-3-pyridyl-C(=O)O]-3-pyridyl

6-[4-(3-$CF_3$-PhO)-2-pyridyl-C(=O)O]-3-pyridyl

5-[4-(3-MeS-PhO)-3-pyridyl-C(=O)O]-2-pyridyl

$R^2$

5-[3-(4-MeO-PhO)-2-pyridyl-C(=O)O]-2-pyridyl

6-[3-(3-BuO-PhO)-2-pyridyl-C(=O)O]-3-pyridyl

6-[(2-F-4-MeO-3-pyridyl)C(=O)O]-3-pyridyl

6-[(2-$CF_3$-4-Et-3-pyridyl)C(=O)O]-3-pyridyl

5-[MeNHC(=O)O]-2-pyridyl

2-F-6-[octyl-NHC(=O)O]-3-pyridyl

5-[PhNHC(=O)O]-3-pyridyl

2-[(3-Br-Ph)NHC(=O)O]-4-pyridyl

5-[(2-F-Ph)NHC(=O)O]-3-pyridyl

2-F-6-[(3-Cl-Ph)NHC(=O)O]-3-pyridyl

6-[(2,4-diF-Ph)NHC(=O)O]-3-pyridyl

3-F-2-[(4-$NO_2$-Ph)NHC(=O)O]-4-pyridyl

6-[(3-Me-Ph)NHC(=O)O]-3-pyridyl

5-[(3-Bu-Ph)NHC(=O)O]-2-pyridyl

6-[(4-$CF_3$-Ph)NHC(=O)O]-3-pyridyl

2-F-6-[(2-MeS-Ph)NHC(=O)O]-3-pyridyl

5-[(4-MeO-Ph)NHC(=O)O]-2-pyridyl

4-[(4-BuO-Ph)NHC(=O)O]-2-pyridyl

6-[(4-CN-Ph)NHC(=O)O]-3-pyridyl

2-F-6-[(3-PhO-Ph)NHC(=O)O]-3-pyridyl

2-[3-(3-F-PhO)Ph-NHC(=O)O]-4-pyridyl

5-[4-(2-$NO_2$-PhO)Ph-NHC(=O)O]-2-pyridyl

5-[2-(4-Me-PhO)Ph-NHC(=O)O]-3-pyridyl

5-[4-(4-Bu-PhO)Ph-NHC(=O)O]-2-pyridyl

6-[4-(3-$CF_3$-PhO)Ph-NHC(=O)O]-3-pyridyl

6-[4-(3-MeS-PhO)Ph-NHC(=O)O]-3-pyridyl

6-[3-(4-MeO-PhO)Ph-NHC(=O)O]-3-pyridyl

2-F-6-[3-(3-BuO-PhO)Ph-NHC(=O)O]-3-pyridyl

$R^2$

5-[(2-F-4-MeO-Ph)NHC(=O)O]-2-pyridyl

6-[(2-$CF_3$-4-Et-Ph)NHC(=O)O]-2-pyridyl

5-[MeOC(=O)]-2-pyridyl

2-F-6-[octyloxy-C(=O)]-3-pyridyl

5-[PhOC(=O)]-3-pyridyl

2-[(3-Br-PhO)C(=O)]-4-pyridyl

5-[(2-F-PhO)C(=O)]-3-pyridyl

2-F-6-[(3-Cl-PhO)C(=O)]-3-pyridyl

6-[(2,4-diF-PhO)C(=O)]-3-pyridyl

3-F-2-[(4-$NO_2$-PhO)C(=O)]-4-pyridyl

6-[(3-Me-PhO)C(=O)]-3-pyridyl

5-[(3-Bu-PhO)C(=O)]-2-pyridyl

6-[(4-$CF_3$-PhO)C(=O)]-3-pyridyl

2-F-6-[(2-MeS-PhO)C(=O)]-3-pyridyl

5-[(4-MeO-PhO)C(=O)]-2-pyridyl

4-[(4-BuO-PhO)C(=O)]-2-pyridyl

6-[(4-CN-PhO)C(=O)]-3-pyridyl

2-F-6-[(3-PhO-PhO)C(=O)]-3-pyridyl

2-[3-(3-F-PhO)PhO-C(=O)]-4-pyridyl

5-[4-(2-$NO_2$-PhO)PhO-C(=O)]-2-pyridyl

5-[2-(4-Me-PhO)PhO-C(=O)]-3-pyridyl

5-[4-(4-Bu-PhO)PhO-C(=O)]-2-pyridyl

6-[4-(3-$CF_3$-PhO)PhO-C(=O)]-3-pyridyl

6-[4-(3-MeS-PhO)PhO-C(=O)]-3-pyridyl

6-[3-(4-MeO-PhO)PhO-C(=O)]-3-pyridyl

2-F-6-[3-(3-BuO-PhO)PhO-C(=O)]-3-pyridyl

5-[(2-F-4-MeO-PhO)C(=O)]-2-pyridyl

6-[(2-$CF_3$-4-Et-PhO)C(=O)]-2-pyridyl

| $R^2$ | $R^2$ |
|---|---|
| 5-[MeC(=O)O]-2-pyridyl | 2-furanyl |
| 2-F-6-[octyl-C(=O)O]-3-pyridyl | 3-furanyl |
| 5-[PhC(=O)O]-3-pyridyl | 2-F-3-furanyl |
| 2-[(3-Br-Ph)C(=O)O]-4-pyridyl | 5-Cl-3-furanyl |
| 5-[(2-F-Ph)C(=O)O]-3-pyridyl | 5-$NO_2$-3-furanyl |
| 2-F-6-[(3-Cl-Ph)C(=O)O]-3-pyridyl | 3,4-diF-2-furanyl |
| 6-[(2,4-diF-Ph)C(=O)O]-3-pyridyl | 4-Me-2-furanyl |
| 3-F-2-[(4-$NO_2$-Ph)C(=O)O]-4-pyridyl | 5-Bu-3-furanyl |
| 6-[(3-Me-Ph)C(=O)O]-3-pyridyl | 4-$CF_3$-2-furanyl |
| 5-[(3-Bu-Ph)C(=O)O]-2-pyridyl | 5-MeS-2-furanyl |
| 6-[(4-$CF_3$-Ph)C(=O)O]-3-pyridyl | 5-MeO-3-furanyl |
| 2-F-6-[(2-MeS-Ph)C(=O)O]-3-pyridyl | 4-BuO-2-furanyl |
| 5-[(4-MeO-Ph)C(=O)O]-2-pyridyl | |
| 4-[(4-BuO-Ph)C(=O)O]-2-pyridyl | |
| 6-[(4-CN-Ph)C(=O)O]-3-pyridyl | |
| 2-F-6-[(3-PhO-Ph)C(=O)O]-3-pyridyl | |
| 2-[3-(3-F-PhO)Ph-C(=O)O]-4-pyridyl | |
| 5-[4-(2-$NO_2$-PhO)Ph-C(=O)O]-2-pyridyl | |
| 5-[2-(4-Me-PhO)Ph-C(=O)O]-3-pyridyl | |
| 5-[4-(4-Bu-PhO)Ph-C(=O)O]-2-pyridyl | |
| 6-[4-(3-$CF_3$-PhO)Ph-C(=O)O]-3-pyridyl | |
| 6-[4-(3-MeS-PhO)Ph-C(=O)O]-3-pyridyl | |
| 6-[3-(4-MeO-PhO)Ph-C(=O)O]-3-pyridyl | |
| 2-F-6-[3-(3-BuO-Ph)PhO-C(=O)O]-3-pyridyl | |
| 5-[(2-F-4-MeO-Ph)C(=O)O]-2-pyridyl | |
| 6-[(2-$CF_3$-4-Et-Ph)C(=O)O]-2-pyridyl | |

TABLE 3

Compounds of Formula If wherein A=S and:

| n | $R^{43}$ | n | $R^{43}$ |
|---|---|---|---|
| 5 | Me | 6 | 4-F-PhO |
| 6 | Ph | 8 | 4-Et-PhO |
| 8 | 4-$CF_3$-Ph | 2 | 4-EtO-PhO |
| 9 | 3-$F_3$CO-Ph | 1 | 4-(4-F-PhO)PhO |
| 5 | 2,4-diCl-Ph | 2 | 4-(3-Et-PhO)PhO |
| 1 | 4-F-Ph | 3 | 4-(3-Bu-PhO)PhO |
| 2 | 4-Et-Ph | 3 | MeNH |
| 3 | 3-EtO-Ph | 4 | $Me_2N$ |
| 4 | 4-(4-F-PhO)Ph | 1 | PhNH |
| 3 | 4-(3-Et-PhO)Ph | 5 | PhN(Me) |
| 1 | 4-(3-BuO-PhO)Ph | 6 | 4-$CF_3$-PhNH |
| 5 | 3-(4-$CF_3$-PhO)Ph | 2 | 3-$F_3$CO-PhNH |
| 8 | MeO | 3 | 2,4-diCl-PhNH |
| 6 | PhO | 8 | 4-Et-PhNH |
| 2 | 4-$F_3$CO-PhO | 4 | 4-(4-F-PhO)PhNH |
| 4 | 3,5-diCl-PhO | 3 | 4-(4-$CF_3$-PhO)PhNH |

TABLE 4

Compounds of Formula Ig wherein A=S and:

| n | T | W | n | T | W |
|---|---|---|---|---|---|
| 1 | O | H | 1 | O | 2,6-diEt-4-pyridyl |
| 4 | O | Et | 4 | O | MeC(=O) |
| 2 | O | Bzl | 2 | O | PhC(=O) |
| 3 | O | 4-Cl-Bzl | 1 | O | 3,5-diF-PhC(=O) |
| 5 | O | Ph | 3 | O | 3,5-MeO-PhC(=O) |
| 1 | O | 2,6-diF-Ph | 6 | O | 4-pyridyl-C(=O) |
| 6 | O | 4-F-Ph | 7 | O | 3,5-diCl-4-pyridyl-C(=O) |
| 7 | O | 3,5-diMe-Ph | 4 | O | BuOC(=O) |
| 8 | O | 4-pyridyl | 2 | O | PhOC(=O) |

| n | T | W | n | T | W |
|---|---|---|---|---|---|
| 5 | O | (3,5-diF-Ph)OC(=O) | 4 | NH | PhC(=O) |
| 3 | O | (4-EtO-Ph)OC(=O) | 5 | NH | (4-pyridyl)C(=O) |
| 6 | O | (4-pyridyl)OC(=O) | 6 | NH | MeOC(=O) |
| 2 | O | NHMeC(=O) | 3 | NH | PhOC(=O) |
| 5 | O | NHPhC(=O) | 1 | NH | (3-pyridyl)OC(=O) |
| 1 | O | NHpyridylC(=O) | 1 | NH | MeNHC(=O) |
| 4 | NH | Me | 2 | N-Me | Me |
| 1 | NH | Bzl | 3 | N-Me | Ph |
| 8 | NH | Ph | 4 | N-Ph | Ph |
| 3 | NH | 3,5-diF-Ph | 5 | --- | H |
| 2 | NH | 4-pyridyl | 1 | --- | Me |
| 1 | NH | EtC(=O) | 2 | --- | Ph |

**TABLE 5**

Compounds of Formula Ih wherein A=S and:

| n | m | $R^{35}$ | n | m | $R^{35}$ |
|---|---|---|---|---|---|
| 2 | 1 | MeS | 2 | 2 | 2-pyrimidyl |
| 3 | 1 | $FCH_2CH_2O$ | 2 | 1 | *c*-hexyl |
| 1 | 1 | *c*-hexyloxy | 1 | 2 | *c*-pentyl |
| 1 | 1 | EtOC(=O) | 1 | 2 | EtO |
| 1 | 2 | OH | 1 | 1 | Ph |
| 3 | 1 | MeC(=O)O | 2 | 2 | PhO |
| 2 | 2 | PhOC(=O)O | 2 | 6 | H |
| 1 | 3 | MeNHC(=O)O | 2 | 8 | H |
| 2 | 1 | PhNH | 1 | 7 | EtS |
| 2 | 3 | $(MeO)_2P(=O)O$ | 3 | 5 | $CF_3O$ |
| 1 | 2 | $MeSO_3$ | 3 | 5 | *c*-hexyl |
| 3 | 2 | 4-pyridyl | 1 | 8 | Ph |
| 1 | 3 | 4-pyridyloxy | | | |

TABLE 6

Compounds of Formula I wherein

$R^1$ = Me, $R^2$ = 4-PhO-Ph, $R^3$ = Ph, $R^4$ = H, A = N-$R^{15}$ and:

| $R^{15}$ | $R^{15}$ |
|---|---|
| H | Ph |
| H (HBr salt) | 4-Cl-Ph |
| H (HCl salt) | 3,5-diCl-Ph |
| H (HI salt) | 3-Br-Ph |
| H ($H_2SO_4$ salt) | 2-F-Ph |
| H ($HNO_3$ salt) | 4-I-Ph |
| H ($CF_3CO_2H$ salt) | 3-$NO_2$-Ph |
| H ($MeSO_3H$ salt) | 4-Me-Ph |
| H ($PhSO_3H$ salt) | 4-Bu-Ph |
| H (4-TsOH salt) | 3-$CF_3$-Ph |
| H [(4-dodecyl-Ph)$SO_3H$ salt] | 4-MeS-Ph |
| H ($H_3PO_4$ salt) | 4-Me-O-Ph |
| H (camphor sulfonic acid salt) | 4-BuO-Ph |
| Me | Bzl |
| Me (HBr salt) | Bzl (HBr salt) |
| Et | 4-Cl-Bzl |
| Pr | 2,4-diCl-Bzl |
| Bu | 4-Br-Bzl |
| octyl | 4-F-Bzl |
| $MeOCH_2CH_2$ | 4-I-Bzl |
| $EtOCH_2CH_2$ | 4-$NO_2$-Bzl |
| cyclopropyl | 4-Me-Bzl |
| cyclohexyl | 4-Bu-Bzl |
| allyl | 4-$CF_3$-Bzl |
| 2-octenyl | 4-MeS-Bzl |
| propargyl | 4-MeO-Bzl |
| 2-octynyl | |

| $R^{15}$ | $R^{15}$ |
|---|---|
| 4-BuO-Bzl | 6-F-2-pyridyl |
| Ph-(CH)Me | 4-I-2-pyridyl |
| Ph-(CH)Bu | 5-$NO_2$-2-pyridyl |
| 2-pyridyl | 6-Me-2-pyridyl |
| 3-pyridyl | 6-Bu-2-pyridyl |
| 4-pyridyl | 5-$CF_3$-3-pyridyl |
| 5-Cl-2-pyridyl | 2-MeS-3-pyridyl |
| 3,5-diCl-2-pyridyl | 2-MeO-3-pyridyl |
| 5-Br-2-pyridyl | 2-BuO-3-pyridyl |

TABLE 7

Compounds of Formula I wherein

$R^1$ = Me, $R^2$ = 4-PhO-Ph, $R^3$ = Ph, $R^4$ = H, A = N-C(=O)$R^{16}$ and:

| $R^{16}$ | $R^{16}$ |
|---|---|
| H | $Cl_2CH$ |
| Me | $BrCH_2$ |
| heptadecyl | $F(CH_2)_2$ |
| Et | $ICH_2$ |
| Pr | $CF_3$ |
| i-Pr | $MeOCH_2$ |
| Bu | $CH_3(CH_2)_7OCH_2$ |
| pentyl | $CH_2{=}CHCH_2OCH_2$ |
| $Me_3C$ | $MeSCH_2$ |
| $ClCH_2$ | $CH_3(CH_2)_7SCH_2$ |
| $ClCH_2C(Me)_2$ | Bzl |

| $B^{16}$ | $B^{16}$ |
|---|---|
| Ph-(CH)Me | (4-BuO-Ph)$OCH_2$ |
| $PhOCH_2$ | 4-Cl-BzlO |
| PhO(CH)Me | 2,4-diCl-BzlO |
| $BzlOCH_2$ | 4-Br-BzlO |
| $PhSCH_2$ | 4-F-BzlO |
| 4-Cl-Bzl | 4-I-BzlO |
| 2,4-diCl-Bzl | 4-$NO_2$-BzlO |
| 4-Br-Bzl | 4-Me-BzlO |
| 4-F-Bzl | 4-Bu-BzlO |
| 4-I-Bzl | 4-$CF_3$-BzlO |
| 4-$NO_2$-Bzl | 4-MeS-BzlO |
| 4-Me-Bzl | 4-MeO-BzlO |
| 4-Bu-Bzl | 4-Bu-BzlO |
| 4-$CF_3$-Bzl | (4-Cl-Ph)$SCH_2$ |
| 4-MeS-Bzl | (2,4-diCl-Ph)$SCH_2$ |
| 4-MeO-Bzl | (4-Br-Ph)$SCH_2$ |
| 4-BuO-Bzl | (4-F-Ph)$SCH_2$ |
| (4-Cl-Ph)$OCH_2$ | (4-I-Ph)$SCH_2$ |
| (2,4-diCl-Ph)$OCH_2$ | (4-NO2-Ph)$SCH_2$ |
| (4-Br-Ph)$OCH_2$ | (4-Me-Ph)$SCH_2$ |
| (4-F-Ph)$OCH_2$ | (4-Bu-Ph)$SCH_2$ |
| (4-I-Ph)$OCH_2$ | (4-$CF_3$-Ph)$SCH_2$ |
| (4-$NO_2$-Ph)$OCH_2$ | (4-MeS-Ph)$SCH_2$ |
| (4-Me-Ph)$OCH_2$ | (4-MeO-Ph)$SCH_2$ |
| (4-Bu-Ph)$OCH_2$ | (4-BuO-Ph)$SCH_2$ |
| (4-$CF_3$-Ph)$OCH_2$ | $CH_3C(=O)CH_2$ |
| (4-MeS-Ph)$OCH_2$ | MeOC(=O)$CH_2$ |
| (4-MeO-Ph)$OCH_2$ | EtOC(=O)$CH_2$ |

| $R^{16}$ | $R^{16}$ |
|---|---|
| $BuOC(=O)CH_2$ | 4-F-Ph |
| vinyl | 4-I-Ph |
| 1-propen-2-yl | 4-$NO_2$-Ph |
| 8-heptadecenyl | 4-CN-Ph |
| $ClCH_2CH=CH$ | 4-Me-Ph |
| $Cl_3CCH-CH$ | 4-Bu-Ph |
| $BrCH_2CH=CH$ | 4-MeO-Ph |
| $FCH_2CH=CH$ | 4-BuO-Ph |
| $ICH_2CH=CH$ | 4-MeS-Ph |
| $MeOCH_2CH=CH$ | 4-BuS-Ph |
| $BuOCH_2CH=CH$ | 4-$CF_3$-Ph |
| 1-heptynyl | 1-naphthalenyl |
| cyclopropyl | 2-naphthalenyl |
| cyclopentyl | 2-furanyl |
| cyclohexyl | 3-furanyl |
| cycloheptyl | 2-thienyl |
| cyclooctyl | 3-thienyl |
| 1-cyclopentenyl | benzoyl |
| 1-cyclohexenyl | 2-pyridinyl |
| 2-Me-cyclopentyl | 3-pyridinyl |
| 2-Me-cyclohexyl | 4-pyridinyl |
| cyclopropylmethyl | 1-Cl-2-naphthalenyl |
| cyclopentylmethyl | 1,6-di-Cl-2-naphthalenyl |
| cyclohexylmethyl | 1-Br-2-naphthalenyl |
| Ph | 1-F-2-naphthalenyl |
| 4-Cl-Ph | 1-I-2-naphthalenyl |
| 3-Cl-Ph | 4-$NO_2$-2-naphthalenyl |
| 2-Cl-Ph | 1-Me-2-naphthalenyl |
| 2,4-diCl-Ph | 3-Bu-2-naphthalenyl |
| 4-Br-Ph | 3-$CF_3$-2-naphthalenyl |

$R^{16}$

1-MeS-2-naphthalenyl
1-MeO-2-naphthalenyl
1-BuO-2-naphthalenyl
5-Cl-2-furanyl
4,5-diCl-2-furanyl
5-Br-2-furanyl
5-F-2-furanyl
5-I-2-furanyl
5-$NO_2$-2-furanyl
5-Me-2-furanyl
5-Br-2-furanyl
5-$CF_3$-3-furanyl
5-MeS-2-furanyl
5-MeO-2-furanyl
5-BuO-2-furanyl
5-Cl-2-thienyl
4,5-diCl-2-thienyl
5-Br-2-thienyl
5-F-2-thienyl
5-I-2-thienyl
5-$NO_2$-2-thienyl
5-Me-2-thienyl
5-Bu-2-thienyl
5-$CF_3$-2-thienyl
5-MeS-2-thienyl
5-MeO-2-thienyl
5-BuO-2-thienyl

$R^{16}$

4-Cl-PhC(=O)
2,4-diCl-PhC(=O)
4-Br-PhC(=O)
4-F-PhC(=O)
4-I-PhC(=O)
3-$NO_2$-PhC(=O)
4-Me-PhC(=O)
4-Bu-PhC(=O)
4-$CF_3$-PhC(=O)
4-MeS-PhC(=O)
4-BuO-PhC(=O)
2-Cl-3-pyridyl
6-Cl-3-pyridyl
5-Br-3-pyridyl
2-F-3-pyridyl
6-I-3-pyridyl
5-$NO_2$-3-pyridyl
6-Me-3-pyridyl
6-Bu-3-pyridyl
5-$CF_3$-2-pyridyl
2-MeS-3-pyridyl
2-MeO-3-pyridyl
2-BuO-3-pyridyl
MeOC(=O)
EtOC(=O)
BuOC(=O)

**TABLE 8**

Compounds of Formula I wherein

$R^1$ = Me, $R^2$ = 4-PhO-Ph, $R^3$ = Ph, $R^4$ = H, A = N-C(=O)$OR^{17}$ and:

| $R^{17}$ | $R^{17}$ |
|---|---|
| Me | 4-Br-Bzl |
| octadecyl | 4-F-Bzl |
| Et | 4-I-Bzl |
| Pr | 4-$NO_2$-Bzl |
| *i*-Pr | 4-Me-Bzl |
| Bu | 4-Bu-Bzl |
| *i*-Bu | 4-$CF_3$-Bzl |
| *sec*-Bu | 4-MeS-Bzl |
| $ClCH_2CH_2$ | 4-MeO-Bzl |
| $Cl_2CHCH_2$ | 4-BuO-Bzl |
| $Cl_3CCH_2$ | 1-Cl-2-naphthalenylmethyl |
| $BrCH_2CH_2$ | 1,4-diCl-naphthalenylmethyl |
| $F(CH_2)_3$ | 1-Br-2-naphthalenylmethyl |
| $CF_3CH_2$ | 1-F-2-naphthalenylmethyl |
| $ICH_2CH_2$ | 1-I-2-naphthalenylmethyl |
| $MeOCH_2CH_2$ | 1-$NO_2$-2-naphthalenylmethyl |
| $CH_2(CH_2)_{11}OCH_2CH_2$ | 1-Me-2-naphthalenylmethyl |
| $EtOCH_2CH_2$ | 1-Bu-2-naphthalenylmethyl |
| $MeSCH_2CH_2$ | 1-$CF_3$-2-naphthalenylmethyl |
| $CH_3(CH_2)_{11}SCH_2CH_2$ | 1-MeS-2-naphthalenylmethyl |
| Bzyl | 1-MeO-2-naphthalenylmethyl |
| Ph-(CH)Me | 1-BuO-2-naphthalenylmethyl |
| 1-naphthalenylmethyl | (4-Cl-Ph)$OCH_2CH_2$ |
| 2-naphthalenylmethyl | (2,4-diCl-Ph)$OCH_2CH_2$ |
| 2-(2-naphthalenyl)ethyl | (4-F-Ph)$OCH_2CH_2$ |
| $PhOCH_2CH_2$ | (4-I-Ph)$OCH_2CH_2$ |
| 4-Cl-Bzl | (4-$NO_2$-Ph)$OCH_2CH_2$ |
| 2,4-diCl-Bzl | (4-Me-Ph)$OCH_2CH_2$ |

| $R^{17}$ |
|---|
| (4-Bu-Ph)$OCH_2CH_2$ |
| (4-$CF_3$-Ph)$OCH_2CH_2$ |
| (4-MeS-Ph)$OCH_2CH_2$ |
| (4-MeO-Ph)$OCH_2CH_2$ |
| (4-BuO-Ph)$OCH_2CH_2$ |
| $CH_2$=CH |
| $CH_3(CH_2)_6CH$=$CHCH_2$ |
| $CH_2$=$CHCH_2$ |
| $ClCH_2CH$=$CHCH_2$ |
| $Cl_3CCH$=$CHCH_2$ |
| $BrCH_2CH$=$CHCH_2$ |
| $FCH_2CH$=$CHCH_2$ |
| $ICH_2CH$=$CHCH_2$ |
| $MeOCH_2CH$=$CHCH_2$ |
| $BuOCH_2CH$=$CHCH_2$ |
| $HC\equiv CCH_2$ |
| $CH_3(CH_2)_4C\equiv CCH_2$ |
| cyclopropyl |
| cyclopentyl |
| cyclohexyl |
| cycloheptyl |
| cyclododecyl |
| 2-cyclopentenyl |
| 2-cyclohexenyl |
| 2-Me-cyclopentyl |
| 2-Me-cyclohexyl |
| cyclopentylmethyl |
| cyclohexylmethyl |
| 1-naphthalenyl |
| 2-naphthalenyl |
| 2-thienyl |
| 3-thienyl |
| 4-Cl-Ph |
| 2,4-diCl-Ph |
| 4-Br-Ph |
| 4-F-Ph |
| 4-I-Ph |
| 4-$NO_2$-Ph |
| 4-Me-Ph |
| 4-Bu-Ph |
| 4-$CF_3$-Ph |
| 4-MeS-Ph |
| 4-MeO-Ph |
| 4-BuO-Ph |
| 1-Cl-2-naphthalenyl |
| 1,3-diCl-2-naphthalenyl |
| 1-Br-2-naphthalenyl |
| 1-F-2-naphthalenyl |
| 1-I-2-naphthalenyl |
| 1-$NO_2$-2-naphthalenyl |
| 1-Me-2-naphthalenyl |
| 1-Bu-2-naphthalenyl |
| 1-$CF_3$-2-naphthalenyl |
| 1-MeS-2-naphthalenyl |
| 1-MeO-2-naphthalenyl |
| 1-BuO-2-naphthalenyl |
| 5-Cl-2-thienyl |
| 4,5-diCl-thienyl |

| $R^{17}$ | $R^{17}$ |
|---|---|
| 5-Br-2-thienyl | 5-Bu-2-thienyl |
| 5-F-2-thienyl | 5-$CF_3$-2-thienyl |
| 5-I-2-thienyl | 5-MeS-2-thienyl |
| 5-$NO_2$-2-thienyl | 5-MeO-2-thienyl |
| 5-Me-2-thienyl | 5-BuO-2-thienyl |

TABLE 9

Compounds of Formula I wherein

$R^1$ = Me, $R^2$ = 4-PhO-Ph, $R^3$ = Ph, $R^4$ = H, A = N-C(=O)$SR^{18}$ and:

| $R^{18}$ | $R^{18}$ |
|---|---|
| Me | $CH_2(CH_2)_{11}OCH_2CH_2$ |
| octadecyl | $EtOCH_2CH_2$ |
| Et | $MeSCH_2CH_2$ |
| Pr | $CH_3(CH_2)_{11}SCH_2CH_2$ |
| *i*-Pr | Bzyl |
| Bu | Ph-(CH)Me |
| *i*-Bu | 1-naphthalenylmethyl |
| *sec*-Bu | 2-naphthalenylmethyl |
| $ClCH_2CH_2$ | 2-(2-naphthalenyl)ethyl |
| $Cl_2CHCH_2$ | $PhOCH_2CH_2$ |
| $Cl_3CCH_2$ | 4-Cl-Bzl |
| $BrCH_2CH_2$ | 2,4-diCl-Bzl |
| $F(CH_2)_3$ | 4-Br-Bzl |
| $CF_3CH_2$ | 4-F-Bzl |
| $ICH_2CH_2$ | 4-I-Bzl |
| $MeOCH_2CH_2$ | 4-$NO_2$-Bzl |

$R^{18}$

4-Me-Bzl

4-Bu-Bzl

4-$CF_3$-Bzl

4-MeS-Bzl

4-MeO-Bzl

4-BuO-Bzl

1-Cl-2-naphthalenylmethyl

1,4-diCl-naphthalenylmethyl

1-Br-2-naphthalenylmethyl

1-F-2-naphthalenylmethyl

1-I-2-naphthalenylmethyl

1-$NO_2$-2-naphthalenylmethyl

1-Me-2-naphthalenylmethyl

1-Bu-2-naphthalenylmethyl

1-$CF_3$-2-naphthalenylmethyl

1-MeS-2-naphthalenylmethyl

1-MeO-2-naphthalenylmethyl

1-BuO-2-naphthalenylmethyl

(4-Cl-Ph)$OCH_2CH_2$

(2,4-diCl-Ph)$OCH_2CH_2$

(4-F-Ph)$OCH_2CH_2$

(4-I-Ph)$OCH_2CH_2$

(4-$NO_2$-Ph)$OCH_2CH_2$

(4-Me-Ph)$OCH_2CH_2$

(4-Bu-Ph)$OCH_2CH_2$

(4-$CF_3$-Ph)$OCH_2CH_2$

(4-MeS-Ph)$OCH_2CH_2$

(4-MeO-Ph)$OCH_2CH_2$

(4-BuO-Ph)$OCH_2CH_2$

$R^{18}$

$CH_2=CH$

$CH_3(CH_2)_6CH=CHCH_2$

$CH_2=CHCH_2$

$ClCH_2CH=CHCH_2$

$Cl_3CCH=CHCH_2$

$BrCH_2CH=CHCH_2$

$FCH_2CH=CHCH_2$

$ICH_2CH=CHCH_2$

$MeOCH_2CH=CHCH_2$

$BuOCH_2CH=CHCH_2$

$HC\equiv CCH_2$

$CH_3(CH_2)_4C\equiv CCH_2$

cyclopropyl

cyclopentyl

cyclohexyl

cycloheptyl

cyclododecyl

2-cyclopentenyl

2-cyclohexenyl

2-Me-cyclopentyl

2-Me-cyclohexyl

cyclopentylmethyl

cyclohexylmethyl

1-naphthalenyl

2-naphthalenyl

2-thienyl

3-thienyl

4-Cl-Ph

2,4-diCl-Ph

| $R^{18}$ | $R^{18}$ |
|---|---|
| 4-Br-Ph | 1-Bu-2-naphthalenyl |
| 4-F-Ph | 1-$CF_3$-2-naphthalenyl |
| 4-I-Ph | 1-MeS-2-naphthalenyl |
| 4-$NO_2$-Ph | 1-MeO-2-naphthalenyl |
| 4-Me-Ph | 1-BuO-2-naphthalenyl |
| 4-Bu-Ph | 5-Cl-2-thienyl |
| 4-$CF_3$-Ph | 4,5-diCl-thienyl |
| 4-MeS-Ph | 5-Br-2-thienyl |
| 4-MeO-Ph | 5-F-2-thienyl |
| 4-BuO-Ph | 5-I-2-thienyl |
| 1-Cl-2-naphthalenyl | 5-$NO_2$-2-thienyl |
| 1,3-diCl-2-naphthalenyl | 5-Me-2-thienyl |
| 1-Br-2-naphthalenyl | 5-Bu-2-thienyl |
| 1-F-2-naphthalenyl | 5-$CF_3$-2-thienyl |
| 1-I-2-naphthalenyl | 5-MeS-2-thienyl |
| 1-$NO_2$-2-naphthalenyl | 5-MeO-2-thienyl |
| 1-Me-2-naphthalenyl | 5-BuO-2-thienyl |

TABLE 10

Compounds of Formula I wherein

$R^1$ = Me, $R^2$ = 4-PhO-Ph, $R^3$ = Ph, $R^4$ = H, A = N-C(=O)$NR^{19}R^{20}$ and:

| $R^{19}$ | $R^{20}$ | $R^{19}$ | $R^{20}$ |
|---|---|---|---|
| H | H | Bu | H |
| Me | H | decyl | H |
| Et | H | cyclopentyl | H |
| Pr | H | cyclohexyl | H |
| i-Pr | H | Ph | H |

| $R^{19}$ | $R^{20}$ | $R^{19}$ | $R^{20}$ |
|---|---|---|---|
| 4-Cl-Ph | H | Me | Me |
| 3,5-diCl-Ph | H | Me | Et |
| 4-Br-Ph | H | Et | Et |
| 2-F-Ph | H | Pr | Pr |
| 4-I-Ph | H | Bu | Bu |
| $4-NO_2-Ph$ | H | $-CH_2CH_2CH_2CH_2-$ | |
| 4-Me-Ph | H | $-CH_2CH_2CH_2CH_2CH_2-$ | |
| 4-Bu-Ph | H | $-CH_2CH_2OCH_2CH_2-$ | |
| $4-CF_3-Ph$ | H | $-CH_2(CH_2)_4CH_2-$ | |
| 4-MeS-Ph | H | $-CH_2CH(Me)CH_2CH(Me)CH_2-$ | |
| 4-MeO-Ph | H | $-CH_2CH(Me)OCH(Me)CH_2-$ | |
| 4-BuO-Ph | H | | |

## TABLE 11

Compounds of Formula I wherein:

$R^1$ = Me, $R^2$ = 4-PhO-Ph, $R^3$ = Ph, $R^4$ = H, A = N-P(=O)LM and:

| L | M | L | M |
|---|---|---|---|
| Me | Me | Pr | Pr |
| Me | Et | Bu | Bu |
| Et | Et | | |

TABLE 12

Compounds of Formula I wherein

$R^1$ = Me, $R^2$ = 4-PhO-Ph, $R^3$ = Ph, $R^4$ = H, A = N-OG and:

| G | G |
|---|---|
| H | $CH_3(CH_2)_3C(=O)$ |
| H (HBr salt) | MeOC(=O) |
| Me | EtOC(=O) |
| Me (HBr salt) | PrOC(=O) |
| hexyl | BuOC(=O) |
| Bzl | i-BuOC(=O) |
| 4-Cl-Bzl | MeNHC(=O) |
| 2,4-diCl-Bzl | BuNHC(=O) |
| 4-Br-Bzl | $CH_3(CH_2)_5NHC(=O)$ |
| 4-F-Bzl | Ph-NH(C=O) |
| 4-I-Bzl | (4-Cl-Ph)NH(C=O) |
| 4-$NO_2$-Bzl | (3,5-diCl-Ph)NH(C=O) |
| 4-Me-Bzl | (4-Br-Ph)NH(C=O) |
| 4-Bu-Bzl | (2-F-Ph)NHC(=O) |
| 4-$CF_3$-Bzl | (2-I-Ph)NHC(=O) |
| 4-MeS-Bzl | (4-$NO_2$-Ph)NHC(=O) |
| 4-MeO-Bzl | (4-Me-Ph)NHC(=O) |
| 4-BuO-Bzl | (4-Bu-Ph)NHC(=O) |
| MeC(=O) | (4-$CF_3$-Ph)NHC(=O) |
| $MeCH_2C(=O)$ | (4-MeS-Ph)NHC(=O) |
| $MeC(CH_2)_2C(=O)$ | (4-MeO-Ph)NHC(=O) |
| $CH_3CH(CH_3)C(=O)$ | (4-BuO-Ph)NHC(=O) |

TABLE 13

Compounds of Formula I wherein

$R^1$ = Me, $R^2$ = 2,4-di-F-Ph, $R^3$ = Ph, $R^4$ = H, A = N-J and:

| J | J |
|---|---|
| H | EtOC(=O)C(=O) |
| H (HBr salt) | MeOC(=O) |
| Me | EtC(=O) |
| Bzl | PrC(=O) |
| HC(=O) | BuC(=O) |
| MeC(=O) | $CH_2$=$CHCH_2OC$(=O) |
| EtC(=O) | MeSC(=O) |
| $Me_3CC$(=O) | EtSC(=O) |
| $BrCH_2C$(=O) | $H_2NC$(=O) |
| $CH_2$=CH-C(=O) | MeNHC(=O) |
| $CH_2$=$C(CH_3)$-C(=O) | PhNHC(=O) |
| PhC(=O) | (4-Cl-Ph)NHC(=O) |
| 4-Cl-PhC(=O) | (3,5-diCl-Ph)NHC(=O) |
| 2-naphthoyl | (4-Me-Ph)NHC(=O) |
| 2-furoyl | HO |
| 2-thienyl | MeO |
| PhC(=O)C(=O) | $CH_3C$(=O)O |
| 3-pyridinyl-C(=O) | MeOC(=O)O |
| 4-pyridinyl-C(=O) | MeNHC(=O)O |
| MeOC(=O)C(=O) | PhNHC(=O)O |
| | $Me_2P$(=O) |

TABLE 14

Compounds of Formula I wherein

$R^1$ = Me, $R^2$ = octyl, $R^3$ = Ph, $R^4$ = H, A = N-J and:

| J | J |
|---|---|
| H | EtOC(=O)C(=O) |
| H (HBr salt) | MeOC(=O) |
| Me | EtC(=O) |
| Bzl | PrC(=O) |
| HC(=O) | BuC(=O) |
| MeC(=O) | $CH_2$=$CHCH_2OC$(=O) |
| EtC(=O) | MeSC(=O) |
| $Me_3CC$(=O) | EtSC(=O) |
| $BrCH_2C$(=O) | $H_2NC$(=O) |
| $CH_2$=CH-C(=O) | MeNHC(=O) |
| $CH_2$=C($CH_3$)-C(=O) | PhNHC(=O) |
| PhC(=O) | (4-Cl-Ph)NHC(=O) |
| 4-Cl-PhC(=O) | (3,5-diCl-Ph)NHC(=O) |
| 2-naphthoyl | (4-Me-Ph)NHC(=O) |
| 2-furoyl | HO |
| 2-thienyl | MeO |
| PhC(=O)C(=O) | $CH_3C$(=O)O |
| 3-pyridinyl-C(=O) | MeOC(=O)O |
| 4-pyridinyl-C(=O) | MeNHC(=O)O |
| MeOC(=O)C(=O) | PhNHC(=O)O |
| | $Me_2P$(=O) |

## TABLE 15

Compounds of Formula I wherein

$R^1$ = Me, $R^2$ = 2-F-6-PhO-3-pyridinyl, $R^3$ = Ph, $R^4$ = H, A = N-J and:

| J | J |
|---|---|
| H | EtOC(=O)C(=O) |
| H (HBr salt) | MeOC(=O) |
| Me | EtC(=O) |
| Bzl | PrC(=O) |
| HC(=O) | BuC(=O) |
| MeC(=O) | $CH_2$=CHC$H_2$OC(=O) |
| EtC(=O) | MeSC(=O) |
| $Me_3$CC(=O) | EtSC(=O) |
| BrC$H_2$C(=O) | $H_2$NC(=O) |
| $CH_2$=CH-C(=O) | MeNHC(=O) |
| $CH_2$=C($CH_3$)-C(=O) | PhNHC(=O) |
| PhC(=O) | (4-Cl-Ph)NHC(=O) |
| 4-Cl-PhC(=O) | (3,5-diCl-Ph)NHC(=O) |
| 2-naphthoyl | (4-Me-Ph)NHC(=O) |
| 2-furoyl | HO |
| 2-thienyl | MeO |
| PhC(=O)C(=O) | $CH_3$C(=O)O |
| 3-pyridinyl-C(=O) | MeOC(=O)O |
| 4-pyridinyl-C(=O) | MeNHC(=O)O |
| MeOC(=O)C(=O) | PhNHC(=O)O |
| | $Me_2$P(=O) |

TABLE 16

Compounds of Formula I wherein

$R^1$ = Me, $R^2$ = 4-(benzoyloxy)Ph, $R^3$ = Ph, $R^4$ = H, A = N-J and:

| J | J |
|---|---|
| H | EtOC(=O)C(=O) |
| H (HBr salt) | MeOC(=O) |
| Me | EtC(=O) |
| Bzl | PrC(=O) |
| HC(=O) | BuC(=O) |
| MeC(=O) | $CH_2$=CHCH$_2$OC(=O) |
| EtC(=O) | MeSC(=O) |
| $Me_3$CC(=O) | EtSC(=O) |
| BrCH$_2$C(=O) | $H_2$NC(=O) |
| $CH_2$=CH-C(=O) | MeNHC(=O) |
| $CH_2$=C(CH$_3$)-C(=O) | PhNHC(=O) |
| PhC(=O) | (4-Cl-Ph)NHC(=O) |
| 4-Cl-PhC(=O) | (3,5-diCl-Ph)NHC(=O) |
| 2-naphthoyl | (4-Me-Ph)NHC(=O) |
| 2-furoyl | HO |
| 2-thienyl | MeO |
| PhC(=O)C(=O) | $CH_3$C(=O)O |
| 3-pyridinyl-C(=O) | MeOC(=O)O |
| 4-pyridinyl-C(=O) | MeNHC(=O)O |
| MeOC(=O)C(=O) | PhNHC(=O)O |
| | $Me_2$P(=O) |

TABLE 17

Compounds of Formula I wherein

$R^2$ = 2,4-di-F-Ph, $R^3$ = Ph, $R^4$ = H, A = N-H and:

| $R^1$ | $R^1$ |
|---|---|
| Me | Et |
| Bu | $CF_3$ |

| $R^1$ | $R^1$ |
|---|---|
| $CF_3(CH_2)_3$ | 4-Br-Bzl |
| allyl | 4-I-Bzl |
| cyclopropyl | 4-$NO_2$-Bzl |
| cyclohexyl | 4-Me-Bzl |
| vinyl | 4-Bu-Bzl |
| $CH_3CH{=}CHCH_2$ | 4-$CF_3$-Bzl |
| MeO(C=O) | 4-MeS-Bzl |
| PrO(C=O) | 4-MeO-Bzl |
| Bzl | 4-BuO-Bzl |
| 4-Cl-Bzl | Ph-(CH)-Me |
| 2,4-diCl-Bzl | Ph-(CH)-Bu |

TABLE 18

Compounds of Formula I wherein

$R^1$ = Me, $R^2$ = 2,4-di-F-Ph, $R^4$ = H, A = N-H and:

| $R^3$ | $R^3$ |
|---|---|
| 2-pyridyl | 4-F-Ph |
| 3-pyridyl | 4-I-Ph |
| 4-pyridyl | 4-$NO_2$-Ph |
| 2-pyrimidinyl | 4-NC-Ph |
| 4-pyrimidinyl | 4-Me-Ph |
| 5-pyrimidinyl | 4-Bu-Ph |
| 4-Cl-Ph | 4-$CF_3$-Ph |
| 3,4-diCl-Ph | 4-MeO-Ph |
| 4-Br-Ph | 4-BuO-Ph |

| $R^3$ | $R^3$ |
|---|---|
| 4-MeS-Ph | 3-Cl-2-pyrimidinyl |
| 4-BuS-Ph | 3,5-diCl-2-pyrimidinyl |
| 4-$CF_3$O-Ph | 3-Br-2-pyrimidinyl |
| 5-Cl-2-pyridyl | 3-F-2-pyrimidinyl |
| 3,5-diCl-2-pyridyl | 3-I-2-pyrimidinyl |
| 5-Br-2-pyridyl | 3-$NO_2$-2-pyrimidinyl |
| 5-F-2-pyridyl | 3-NC-2-pyrimidinyl |
| 5-I-2-pyridyl | 3-Me-2-pyrimidinyl |
| 5-$NO_2$-2-pyridyl | 3,5-di-Me-2-pyrimidinyl |
| 5-CN-2-pyridyl | 3-Bu-2-pyrimidinyl |
| 6-Me-2-pyridyl | 3-$CF_3$-2-pyrimidinyl |
| 6-Bu-2-pyridyl | 3-MeO-2-pyrimidinyl |
| 5-$CF_3$-2-pyridyl | 3,5-di-MeO-2-pyrimidinyl |
| 5-MeO-2-pyridyl | 3-BuO-2-pyrimidinyl |
| 5-BuO-2-pyridyl | 3-MeS-2-pyrimidinyl |
| 5-MeS-2-pyridyl | 3-BuS-2-pyrimidinyl |
| 5-BuS-2-pyridyl | 3-$CF_3$O-2-pyrimidinyl |
| 5-$CF_3$O-2-pyridyl | Bzl |

**TABLE 19**

Compounds of Formula I wherein

$R^1$ = Me, $R^3$ = Ph, $R^4$ = Me, A = N-H and:

| $R^2$ | $R^2$ |
|---|---|
| Ph | octyl |
| 4-PhO-Ph | 2-F-6-PhO-3-pyridinyl |
| 2,4-di-F-Ph | 4-[Ph(C=O)O]Ph |

## TABLE 20

Compounds of Formula Ia wherein

$R^1$ = Me, $R^3$ = Ph, $R^4$ = H and:

| $R^2$ | $R^2$ |
|---|---|
| Et | 2-(MeS(O))Et |
| heptyl | 2-($EtSO_2$)Et |
| octyl | EtOC(=O)$(CH_2)_4$ |
| decyl | 3-hydroxypropyl |
| undecyl | $HO_2C(CH_2)_7$ |
| eicosanyl | $(MeO)_2P(=O)OCH_2(CH_2)_3CH_2$ |
| 3-Me-Bu | $(BuO)_2P(=S)OCH_2(CH_2)_3CH_2$ |
| 3-Me-hexyl | 8-($MeSO_3$)octyl |
| 2-Me-pentyl | 5-($BuSO_3$)pentyl |
| 9,9,9-triF-nonyl | $(PhSO_3)CH_2CH_2$ |
| 2,4-diCl-octyl | 3-(4-F-$PhSO_3$)propyl |
| 4-(*c*-propyl)Bu | (4-Et-$PhSO_3$)Bu$(CH_2)_4$ |
| 8-(*c*-hexyl)octyl | $(Me_3N^+)(CH_2)_4$ $I^-$ |
| 6-Ph-octyl | $(H_3N^+)(CH_2)_4$ $Cl^-$ |
| 4-(3-$CF_3$-Ph)Bu | MeC(=O)NMe$(CH_2)_4$ |
| 8-(4-$F_3$CO-Ph)octyl | 3-($PhSO_2$)propyl |
| 10-(2,4-diF-Ph)decyl | (2,6-diMe-$PhSO_2$)$(CH_2)_4$ |
| 4-(4-Cl-Ph)Bu | 6-(2-EtO-$PhSO_2$)hexyl |
| 5-(4-Et-Ph)pentyl | 8-(PhS(O))octyl |
| 8-(3-propyloxy-Ph)octyl | 7-(4-F-PhS(O))heptyl |
| 6-(4-(4-F-PhO)Ph)hexyl | (3-Me-PhS(O))$CH_2CH_2$ |
| 7-(3-(4-Et-PhO)Ph)heptyl | 5-(3-BuO-PhS(O))pentyl |
| 10-(4-(3-MeS-PhO)Ph)decyl | (4-pyridyl)$CH_2$ |
| 2-CN-propyl | $Me_2N(CH_2)_6$ |
| 3-$NO_2$-propyl | 8-(2,6-diMe-4-pyridyl)octyl |
| 2-MeS-hexyl | 10-(6-Cl-3-pyridyl)decyl |

$R^2$

6-(4-pyridyloxy)hexyl
3-(2,6-diCl-4-pyridyloxy) propyl
(4-MeO-2-pyridyloxy)$(CH_2)_4$
3-(2-thienyl)propyl
6-(3-Me-2-thienyl)hexyl
8-(2-pyrimidinyl)octyl
7-(4-MeO-2-pyrimidinyl)heptyl
10-(2-furanyl)decyl
(5-Et-2-furanyl)$(CH_2)_4$
3-(1-naphthalenyl)propyl
6-(6-F-2-naphthalenyl)hexyl
5-(2-pyrimidinyloxy)pentyl
8-(1-naphthalenyloxy)octyl
3-(2-tetrahydropyranyl)propyl
8-(2-tetrahydropyranyloxy) octyl
2-octenyl
2-decenyl
4-octenyl
3-EtS-2-propenyl
4-*c*-hexyloxy-2-butenyl
5-*c*-pentyloxy-3-pentenyl
6-hydroxy-4-hexenyl
3-(MeC(=O)O)-2-propenyl
3-(4-pyridyl)-2-propenyl
5-EtO-2-pentenyl
3-$CF_3$-2-propenyl

$R^2$

3-Ph-2-propenyl
4-PhO-2-butenyl
(Z)-(4-PhO-2-butenyl)
7-octynyl
2-hydroxy-4-butynyl
2-EtO-6-heptynyl
2,4-diF-7-octynyl
3-*c*-hexenyl
3-*c*-pentenyl
$(CH_2CH_2OCH_2CH_2)CH$
$(CH_2CH_2SCH_2CH_2)CH$
$(CH_2CH_2SO_2CH_2CH_2)CH$
$PhOCH_2CH_2CH_2$
$PhO(CH_2)_4$
$PhO(CH_2)_5$
$PhO(CH_2)_8$
(3-$CF_3$-PhO)$(CH_2)_3$
(3,5-diCl-PhO)$(CH_2)_6$
(2-F-PhO)$(CH_2)_3$
(2-Me-PhO)$(CH_2)_3$
(3,5-diMeO-PhO)$(CH_2)_8$
(4-(4-F-PhO)PhO)$(CH_2)_3$
(3-(3,5-diMe-PhO)PhO)$(CH_2)_6$
$PhS(CH_2)_3$
(3-$CF_3$-PhS)$(CH_2)_7$
(3-$F_3$CO-PhS)$(CH_2)_6$

| $R^2$ |
|---|
| (2,4-diF-PhS)$(CH_2)_5$ |
| (4-Et-PhS)$(CH_2)_5$ |
| (2-EtO-PhS)$(CH_2)_{10}$ |
| (3-(4-Cl-PhO)PhS)$(CH_2)_7$ |
| (3-(2,4-diMe-PhO)PhS)$(CH_2)_3$ |
| (4-(4-EtO-PhO)PhS)$CH_2)_7$ |
| (3-(2-MeS-PhO)PhS)$(CH_2)_3$ |
| $FCH_2CH_2O(CH_2)_6$ |
| (*c*-hexyloxy)$(CH_2)_6$ |
| (*c*-pentyloxy)$(CH_2)_8$ |
| $(CH_2{=}CHCH_2O)(CH_2)_7$ |
| $CH{\equiv}CCH_2OCH_2CH_2$ |
| $CH({=}O)CH_2CH_2$ |
| $MeC({=}O)CH_2CH_2CH_2$ |
| $BuC({=}O)(CH_2)_7$ |
| $MeC({=}NMe)CH_2$ |
| $MeC({=}NOMe)CH_2CH_2CH_2$ |
| $MeC({=}NNHPh)CH_2CH_2CH_2$ |
| hexyl |
| cyclopentyl |
| cyclohexyl |
| vinyl |
| 3-hexenyl |
| 2-cyclohexenyl |
| 4-F-Ph |
| 4-Cl-Ph |

| $R^2$ |
|---|
| 3-Cl-Ph |
| 3-F-Ph |
| 4-Br-Ph |
| 3,4-diCl-Ph |
| 2,3-diF-Ph |
| Ph |
| 2,5-diF-Ph |
| 3,5-diF-Ph |
| 2,6-diF-Ph |
| 3,4-diF-Ph |
| 5-Cl-2-F-Ph |
| 2-F-5-Me-Ph |
| 2,5-diF-4-Me-Ph |
| 2-F-Ph |
| 4-Me-Ph |
| 4-$NO_2$-Ph |
| 4-hexyl-Ph |
| 4-(cyclohexyl)Ph |
| 4-$CF_3$-Ph |
| 4-MeS-Ph |
| 4-(hexylthio)Ph |
| 3-MeO-Ph |
| 4-(pentyloxy)Ph |
| 4-$CF_3O$-Ph |
| 4-(cyclohexyloxy)Ph |
| 4-($BuOCH_2$)Ph |

| $R^2$ | $R^2$ |
|---|---|
| 4-$MeOCH_2$-Ph | 4-(4-MeOC(=O)-PhO)Ph |
| 4-(2-propenyl)Ph | 4-(4-allyl-PhO)Ph |
| 4-(3-pentenyl)Ph | 4-(4-propargyl-PhO)Ph |
| 4-($Cl_2C$=$CHCH_2$)Ph | 4-((3-PhO)PhO)Ph |
| 4-(2-propenyloxy)Ph | 4-(3-(2-Cl-PhO)PhO)Ph |
| 4-(2-hexenyloxy)Ph | 4-(3-(4-Me-PhO)PhO)Ph |
| 4-(propargyl)Ph | 4-(3-(3-MeO-PhO)PhO)Ph |
| 4-(2-butynyloxy)Ph | 4-PhCH=CH-Ph |
| 4-$BuSO_2$-Ph | 4-(3-Br-Ph)CH=CH-Ph |
| 4-PhO-Ph | 4-(2-F-Ph)CH=CH-Ph |
| 4-(4-Cl-PhO)Ph | 4-(3-Cl-Ph)CH=CH-Ph |
| 4-(3-Cl-PhO)Ph | 4-(2,4-diF-Ph)CH=CH-Ph |
| 4-(2,4-diF-PhO)Ph | 4-(4-$NO_2$-Ph)CH=CH-Ph |
| 4-(4-$NO_2$-PhO)Ph | 4-(3-Me-Ph)CH=CH-Ph |
| 4-(4-CN-PhO)Ph | 4-(3-Bu-Ph)CH=CH-Ph |
| 4-(4-Me-PhO)Ph | 4-(4-$CF_3$-Ph)CH=CH-Ph |
| 4-(3-hexyl-PhO)Ph | 4-(2-MeS-Ph)CH=CH-Ph |
| 4-(3-cyclohexyl-PhO)Ph | 4-(4-MeO-Ph)CH=CH-Ph |
| 4-(3-$CF_3$-PhO)Ph | 4-(4-BuO-Ph)CH=CH-Ph |
| 4-(4-MeS-PhO)Ph | 4-$PhCH_2CH_2$-Ph |
| 4-(4-$MeSO_2$-PhO)Ph | 4-(3-Br-Ph)$CH_2CH_2$-Ph |
| 4-(3-$BuSO_2$-PhO)Ph | 4-(2-F-Ph)$CH_2CH_2$-Ph |
| 4-(3-MeO-PhO)Ph | 4-(3-Cl-Ph)$CH_2CH_2$-Ph |
| 4-(3-hexyloxy-PhO)Ph | 4-(2,4-diF-Ph)$CH_2CH_2$-Ph |
| 4-(4-$CF_3O$-PhO)Ph | 4-(4-$NO_2$-Ph)$CH_2CH_2$-Ph |
| 4-(4-cyclohexyloxy-PhO)Ph | 4-(3-Me-Ph)$CH_2CH_2$-Ph |

| $R^2$ | $R^2$ |
|---|---|
| 4-(3-Bu-Ph)$CH_2CH_2$-Ph | 4-(2-MeS-Ph)$CH_2$-Ph |
| 4-(4-$CF_3$-Ph)$CH_2CH_2$-Ph | 4-(4-MeO-Ph)$CH_2$-Ph |
| 4-(2-MeS-Ph)$CH_2CH_2$-Ph | 4-(4-BuO-Ph)$CH_2$-Ph |
| 4-(4-MeO-Ph)$CH_2CH_2$-Ph | 4-PhS-Ph |
| 4-(4-BuO-Ph)$CH_2CH_2$-Ph | 4-(3-Br-Ph)S-Ph |
| 4-Ph$OCH_2$-Ph | 4-(2-F-Ph)S-Ph |
| 4-(3-Br-Ph)$OCH_2$-Ph | 4-(3-Cl-Ph)S-Ph |
| 4-(2-F-Ph)$OCH_2$-Ph | 4-(2,4-diF-Ph)S-Ph |
| 4-(3-Cl-Ph)$OCH_2$-Ph | 4-(4-$NO_2$-Ph)S-Ph |
| 4-(2,4-diF-Ph)$OCH_2$-Ph | 4-(3-Me-Ph)S-Ph |
| 4-(4-$NO_2$-Ph)$OCH_2$-Ph | 4-(3-Bu-Ph)S-Ph |
| 4-(3-Me-Ph)$OCH_2$-Ph | 4-(4-$CF_3$-Ph)S-Ph |
| 4-(3-Bu-Ph)$OCH_2$-Ph | 4-(2-MeS-Ph)S-Ph |
| 4-(4-$CF_3$-Ph)$OCH_2$-Ph | 4-(4-MeO-Ph)S-Ph |
| 4-(2-MeS-Ph)$OCH_2$-Ph | 4-(4-BuO-Ph)S-Ph |
| 4-(4-MeO-Ph)$OCH_2$-Ph | 4-Ph-Ph |
| 4-(4-BuO-Ph)$OCH_2$-Ph | 4-(3-Br-Ph)Ph |
| 4-Ph$CH_2$-Ph | 4-(2-F-Ph)Ph |
| 4-(3-Br-Ph)$CH_2$-Ph | 4-(3-Cl-Ph)Ph |
| 4-(2-F-Ph)$CH_2$-Ph | 4-(2,4-diF-Ph)Ph |
| 4-(3-Cl-Ph)$CH_2$-Ph | 4-(4-$NO_2$-Ph)Ph |
| 4-(2,4-diF-Ph)$CH_2$-Ph | 4-(3-Me-Ph)Ph |
| 4-(4-$NO_2$-Ph)$CH_2$-Ph | 4-(3-Bu-Ph)Ph |
| 4-(3-Me-Ph)$CH_2$-Ph | 4-(4-$CF_3$-Ph)Ph |
| 4-(3-Bu-Ph)$CH_2$-Ph | 4-(2-MeS-Ph)Ph |
| 4-(4-$CF_3$-Ph)$CH_2$-Ph | 4-(4-MeO-Ph)Ph |

$R^2$

4-(4-BuO-Ph)Ph

4-PhCH$_2$O-Ph

4-(3-Br-Ph)CH$_2$O-Ph

4-(2-F-Ph)CH$_2$O-Ph

4-(3-Cl-Ph)CH$_2$O-Ph

4-(2,4-diF-Ph)CH$_2$O-Ph

4-(4-NO$_2$-Ph)CH$_2$O-Ph

4-(3-Me-Ph)CH$_2$O-Ph

4-(3-Bu-Ph)CH$_2$O-Ph

4-(4-CF$_3$-Ph)CH$_2$O-Ph

4-(2-MeS-Ph)CH$_2$O-Ph

4-(4-MeO-Ph)CH$_2$O-Ph

4-(4-BuO-Ph)CH$_2$O-Ph

4-(4-CN-Ph)CH$_2$O-Ph

4-(3-PhO-Ph)CH$_2$O-Ph

4-[3-(3-F-PhO)Ph]CH$_2$O-Ph

4-[4-(2-NO$_2$-PhO)Ph]CH$_2$O-Ph

4-[3-(4-Me-PhO)Ph]CH$_2$O-Ph

4-[4-(4-Bu-PhO)Ph]CH$_2$O-Ph

4-[3-(3-CF$_3$-PhO)Ph]CH$_2$O-Ph

4-[2-(3-MeS-PhO)Ph]CH$_2$O-Ph

4-[4-(4-MeO-PhO)Ph]CH$_2$O-Ph

4-[3-(3-BuO-PhO)Ph]CH$_2$O-Ph

4-(2-F-4-MeO-Ph)CH$_2$O-Ph

3-(2-CF$_3$-4-Et-Ph)CH$_2$O-Ph

4-[MeNHC(=O)O]Ph

$R^2$

4-[octyl-NHC(=O)O]Ph

4-[PhNHC(=O)O]Ph

4-[(3-Br-Ph)NHC(=O)O]Ph

4-[(2-F-Ph)NHC(=O)O]Ph

4-[(3-Cl-Ph)NHC(=O)O]Ph

4-[(2,4-diF-Ph)NHC(=O)O]Ph

4-[(4-NO$_2$-Ph)NHC(=O)O]Ph

4-[(3-Me-Ph)NHC(=O)O]Ph

4-[(3-Bu-Ph)NHC(=O)O]Ph

4-[(4-CF$_3$-Ph)NHC(=O)O]Ph

4-[(2-MeS-Ph)NHC(=O)O]Ph

4-[(4-MeO-Ph)NHC(=O)O]Ph

4-[(4-BuO-Ph)NHC(=O)O]Ph

4-[(4-CN-Ph)NHC(=O)O]Ph

4-[(3-PhO-Ph)NHC(=O)O]Ph

4-[3-(3-F-PhO)Ph-NHC(=O)O]Ph

4-[4-(2-NO$_2$-PhO)Ph-NHC(=O)O]Ph

4-[2-(4-Me-PhO)Ph-NHC(=O)O]Ph

4-[4-(4-Bu-PhO)Ph-NHC(=O)O]Ph

4-[4-(3-CF$_3$-PhO)Ph-NHC(=O)O]Ph

4-[4-(3-MeS-PhO)Ph-NHC(=O)O]Ph

4-[3-(4-MeO-PhO)Ph-NHC(=O)O]Ph

4-[3-(3-BuO-PhO)Ph-NHC(=O)O]Ph

4-[(2-F-4-MeO-Ph)NHC(=O)O]Ph

3-[(2-CF$_3$-4-Et-Ph)NHC(=O)O]Ph

4-[(3-pyridyl)NHC(=O)O]Ph

$R^2$

4-[(3-Br-2-pyridyl)NHC(=O)O]Ph
4-[(2-F-3-pyridyl)NHC(=O)O]Ph
4-[(3-Cl-4-pyridyl)NHC(=O)O]Ph
4-[(2,4-diF-3-pyridyl)NHC(=O)O]Ph
4-[(4-$NO_2$-2-pyridyl)NHC(=O)O]Ph
4-[(3-Me-2-pyridyl)NHC(=O)O]Ph
4-[(3-Bu-2-pyridyl)NHC(=O)O]Ph
4-[(4-$CF_3$-3-pyridyl)NHC(=O)O]Ph
4-[(2-MeS-4-pyridyl)NHC(=O)O]Ph
4-[(4-MeO-3-pyridyl)NHC(=O)O]Ph
4-[(4-BuO-3-pyridyl)NHC(=O)O]Ph
4-[(4-CN-2-pyridyl)NHC(=O)O]Ph
4-[(3-PhO-2-pyridyl)NHC(=O)O]Ph
4-[3-(3-F-PhO)-2-pyridyl-NHC(=O)O]Ph
4-[4-(2-$NO_2$-PhO)-3-pyridyl-NHC(=O)O]Ph
4-[2-(4-Me-PhO)-4-pyridyl-NHC(=O)O]Ph
4-[4-(4-Bu-PhO)-3-pyridyl-NHC(=O)O]Ph
4-[4-(3-$CF_3$-PhO)-2-pyridyl-NHC(=O)O]Ph
4-[4-(3-MeS-PhO)-3-pyridyl-NHC(=O)O]Ph
4-[3-(4-MeO-PhO)-2-pyridyl-NHC(=O)O]Ph
4-[3-(3-BuO-PhO)-2-pyridyl-NHC(=O)O]Ph
4-[(2-F-4-MeO-3-pyridyl)NHC(=O)O]Ph
3-[(2-$CF_3$-4-Et-3-pyridyl)NHC(=O)O]Ph
4-[MeO-C(=O)]Ph
4-[octyloxy-C(=O)]Ph
4-[PhO-C(=O)]Ph

$R^2$

4-[3-Br-PhO-C(=O)]Ph
4-[2-F-PhO-C(=O)]Ph
4-[3-Cl-PhO-C(=O)]Ph
4-[2,4-diF-PhO-C(=O)]Ph
4-[4-$NO_2$-PhO-(=O)]Ph
4-[3-Me-PhO-C(=O)]Ph
4-[3-Bu-PhO-C(=O)]Ph
4-[4-$CF_3$-PhO-C(=O)]Ph
4-[2-MeS-PhO-C(=O)]Ph
4-[4-MeO-PhO-C(=O)]Ph
4-[4-BuO-PhO-C(=O)]Ph
4-[4-CN-PhO-C(=O)]Ph
4-[3-PhO-PhO-C(=O)]Ph
4-[3-(3-F-PhO)PhO-C(=O)]Ph
4-[4-(2-$NO_2$-PhO)PhO-C(=O)]Ph
4-[2-(4-Me-PhO)PhO-C(=O)]Ph
4-[4-(4-Bu-PhO)PhO-C(=O)]Ph
4-[4-(3-$CF_3$-PhO)PhO-C(=O)]Ph
4-[4-(3-MeS-PhO)PhO-C(=O)]Ph
4-[3-(4-MeO-PhO)PhO-C(=O)]Ph
4-[3-(3-BuO-PhO)PhO-C(=O)]Ph
4-[2-F-4-MeO-PhO-C(=O)]Ph
3-[2-$CF_3$-4-Et-PhO-C(=O)]Ph
4-[(3-pyridyloxy)C(=O)]Ph
4-[(3-Br-2-pyridyloxy)C(=O)]Ph
4-[(2-F-3-pyridyloxy)C(=O)]Ph

$R^2$

4-[(3-Cl-4-pyridyloxy)C(=O)]Ph

4-[(2,4-diF-3-pyridyloxy)C(=O)]Ph

4-[(4-$NO_2$-2-pyridyloxy)C(=O)]Ph

4-[(3-Me-2-pyridyloxy)C(=O)]Ph

4-[(3-Bu-2-pyridyloxy)C(=O)]Ph

4-[(4-$CF_3$-3-pyridyloxy)C(=O)]Ph

4-[(2-MeS-4-pyridyloxy)C(=O)]Ph

4-[(4-MeO-3-pyridyloxy)C(=O)]Ph

4-[(4-BuO-3-pyridyloxy)C(=O)]Ph

4-[(4-CN-2-pyridyloxy)C(=O)]Ph

4-[(3-PhO-2-pyridyloxy)C(=O)]Ph

4-[3-(3-F-PhO)-2-pyridyloxy-C(=O)]Ph

4-[4-(2-$NO_2$-PhO)-3-pyridyloxy-C(=O)]Ph

4-[2-(4-Me-PhO)-4-pyridyloxy-C(=O)]Ph

4-[4-(4-Bu-PhO)-3-pyridyloxy-C(=O)]Ph

4-[4-(3-$CF_3$-PhO)-2-pyridyloxy-C(=O)]Ph

4-[4-(3-MeS-PhO)-3-pyridyloxy-C(=O)]Ph

4-[3-(4-MeO-PhO)-2-pyridyloxy-C(=O)]Ph

4-[3-(3-BuO-PhO)-2-pyridyloxy-C(=O)]Ph

4-[(2-F-4-MeO-3-pyridyloxy)C(=O)]Ph

3-[(2-$CF_3$-4-Et-3-pyridyloxy)C(=O)]Ph

4-[MeC(=O)O]Ph

4-[octyl-C(=O)O]Ph

4-[PhC(=O)O]Ph

4-[3-Br-PhC(=O)O]Ph

4-[2-F-PhC(=O)O]Ph

$R^2$

4-[3-Cl-PhC(=O)O]Ph

4-[2,4-diF-PhC(=O)O]Ph

4-[4-$NO_2$-Ph(=O)O]Ph

4-[3-Me-PhC(=O)O]Ph

4-[3-Bu-PhC(=O)O]Ph

4-[4-$CF_3$-PhC(=O)O]Ph

4-[2-MeS-PhC(=O)O]Ph

4-[4-MeO-PhC(=O)O]Ph

4-[4-BuO-PhC(=O)O]Ph

4-[4-CN-PhC(=O)O]Ph

4-[3-PhO-PhC(=O)O]Ph

4-[3-(3-F-PhO)PhC(=O)O]Ph

4-[4-(2-$NO_2$-PhO)PhC(=O)O]Ph

4-[2-(4-Me-PhO)PhC(=O)O]Ph

4-[4-(4-Bu-PhO)PhC(=O)O]Ph

4-[4-(3-$CF_3$-PhO)PhC(=O)O]Ph

4-[4-(3-MeS-PhO)PhC(=O)O]Ph

4-[3-(4-MeO-PhO)PhC(=O)O]Ph

4-[3-(3-BuO-PhO)PhC(=O)O]Ph

4-[(2-F-4-MeO-Ph)C(=O)O]Ph

3-[(2-$CF_3$-4-Et-Ph)C(=O)O]Ph

4-[(3-pyridyl)C(=O)O]Ph

4-[(3-Br-2-pyridyl)C(=O)O]Ph

4-[(2-F-3-pyridyl)C(=O)O]Ph

4-[(3-Cl-4-pyridyl)C(=O)O]Ph

4-[(2,4-diF-3-pyridyl)C(=O)O]Ph

$R^2$

4-[(4-$NO_2$-2-pyridyl)C(=O)O]Ph
4-[(3-Me-2-pyridyl)C(=O)O]Ph
4-[(3-Bu-2-pyridyl)C(=O)O]Ph
4-[(4-$CF_3$-3-pyridyl)C(=O)O]Ph
4-[(2-MeS-4-pyridyl)C(=O)O]Ph
4-[(4-MeO-3-pyridyl)C(=O)O]Ph
4-[(4-BuO-3-pyridyl)C(=O)O]Ph
4-[(4-CN-2-pyridyl)C(=O)O]Ph
4-[(3-PhO-2-pyridyl)C(=O)O]Ph
4-[3-(3-F-PhO)-2-pyridyl-C(=O)O]Ph
4-[4-(2-$NO_2$-PhO)-3-pyridyl-C(=O)O]Ph
4-[2-(4-Me-PhO)-4-pyridyl-C(=O)O]Ph
4-[4-(4-Bu-PhO)-3-pyridyl-C(=O)O]Ph
4-[4-(3-$CF_3$-PhO)-2-pyridyl-C(=O)O]Ph
4-[4-(3-MeS-PhO)-3-pyridyl-C(=O)O]Ph
4-[3-(4-MeO-PhO)-2-pyridyl-C(=O)O]Ph
4-[3-(3-BuO-PhO)-2-pyridyl-C(=O)O]Ph
4-[(2-F-4-MeO-3-pyridyl)C(=O)O]Ph
3-[(2-$CF_3$-4-Et-3-pyridyl)C(=O)O]Ph
2-F-4-$PhCH_2CH_2$-Ph
3-Cl-4-(2-F-Ph)$OCH_2$-Ph
3-Me-4-(4-MeO-Ph)$CH_2$-Ph
2-F-4-PhS-Ph
3-BuO-4-Ph-Ph
3-MeS-4-PhO-Ph
2-F-4-$PhCH_2O$-Ph

$R^2$

2-F-4-[octyl-NHC(=O)O]Ph
4-F-3-thienyl
4-Cl-2-thienyl
5-Cl-2-thienyl
3-F-2-thienyl
4-Br-2-thienyl
3,4-diCl-2-thienyl
2,4-diF-3-thienyl
2-thienyl
3-thienyl
2,5-diF-3-thienyl
3,5-diF-2-thienyl
3,4-diF-2-thienyl
5-Cl-2-F-3-thienyl
2-F-5-Me-3-thienyl
2,5-diF-4-Me-3-thienyl
2-F-3-thienyl
4-Me-2-thienyl
4-$NO_2$-2-thienyl
4-hexyl-2-thienyl
5-cyclohexyl-3-thienyl
5-$CF_3$-3-thienyl
4-MeS-2-thienyl
4-hexylthio-2-thienyl
5-MeO-3-thienyl
4-pentyloxy-2-thienyl

| $R^2$ | $R^2$ |
|---|---|
| 4-$CF_3$O-2-thienyl | 5-(3-hexyloxy-PhO)-3-thienyl |
| 5-cyclohexyloxy-3-thienyl | 5-(4-$CF_3$O-PhO)-3-thienyl |
| 4-BuO$CH_2$-2-thienyl | 5-(4-cyclohexyloxy-PhO)-3-thienyl |
| 4-MeO$CH_2$-2-thienyl | 4-(4-MeOC(=O)-PhO)-2-thienyl |
| 5-(2-propenyl)-3-thienyl | 4-(4-allyl-PhO)-2-thienyl |
| 5-(3-pentenyl)-3-thienyl | 4-(4-propargyl-PhO)-2-thienyl |
| 4-($Cl_2$C=CH$CH_2$)-2-thienyl | 5-((3-PhO)PhO)-3-thienyl |
| 4-(2-propenyloxy)-2-thienyl | 5-(3-(2-Cl-PhO)PhO)-3-thienyl |
| 4-(2-hexenyloxy)-2-thienyl | 5-(3-(4-Me-PhO)PhO)-3-thienyl |
| 5-(propargyl)-3-thienyl | 5-(3-(3-MeO-PhO)PhO)-3-thienyl |
| 4-(2-butynyloxy)-2-thienyl | 4-PhCH=CH-2-thienyl |
| 5-Bu$SO_2$-3-thienyl | 4-(3-Br-Ph)CH=CH-2-thienyl |
| 5-PhO-3-thienyl | 4-(2-F-Ph)CH=CH-2-thienyl |
| 4-(4-Cl-PhO)-2-thienyl | 4-(3-Cl-Ph)CH=CH-2-thienyl |
| 4-(3-Cl-PhO)-2-thienyl | 5-(2,4-diF-Ph)CH=CH-3-thienyl |
| 5-(2,4-diF-PhO)-3-thienyl | 5-(4-$NO_2$-Ph)CH=CH-3-thienyl |
| 5-(4-$NO_2$-PhO)-3-thienyl | 5-(3-Me-Ph)CH=CH-3-thienyl |
| 5-(4-CN-PhO)-3-thienyl | 5-(3-Bu-Ph)CH=CH-3-thienyl |
| 5-(4-Me-PhO)-3-thienyl | 4-(4-$CF_3$-Ph)CH=CH-2-thienyl |
| 4-(3-hexyl-PhO)-2-thienyl | 4-(2-MeS-Ph)CH=CH-2-thienyl |
| 4-(3-cyclohexyl-PhO)-2-thienyl | 4-(4-MeO-Ph)CH=CH-2-thienyl |
| 4-(3-$CF_3$-PhO)-2-thienyl | 4-(4-BuO-Ph)CH=CH-2-thienyl |
| 4-(4-MeS-PhO)-2-thienyl | 5-Ph$CH_2CH_2$-3-thienyl |
| 5-(4-Me$SO_2$-PhO)-3-thienyl | 5-(3-Br-Ph)$CH_2CH_2$-3-thienyl |
| 5-(3-Bu$SO_2$-PhO)-3-thienyl | 4-(2-F-Ph)$CH_2CH_2$-2-thienyl |
| 5-(3-MeO-PhO)-3-thienyl | 4-(3-Cl-Ph)$CH_2CH_2$-2-thienyl |

$R^2$

4-(2,4-diF-Ph)$CH_2CH_2$-2-thienyl
5-(4-$NO_2$-Ph)$CH_2CH_2$-3-thienyl
5-(3-Me-Ph)$CH_2CH_2$-3-thienyl
4-(3-Bu-Ph)$CH_2CH_2$-2-thienyl
5-(4-$CF_3$-Ph)$CH_2CH_2$-3-thienyl
4-(2-MeS-Ph)$CH_2CH_2$-2-thienyl
5-(4-MeO-Ph)$CH_2CH_2$-3-thienyl
4-(4-BuO-Ph)$CH_2CH_2$-2-thienyl
4-Ph$OCH_2$-2-thienyl
5-(3-Br-Ph)$OCH_2$-3-thienyl
5-(2-F-Ph)$OCH_2$-3-thienyl
4-(3-Cl-Ph)$OCH_2$-2-thienyl
5-(2,4-diF-Ph)$OCH_2$-3-thienyl
5-(4-$NO_2$-Ph)$OCH_2$-3-thienyl
4-(3-Me-Ph)$OCH_2$-2-thienyl
5-(3-Bu-Ph)$OCH_2$-3-thienyl
5-(4-$CF_3$-Ph)$OCH_2$-3-thienyl
4-(2-MeS-Ph)$OCH_2$-2-thienyl
5-(4-MeO-Ph)$OCH_2$-3-thienyl
4-(4-BuO-Ph)$OCH_2$-2-thienyl
5-Ph$CH_2$-3-thienyl
5-(3-Br-Ph)$CH_2$-3-thienyl
5-(2-F-Ph)$CH_2$-3-thienyl
5-(3-Cl-Ph)$CH_2$-3-thienyl
4-(2,4-diF-Ph)$CH_2$-2-thienyl
4-(4-$NO_2$-Ph)$CH_2$-2-thienyl

$R^2$

5-(3-Me-Ph)$CH_2$-3-thienylPh
4-(3-Bu-Ph)$CH_2$-2-thienyl
4-(4-$CF_3$-Ph)$CH_2$-2-thienyl
5-(2-MeS-Ph)$CH_2$-3-thienyl
4-(4-MeO-Ph)$CH_2$-2-thienyl
4-(4-BuO-Ph)$CH_2$-2-thienyl
5-PhS-3-thienyl
4-(3-Br-Ph)S-2-thienyl
5-(2-F-Ph)S-3-thienyl
4-(3-Cl-Ph)S-2-thienyl
5-(2,4-diF-Ph)S-3-thienyl
4-(4-$NO_2$-Ph)S-2-thienyl
5-(3-Me-Ph)S-3-thienyl
4-(3-Bu-Ph)S-2-thienyl
5-(4-$CF_3$-Ph)S-3-thienyl
4-(2-MeS-Ph)S-2-thienyl
5-(4-MeO-Ph)S-3-thienyl
5-(4-BuO-Ph)S-3-thienyl
4-Ph-2-thienyl
4-(3-Br-Ph)-2-thienyl
4-(2-F-Ph)-2-thienyl
5-(3-Cl-Ph)-3-thienyl
5-(2,4-diF-Ph)-3-thienyl
5-(4-$NO_2$-Ph)-3-thienyl
4-(3-Me-Ph)-2-thienyl
5-(3-Bu-Ph)-3-thienyl

$R^2$

4-(4-$CF_3$-Ph)-2-thienyl

5-(2-MeS-Ph)-3-thienyl

5-(4-MeO-Ph)-3-thienyl

4-(4-BuO-Ph)-2-thienyl

5-$PhCH_2O$-3-thienyl

4-(3-Br-Ph)$CH_2O$-2-thienyl

5-(2-F-Ph)$CH_2O$-3-thienyl

5-(3-Cl-Ph)$CH_2O$-3-thienyl

5-(2,4-diF-Ph)$CH_2O$-3-thienyl

4-(4-$NO_2$-Ph)$CH_2O$-2-thienyl

5-(3-Me-Ph)$CH_2O$-3-thienyl

4-(3-Bu-Ph)$CH_2O$-2-thienyl

4-(4-$CF_3$-Ph)$CH_2O$-2-thienyl

5-(2-MeS-Ph)$CH_2O$-3-thienyl

4-(4-MeO-Ph)$CH_2O$-2-thienyl

5-(4-BuO-Ph)$CH_2O$-3-thienyl

4-(4-CN-Ph)$CH_2O$-2-thienyl

5-(3-PhO-Ph)$CH_2O$-3-thienyl

5-[3-(3-F-PhO)Ph]$CH_2O$-3-thienyl

4-[4-(2-$NO_2$-PhO)Ph]$CH_2O$-2-thienyl

4-[3-(4-Me-PhO)Ph]$CH_2O$-2-thienyl

4-[4-(4-Bu-PhO)Ph]$CH_2O$-2-thienyl

5-[3-(3-$CF_3$-PhO)Ph]$CH_2O$-3-thienyl

5-[2-(3-MeS-PhO)Ph]$CH_2O$-3-thienyl

4-[4-(4-MeO-PhO)Ph]$CH_2O$-2-thienyl

5-[3-(3-BuO-PhO)Ph]$CH_2O$-3-thienyl

$R^2$

4-(2-F-4-MeO-Ph)$CH_2$O-2-thienyl

5-(2-$CF_3$-4-Et-Ph)$CH_2$O-3-thienyl

4-[MeNHC(=O)O]-2-thienyl

4-[octyl-NHC(=O)O]-2-thienyl

5-[PhNHC(=O)O]-3-thienyl

5-[(3-Br-Ph)NHC(=O)O]-3-thienyl

4-[(2-F-Ph)NHC(=O)O]-2-thienyl

5-[(3-Cl-Ph)NHC(=O)O]-3-thienyl

5-[(2,4-diF-Ph)NHC(=O)O]-3-thienyl

5-[(4-$NO_2$-Ph)NHC(=O)O]-3-thienyl

4-[(3-Me-Ph)NHC(=O)O]-2-thienyl

5-[(3-Bu-Ph)NHC(=O)O]-3-thienyl

5-[(4-$CF_3$-Ph)NHC(=O)O]-3-thienyl

4-[(2-MeS-Ph)NHC(=O)O]-2-thienyl

4-[(4-MeO-Ph)NHC(=O)O]-2-thienyl

5-[(4-BuO-Ph)NHC(=O)O]-3-thienyl

5-[(4-CN-Ph)NHC(=O)O]-3-thienyl

4-[(3-PhO-Ph)NHC(=O)O]-2-thienyl

4-[3-(3-F-PhO)Ph-NHC(=O)O]-2-thienyl

4-[4-(2-$NO_2$-PhO)Ph-NHC(=O)O]-2-thienyl

4-[2-(4-Me-PhO)Ph-NHC(=O)O]-2-thienyl

5-[4-(4-Bu-PhO)Ph-NHC(=O)O]-3-thienyl

5-[4-(3-$CF_3$-PhO)Ph-NHC(=O)O]-3-thienyl

5-[4-(3-MeS-PhO)Ph-NHC(=O)O]-3-thienyl

4-[3-(4-MeO-PhO)Ph-NHC(=O)O]-2-thienyl

5-[3-(3-BuO-PhO)Ph-NHC(=O)O]-3-thienyl

$R^2$

4-[(2-F-4-MeO-Ph)NHC(=O)O]-2-thienyl

5-[(2-$CF_3$-4-Et-Ph)NHC(=O)O]-3-thienyl

5-[(3-pyridyl)NHC(=O)O]-3-thienyl

4-[(3-Br-2-pyridyl)NHC(=O)O]-2-thienyl

5-[(2-F-3-pyridyl)NHC(=O)O]-3-thienyl

5-[(3-Cl-4-pyridyl)NHC(=O)O]-3-thienyl

5-[(2,4-diF-3-pyridyl)NHC(=O)O]-3-thienyl

4-[(4-$NO_2$-2-pyridyl)NHC(=O)O]-2-thienyl

4-[(3-Me-2-pyridyl)NHC(=O)O]-2-thienyl

5-[(3-Bu-2-pyridyl)NHC(=O)O]-3-thienyl

5-[(4-$CF_3$-3-pyridyl)NHC(=O)O]-3-thienyl

4-[(2-MeS-4-pyridyl)NHC(=O)O]-2-thienyl

5-[(4-MeO-3-pyridyl)NHC(=O)O]-3-thienyl

4-[(4-BuO-3-pyridyl)NHC(=O)O]-2-thienyl

5-[(4-CN-2-pyridyl)NHC(=O)O]-3-thienyl

4-[(3-PhO-2-pyridyl)NHC(=O)O]-2-thienyl

4-[3-(3-F-PhO)-2-pyridyl-NHC(=O)O]-2-thienyl

5-[4-(2-$NO_2$-PhO)-3-pyridyl-NHC(=O)O]-3-thienyl

4-[2-(4-Me-PhO)-4-pyridyl-NHC(=O)O]-2-thienyl

5-[4-(4-Bu-PhO)-3-pyridyl-NHC(=O)O]-3-thienyl

4-[4-(3-$CF_3$-PhO)-2-pyridyl-NHC(=O)O]-2-thienyl

5-[4-(3-MeS-PhO)-3-pyridyl-NHC(=O)O]-3-thienyl

4-[3-(4-MeO-PhO)-2-pyridyl-NHC(=O)O]-2-thienyl

5-[3-(3-BuO-PhO)-2-pyridyl-NHC(=O)O]-3-thienyl

5-[(2-F-4-MeO-3-pyridyl)NHC(=O)O]-3-thienyl

4-[(2-$CF_3$-4-Et-3-pyridyl)NHC(=O)O]-2-thienyl

$R^2$

4-[MeO-C(=O)]-2-thienyl

4-[octyloxy-C(=O)]-2-thienyl

5-[PhO-C(=O)]-3-thienyl

5-[3-Br-PhO-C(=O)]-3-thienyl

4-[2-F-PhO-C(=O)]-2-thienyl

4-[3-Cl-PhO-C(=O)]-2-thienyl

5-[2,4-diF-PhO-C(=O)]-3-thienyl

5-[4-$NO_2$-PhO-(=O)]-3-thienyl

4-[3-Me-PhO-C(=O)]-2-thienyl

4-[3-Bu-PhO-C(=O)]-2-thienyl

5-[4-$CF_3$-PhO-C(=O)]-3-thienyl

5-[2-MeS-PhO-C(=O)]-3-thienyl

4-[4-MeO-PhO-C(=O)]-2-thienyl

4-[4-BuO-PhO-C(=O)]-2-thienyl

5-[4-CN-PhO-C(=O)]-3-thienyl

5-[3-PhO-PhO-C(=O)]-3-thienyl

4-[3-(3-F-PhO)PhO-C(=O)]-2-thienyl

5-[4-(2-$NO_2$-PhO)PhO-C(=O)]-3-thienyl

4-[2-(4-Me-PhO)PhO-C(=O)]-2-thienyl

5-[4-(4-Bu-PhO)PhO-C(=O)]-3-thienyl

4-[4-(3-$CF_3$-PhO)PhO-C(=O)]-2-thienyl

5-[4-(3-MeS-PhO)PhO-C(=O)]-3-thienyl

4-[3-(4-MeO-PhO)PhO-C(=O)]-2-thienyl

5-[3-(3-BuO-PhO)PhO-C(=O)]-3-thienyl

4-[(2-F-4-MeO-PhO-C(=O)]-2-thienyl

5-[(2-$CF_3$-4-Et-PhO-C(=O)]-3-thienyl

$R^2$

5-[(3-pyridyloxy)C(=O)]-3-thienyl

4-[(3-Br-2-pyridyloxy)C(=O)]-2-thienyl

5-[(2-F-3-pyridyloxy)C(=O)]-3-thienyl

5-[(3-Cl-4-pyridyloxy)C(=O)]-3-thienyl

4-[(2,4-diF-3-pyridyloxy)C(=O)]-2-thienyl

4-[(4-$NO_2$-2-pyridyloxy)C(=O)]-2-thienyl

5-[(3-Me-2-pyridyloxy)C(=O)]-3-thienyl

5-[(3-Bu-2-pyridyloxy)C(=O)]-3-thienyl

5-[(4-$CF_3$-3-pyridyloxy)C(=O)]-3-thienyl

5-[(2-MeS-4-pyridyloxy)C(=O)]-3-thienyl

5-[(4-MeO-3-pyridyloxy)C(=O)]-3-thienyl

4-[(4-BuO-3-pyridyloxy)C(=O)]-2-thienyl

4-[(4-CN-2-pyridyloxy)C(=O)]-2-thienyl

4-[(3-PhO-2-pyridyloxy)C(=O)]-2-thienyl

5-[3-(3-F-PhO)-2-pyridyloxy-C(=O)]-3-thienyl

5-[4-(2-$NO_2$-PhO)-3-pyridyloxy-C(=O)]-3-thienyl

5-[2-(4-Me-PhO)-4-pyridyloxy-C(=O)]-3-thienyl

5-[4-(4-Bu-PhO)-3-pyridyloxy-C(=O)]-3-thienyl

4-[4-(3-$CF_3$-PhO)-2-pyridyloxy-C(=O)]-2-thienyl

4-[4-(3-MeS-PhO)-3-pyridyloxy-C(=O)]-2-thienyl

4-[3-(4-MeO-PhO)-2-pyridyloxy-C(=O)]-2-thienylPh

5-[3-(3-BuO-PhO)-2-pyridyloxy-C(=O)]-3-thienyl

4-[(2-F-4-MeO-3-pyridyloxy)C(=O)]-2-thienyl

4-[(2-$CF_3$-4-Et-3-pyridyloxy)C(=O)]-2-thienyl

4-[MeC(=O)O]-2-thienyl

4-[octyl-C(=O)O]-2-thienyl

$B^2$

5-[PhC(=O)O]-3-thienyl

4-[3-Br-PhC(=O)O]-2-thienyl

5-[2-F-PhC(=O)O]-3-thienyl

5-[3-Cl-PhC(=O)O]-3-thienyl

4-[2,4-diF-PhC(=O)O]-2-thienyl

5-[4-$NO_2$-Ph(=O)O]-3-thienyl

4-[3-Me-PhC(=O)O]-2-thienyl

5-[3-Bu-PhC(=O)O]-3-thienyl

4-[4-$CF_3$-PhC(=O)O]-2-thienyl

4-[2-MeS-PhC(=O)O]-2-thienyl

5-[4-MeO-PhC(=O)O]-3-thienyl

5-[4-BuO-PhC(=O)O]-3-thienyl

4-[4-CN-PhC(=O)O]-2-thienyl

4-[3-PhO-PhC(=O)O]-2-thienyl

5-[3-(3-F-PhO)PhC(=O)O]-3-thienyl

4-[4-(2-$NO_2$-PhO)PhC(=O)O]-2-thienyl

5-[2-(4-Me-PhO)PhC(=O)O]-3-thienyl

4-[4-(4-Bu-PhO)PhC(=O)O]-2-thienyl

5-[4-(3-$CF_3$-PhO)PhC(=O)O]-3-thienyl

4-[4-(3-MeS-PhO)PhC(=O)O]-2-thienyl

5-[3-(4-MeO-PhO)PhC(=O)O]-3-thienyl

5-[3-(3-BuO-PhO)PhC(=O)O]-3-thienyl

4-[(2-F-4-MeO-Ph)C(=O)O]-2-thienyl

5-[(2-$CF_3$-4-Et-Ph)C(=O)O]-3-thienyl

5-[(3-pyridyl)C(=O)O]-3-thienyl

4-[(3-Br-2-pyridyl)C(=O)O]-2-thienyl

$R^2$

4-[(2-F-3-pyridyl)C(=O)O]-2-thienyl

5-[(3-Cl-4-pyridyl)C(=O)O]-3-thienyl

5-[(2,4-diF-3-pyridyl)C(=O)O]-3-thienyl

5-[(4-$NO_2$-2-pyridyl)C(=O)O]-3-thienyl

5-[(3-Me-2-pyridyl)C(=O)O]-3-thienyl

5-[(3-Bu-2-pyridyl)C(=O)O]-3-thienyl

4-[(4-$CF_3$-3-pyridyl)C(=O)O]-2-thienyl

5-[(2-MeS-4-pyridyl)C(=O)O]-3-thienyl

5-[(4-MeO-3-pyridyl)C(=O)O]-3-thienyl

4-[(4-BuO-3-pyridyl)C(=O)O]-2-thienyl

4-[(4-CN-2-pyridyl)C(=O)O]-2-thienyl

5-[(3-PhO-2-pyridyl)C(=O)O]-3-thienyl

5-[3-(3-F-PhO)-2-pyridyl-C(=O)O]-3-thienyl

5-[4-(2-$NO_2$-PhO)-3-pyridyl-C(=O)O]-3-thienyl

4-[2-(4-Me-PhO)-4-pyridyl-C(=O)O]-2-thienyl

5-[4-(4-Bu-PhO)-3-pyridyl-C(=O)O]-3-thienyl

5-[4-(3-$CF_3$-PhO)-2-pyridyl-C(=O)O]-3-thienyl

5-[4-(3-MeS-PhO)-3-pyridyl-C(=O)O]-3-thienyl

4-[3-(4-MeO-PhO)-2-pyridyl-C(=O)O]-2-thienyl

4-[3-(3-BuO-PhO)-2-pyridyl-C(=O)O]-2-thienyl

5-[(2-F-4-MeO-3-pyridyl)C(=O)O]-3-thienyl

4-[(2-$CF_3$-4-Et-3-pyridyl)C(=O)O]-2-thienyl

3-F-4-$PhCH_2CH_2$-2-thienyl

3-F-4-(2-F-Ph)$OCH_2$-2-thienyl

4-F-5-(4-MeO-Ph)$CH_2$-3-thienyl

2-F-5-PhS-3-thienyl

| $R^2$ | $R^2$ |
|---|---|
| 5-MeS-4-PhO-2-thienyl | 6-pentyloxy-3-pyridyl |
| 2-F-5-PhCH$_2$O-3-thienyl | 6-CF$_3$O-3-pyridyl |
| 2-F-5-[octyl-NHC(=O)O]-3-thienyl | 6-cyclohexyloxy-3-pyridyl |
| 4-F-3-pyridyl | 6-(BuOCH$_2$)-3-pyridyl |
| 4-Cl-3-pyridyl | 5-MeOCH$_2$-2-pyridyl |
| 3-F-2-pyridyl | 6-(2-propenyl)-3-pyridyl |
| 4-Br-3-pyridyl | 5-(3-pentenyl)-2-pyridyl |
| 2,3-diF-4-pyridyl | 4-(Cl$_2$C=CHCH$_2$)-2-pyridyl |
| 2-pyridyl | 4-(2-propenyloxy)-2-pyridyl |
| 3-pyridyl | 6-(2-hexenyloxy)-3-pyridyl |
| 4-pyridyl | 6-(propargyl)-3-pyridyl |
| 2,5-diF-3-pyridyl | 2-(2-butynyloxy)-4-pyridyl |
| 3,5-diF-4-pyridyl | 4-BuSO$_2$-2-pyridyl |
| 2,6-diF-3-pyridyl | 6-PhO-3-pyridyl |
| 3,4-diF-2-pyridyl | 6-(4-Cl-PhO)-3-pyridyl |
| 5-Cl-2-F-3-pyridyl | 6-(3-Cl-PhO)-3-pyridyl |
| 2-F-5-Me-3-pyridyl | 5-(2,4-diF-PhO)-2-pyridyl |
| 2-F-4-pyridyl | 5-(4-NO$_2$-PhO)-3-pyridyl |
| 4-Me-2-pyridyl | 2-(4-CN-PhO)-4-pyridyl |
| 4-NO$_2$-2-pyridyl | 5-(4-Me-PhO)-2-pyridyl |
| 5-hexyl-3-pyridyl | 6-(3-hexyl-PhO)-3-pyridyl |
| 5-(cyclohexyl)-3-pyridyl | 6-(3-cyclohexyl-PhO)-3-pyridyl |
| 5-CF$_3$-3-pyridyl | 4-(3-CF$_3$-PhO)-2-pyridyl |
| 2-MeS-4-pyridyl | 4-(4-MeS-PhO)-2-pyridyl |
| 4-hexylthio-2-pyridyl | 5-(4-MeSO$_2$-PhO)-2-pyridyl |
| 5-MeO-4-pyridyl | 6-(3-BuSO$_2$-PhO)-3-pyridyl |

$R^2$

6-(3-MeO-PhO)-3-pyridyl
6-(3-hexyloxy-PhO)-3-pyridyl
6-(4-$CF_3$O-PhO)-3-pyridyl
5-(4-cyclohexyloxy-PhO)-2-pyridyl
5-(4-MeOC(=O)-PhO)-2-pyridyl
5-(4-allyl-PhO)-3-pyridyl
6-(4-propargyl-PhO)-3-pyridyl
6-((3-PhO)PhO)-3-pyridyl
2-(3-(2-Cl-PhO)PhO)-4-pyridyl
4-(3-(4-Me-PhO)PhO)-2-pyridyl
5-(3-(3-MeO-PhO)PhO)-2-pyridyl
6-PhCH=CH-3-pyridyl
6-(3-Br-Ph)CH=CH-3-pyridyl
5-(2-F-Ph)CH=CH-2-pyridyl
2-(3-Cl-Ph)CH=CH-4-pyridyl
5-(2,4-diF-Ph)CH=CH-3-pyridyl
6-(4-$NO_2$-Ph)CH=CH-3-pyridyl
4-(3-Me-Ph)CH=CH-2-pyridyl
5-(3-Bu-Ph)CH=CH-2-pyridyl
6-(4-$CF_3$-Ph)CH=CH-3-pyridyl
6-(2-MeS-Ph)CH=CH-3-pyridyl
5-(4-MeO-Ph)CH=CH-2-pyridyl
6-(4-BuO-Ph)CH=CH-3-pyridyl
6-Ph$CH_2CH_2$-3-pyridyl
6-(3-Br-Ph)$CH_2CH_2$-3-pyridyl
5-(2-F-Ph)$CH_2CH_2$-2-pyridyl

$R^2$

2-(3-Cl-Ph)$CH_2CH_2$-4-pyridyl
5-(2,4-diF-Ph)$CH_2CH_2$-3-pyridyl
6-(4-$NO_2$-Ph)$CH_2CH_2$-3-pyridyl
4-(3-Me-Ph)$CH_2CH_2$-2-pyridyl
5-(3-Bu-Ph)$CH_2CH_2$-2-pyridyl
6-(4-$CF_3$-Ph)$CH_2CH_2$-3-pyridyl
6-(2-MeS-Ph)$CH_2CH_2$-3-pyridyl
5-(4-MeO-Ph)$CH_2CH_2$-2-pyridyl
6-(4-BuO-Ph)$CH_2CH_2$-3-pyridyl
6-PhO$CH_2$-3-pyridyl
6-(3-Br-Ph)O$CH_2$-3-pyridyl
5-(2-F-Ph)O$CH_2$-2-pyridyl
2-(3-Cl-Ph)O$CH_2$-4-pyridyl
5-(2,4-diF-Ph)O$CH_2$-3-pyridyl
6-(4-$NO_2$-Ph)O$CH_2$-3-pyridyl
4-(3-Me-Ph)O$CH_2$-2-pyridyl
5-(3-Bu-Ph)O$CH_2$-2-pyridyl
6-(4-$CF_3$-Ph)O$CH_2$-3-pyridyl
6-(2-MeS-Ph)O$CH_2$-3-pyridyl
5-(4-MeO-Ph)O$CH_2$-2-pyridyl
6-(4-BuO-Ph)O$CH_2$-3-pyridyl
6-Ph$CH_2$-3-pyridyl
6-(3-Br-Ph)$CH_2$-3-pyridyl
5-(2-F-Ph)$CH_2$-2-pyridyl
2-(3-Cl-Ph)$CH_2$-4-pyridyl
5-(2,4-diF-Ph)$CH_2$-3-pyridyl
6-(4-$NO_2$-Ph)$CH_2$-3-pyridyl

$R^2$

4-(3-Me-Ph)$CH_2$-2-pyridyl

5-(3-Bu-Ph)$CH_2$-2-pyridyl

6-(4-$CF_3$-Ph)$CH_2$-3-pyridyl

6-(2-MeS-Ph)$CH_2$-3-pyridyl

5-(4-MeO-Ph)$CH_2$-2-pyridyl

6-(4-BuO-Ph)$CH_2$-3-pyridyl

6-PhS-3-pyridyl

6-(3-Br-Ph)S-3-pyridyl

5-(2-F-Ph)S-2-pyridyl

2-(3-Cl-Ph)S-4-pyridyl

5-(2,4-diF-Ph)S-3-pyridyl

6-(4-$NO_2$-Ph)S-3-pyridyl

4-(3-Me-Ph)S-2-pyridyl

5-(3-Bu-Ph)S-2-pyridyl

6-(4-$CF_3$-Ph)S-3-pyridyl

6-(2-MeS-Ph)S-3-pyridyl

5-(4-MeO-Ph)S-2-pyridyl

6-(4-BuO-Ph)S-3-pyridyl

6-Ph-3-pyridyl

6-(3-Br-Ph)-3-pyridyl

5-(2-F-Ph)-2-pyridyl

2-(3-Cl-Ph)-4-pyridyl

5-(2,4-diF-Ph)-3-pyridyl

6-(4-$NO_2$-Ph)-3-pyridyl

4-(3-Me-Ph)-2-pyridyl

5-(3-Bu-Ph)-2-pyridyl

$B^2$

6-(4-$CF_3$-Ph)-3-pyridyl

6-(2-MeS-Ph)-3-pyridyl

5-(4-MeO-Ph)-2-pyridyl

6-(4-BuO-Ph)-3-pyridyl

6-Ph$CH_2$O-3-pyridyl

5-(3-Br-Ph)$CH_2$O-2-pyridyl

6-(2-F-Ph)$CH_2$O-3-pyridyl

4-(3-Cl-Ph)$CH_2$O-2-pyridyl

2-(2,4-diF-Ph)$CH_2$O-4-pyridyl

6-(4-$NO_2$-Ph)$CH_2$O-3-pyridyl

6-(3-Me-Ph)$CH_2$O-3-pyridyl

5-(3-Bu-Ph)$CH_2$O-2-pyridyl

2-(4-$CF_3$-Ph)$CH_2$O-4-pyridyl

5-(2-MeS-Ph)$CH_2$O-3-pyridyl

5-(4-MeO-Ph)$CH_2$O-2-pyridyl

6-(4-BuO-Ph)$CH_2$O-3-pyridyl

5-(4-CN-Ph)$CH_2$O-2-pyridyl

6-(3-PhO-Ph)$CH_2$O-3-pyridyl

6-[3-(3-F-PhO)Ph]$CH_2$O-3-pyridyl

6-[4-(2-$NO_2$-PhO)Ph]$CH_2$O-3-pyridyl

5-[3-(4-Me-PhO)Ph]$CH_2$O-2-pyridyl

5-[4-(4-Bu-PhO)Ph]$CH_2$O-2-pyridyl

6-[3-(3-$CF_3$-PhO)Ph]$CH_2$O-3-pyridyl

6-[2-(3-MeS-PhO)Ph]$CH_2$O-3-pyridyl

6-[4-(4-MeO-PhO)Ph]$CH_2$O-3-pyridyl

5-[3-(3-BuO-PhO)Ph]$CH_2$O-2-pyridyl

| $B^2$ |
|---|
| 6-(2-F-4-MeO-Ph)$CH_2$O-3-pyridyl |
| 6-(2-$CF_3$-4-Et-Ph)$CH_2$O-3-pyridyl |
| 6-[(3-pyridyl)NHC(=O)O]-3-pyridyl |
| 5-[(3-Br-2-pyridyl)NHC(=O)O]-2-pyridyl |
| 6-[(2-F-3-pyridyl)NHC(=O)O]-3-pyridyl |
| 4-[(3-Cl-4-pyridyl)NHC(=O)O]-2-pyridyl |
| 2-[(2,4-diF-3-pyridyl)NHC(=O)O]-4-pyridyl |
| 6-[(4-$NO_2$-2-pyridyl)NHC(=O)O]-3-pyridyl |
| 6-[(3-Me-2-pyridyl)NHC(=O)O]-3-pyridyl |
| 5-[(3-Bu-2-pyridyl)NHC(=O)O]-2-pyridyl |
| 2-[(4-$CF_3$-3-pyridyl)NHC(=O)O]-4-pyridyl |
| 5-[(2-MeS-4-pyridyl)NHC(=O)O]-3-pyridyl |
| 5-[(4-MeO-3-pyridyl)NHC(=O)O]-2-pyridyl |
| 2-[(4-BuO-3-pyridyl)NHC(=O)O]-4-pyridyl |
| 5-[(4-CN-2-pyridyl)NHC(=O)O]-3-pyridyl |
| 5-[(3-PhO-2-pyridyl)NHC(=O)O]-2-pyridyl |
| 6-[3-(3-F-PhO)-2-pyridyl-NHC(=O)O]-3-pyridyl |
| 5-[4-(2-$NO_2$-PhO)-3-pyridyl-NHC(=O)O]-2-pyridyl |
| 6-[2-(4-Me-PhO)-4-pyridyl-NHC(=O)O]-3-pyridyl |
| 6-[4-(4-Bu-PhO)-3-pyridyl-NHC(=O)O]-3-pyridyl |
| 6-[4-(3-$CF_3$-PhO)-2-pyridyl-NHC(=O)O]-3-pyridyl |
| 5-[4-(3-MeS-PhO)-3-pyridyl-NHC(=O)O]-2-pyridyl |
| 5-[3-(4-MeO-PhO)-2-pyridyl-NHC(=O)O]-2-pyridyl |
| 6-[3-(3-BuO-PhO)-2-pyridyl-NHC(=O)O]-3-pyridyl |
| 6-[(2-F-4-MeO-3-pyridyl)NHC(=O)O]-3-pyridyl |
| 6-[(2-$CF_3$-4-Et-3-pyridyl)NHC(=O)O]-3-pyridyl |

$R^2$

6-[(3-pyridyloxy)C(=O)]-3-pyridyl

5-[(3-Br-2-pyridyloxy)C(=O)]-2-pyridyl

6-[(2-F-3-pyridyloxy)C(=O)]-3-pyridyl

4-[(3-Cl-4-pyridyloxy)C(=O)]-2-pyridyl

2-[(2,4-diF-3-pyridyloxy)C(=O)]-4-pyridyl

6-[(4-$NO_2$-2-pyridyloxy)C(=O)]-3-pyridyl

6-[(3-Me-2-pyridyloxy)C(=O)]-3-pyridyl

5-[(3-Bu-2-pyridyloxy)C(=O)]-2-pyridyl

2-[(4-$CF_3$-3-pyridyloxy)C(=O)]-4-pyridyl

5-[(2-MeS-4-pyridyloxy)C(=O)]-3-pyridyl

5-[(4-MeO-3-pyridyloxy)C(=O)]-2-pyridyl

4-[(4-BuO-3-pyridyloxy)C(=O)]-3-pyridyl

2-[(4-CN-2-pyridyloxy)C(=O)]-4-pyridyl

5-[(3-PhO-2-pyridyloxy)C(=O)]-2-pyridyl

5-[3-(3-F-PhO)-2-pyridyloxy-C(=O)]-3-pyridyl

6-[4-(2-$NO_2$-PhO)-3-pyridyloxy-C(=O)]-3-pyridyl

5-[2-(4-Me-PhO)-4-pyridyloxy-C(=O)]-2-pyridyl

6-[4-(4-Bu-PhO)-3-pyridyloxy-C(=O)]-3-pyridyl

6-[4-(3-$CF_3$-PhO)-2-pyridyloxy-C(=O)]-3-pyridyl

6-[4-(3-MeS-PhO)-3-pyridyloxy-C(=O)]-3-pyridyl

5-[3-(4-MeO-PhO)-2-pyridyloxy-C(=O)]-2-pyridyl

5-[3-(3-BuO-PhO)-2-pyridyloxy-C(=O)]-2-pyridyl

6-[(2-F-4-MeO-3-pyridyloxy)C(=O)]-3-pyridyl

6-[(2-$CF_3$-4-Et-3-pyridyloxy)C(=O)]-3-pyridyl

6-[(3-pyridyl)C(=O)O]-3-pyridyl

5-[(3-Br-2-pyridyl)C(=O)O]-2-pyridyl

$R^2$

6-[(2-F-3-pyridyl)C(=O)O]-3-pyridyl

4-[(3-Cl-4-pyridyl)C(=O)O]-2-pyridyl

2-[(2,4-diF-3-pyridyl)C(=O)O]-4-pyridyl

6-[(4-$NO_2$-2-pyridyl)C(=O)O]-3-pyridyl

6-[(3-Me-2-pyridyl)C(=O)O]-3-pyridyl

5-[(3-Bu-2-pyridyl)C(=O)O]-2-pyridyl

2-[(4-$CF_3$-3-pyridyl)C(=O)O]-4-pyridyl

5-[(2-MeS-4-pyridyl)C(=O)O]-3-pyridyl

5-[(4-MeO-3-pyridyl)C(=O)O]-2-pyridyl

2-[(4-BuO-3-pyridyl)C(=O)O]-4-pyridyl

5-[(4-CN-2-pyridyl)C(=O)O]-3-pyridyl

5-[(3-PhO-2-pyridyl)C(=O)O]-2-pyridyl

6-[3-(3-F-PhO)-2-pyridyl-C(=O)O]-3-pyridyl

5-[4-(2-$NO_2$-PhO)-3-pyridyl-C(=O)O]-2-pyridyl

6-[2-(4-Me-PhO)-4-pyridyl-C(=O)O]-3-pyridyl

6-[4-(4-Bu-PhO)-3-pyridyl-C(=O)O]-3-pyridyl

6-[4-(3-$CF_3$-PhO)-2-pyridyl-C(=O)O]-3-pyridyl

5-[4-(3-MeS-PhO)-3-pyridyl-C(=O)O]-2-pyridyl

5-[3-(4-MeO-PhO)-2-pyridyl-C(=O)O]-2-pyridyl

6-[3-(3-BuO-PhO)-2-pyridyl-C(=O)O]-3-pyridyl

6-[(2-F-4-MeO-3-pyridyl)C(=O)O]-3-pyridyl

6-[(2-$CF_3$-4-Et-3-pyridyl)C(=O)O]-3-pyridyl

5-[MeNHC(=O)O]-2-pyridyl

2-F-6-[octyl-NHC(=O)O]-3-pyridyl

5-[PhNHC(=O)O]-3-pyridyl

2-[(3-Br-Ph)NHC(=O)O]-4-pyridyl

$B^2$

5-[(2-F-Ph)NHC(=O)O]-3-pyridyl

2-F-6-[(3-Cl-Ph)NHC(=O)O]-3-pyridyl

6-[(2,4-diF-Ph)NHC(=O)O]-3-pyridyl

3-F-2-[(4-$NO_2$-Ph)NHC(=O)O]-4-pyridyl

6-[(3-Me-Ph)NHC(=O)O]-3-pyridyl

5-[(3-Bu-Ph)NHC(=O)O]-2-pyridyl

6-[(4-$CF_3$-Ph)NHC(=O)O]-3-pyridyl

2-F-6-[(2-MeS-Ph)NHC(=O)O]-3-pyridyl

5-[(4-MeO-Ph)NHC(=O)O]-2-pyridyl

4-[(4-BuO-Ph)NHC(=O)O]-2-pyridyl

6-[(4-CN-Ph)NHC(=O)O]-3-pyridyl

2-F-6-[(3-PhO-Ph)NHC(=O)O]-3-pyridyl

2-[3-(3-F-PhO)Ph-NHC(=O)O]-4-pyridyl

5-[4-(2-$NO_2$-PhO)Ph-NHC(=O)O]-2-pyridyl

5-[2-(4-Me-PhO)Ph-NHC(=O)O]-3-pyridyl

5-[4-(4-Bu-PhO)Ph-NHC(=O)O]-2-pyridyl

6-[4-(3-$CF_3$-PhO)Ph-NHC(=O)O]-3-pyridyl

6-[4-(3-MeS-PhO)Ph-NHC(=O)O]-3-pyridyl

6-[3-(4-MeO-PhO)Ph-NHC(=O)O]-3-pyridyl

2-F-6-[3-(3-BuO-PhO)Ph-NHC(=O)O]-3-pyridyl

5-[(2-F-4-MeO-Ph)NHC(=O)O]-2-pyridyl

6-[(2-$CF_3$-4-Et-Ph)NHC(=O)O]-2-pyridyl

5-[MeOC(=O)]-2-pyridyl

2-F-6-[octyloxy-C(=O)]-3-pyridyl

5-[PhOC(=O)]-3-pyridyl

2-[(3-Br-PhO)C(=O)]-4-pyridyl

$R^2$

5-[(2-F-PhO)C(=O)]-3-pyridyl

2-F-6-[(3-Cl-PhO)C(=O)]-3-pyridyl

6-[(2,4-diF-PhO)C(=O)]-3-pyridyl

3-F-2-[(4-$NO_2$-PhO)C(=O)]-4-pyridyl

6-[(3-Me-PhO)C(=O)]-3-pyridyl

5-[(3-Bu-PhO)C(=O)]-2-pyridyl

6-[(4-$CF_3$-PhO)C(=O)]-3-pyridyl

2-F-6-[(2-MeS-PhO)C(=O)]-3-pyridyl

5-[(4-MeO-PhO)C(=O)]-2-pyridyl

4-[(4-BuO-PhO)C(=O)]-2-pyridyl

6-[(4-CN-PhO)C(=O)]-3-pyridyl

2-F-6-[(3-PhO-PhO)C(=O)]-3-pyridyl

2-[3-(3-F-PhO)PhO-C(=O)]-4-pyridyl

5-[4-(2-$NO_2$-PhO)PhO-C(=O)]-2-pyridyl

5-[2-(4-Me-PhO)PhO-C(=O)]-3-pyridyl

5-[4-(4-Bu-PhO)PhO-C(=O)]-2-pyridyl

6-[4-(3-$CF_3$-PhO)PhO-C(=O)]-3-pyridyl

6-[4-(3-MeS-PhO)PhO-C(=O)]-3-pyridyl

6-[3-(4-MeO-PhO)PhO-C(=O)]-3-pyridyl

2-F-6-[3-(3-BuO-PhO)PhO-C(=O)]-3-pyridyl

5-[(2-F-4-MeO-PhO)C(=O)]-2-pyridyl

6-[(2-$CF_3$-4-Et-PhO)C(=O)]-2-pyridyl

5-[MeC(=O)O]-2-pyridyl

2-F-6-[octyl-C(=O)O]-3-pyridyl

5-[PhC(=O)O]-3-pyridyl

2-[(3-Br-Ph)C(=O)O]-4-pyridyl

$R^2$

5-[(2-F-Ph)C(=O)O]-3-pyridyl

2-F-6-[(3-Cl-Ph)C(=O)O]-3-pyridyl

6-[(2,4-diF-Ph)C(=O)O]-3-pyridyl

3-F-2-[(4-$NO_2$-Ph)C(=O)O]-4-pyridyl

6-[(3-Me-Ph)C(=O)O]-3-pyridyl

5-[(3-Bu-Ph)C(=O)O]-2-pyridyl

6-[(4-$CF_3$-Ph)C(=O)O]-3-pyridyl

2-F-6-[(2-MeS-Ph)C(=O)O]-3-pyridyl

5-[(4-MeO-Ph)C(=O)O]-2-pyridyl

4-[(4-BuO-Ph)C(=O)O]-2-pyridyl

6-[(4-CN-Ph)C(=O)O]-3-pyridyl

2-F-6-[(3-PhO-Ph)C(=O)O]-3-pyridyl

2-[3-(3-F-PhO)Ph-C(=O)O]-4-pyridyl

5-[4-(2-$NO_2$-PhO)Ph-C(=O)O]-2-pyridyl

5-[2-(4-Me-PhO)Ph-C(=O)O]-3-pyridyl

5-[4-(4-Bu-PhO)Ph-C(=O)O]-2-pyridyl

6-[4-(3-$CF_3$-PhO)Ph-C(=O)O]-3-pyridyl

6-[4-(3-MeS-PhO)Ph-C(=O)O]-3-pyridyl

6-[3-(4-MeO-PhO)Ph-C(=O)O]-3-pyridyl

2-F-6-[3-(3-BuO-Ph)PhO-C(=O)O]-3-pyridyl

5-[(2-F-4-MeO-Ph)C(=O)O]-2-pyridyl

6-[(2-$CF_3$-4-Et-Ph)C(=O)O]-2-pyridyl

2-furanyl

3-furanyl

2-F-3-furanyl

5-Cl-3-furanyl

$R^2$

5-$NO_2$-3-furanyl

3,4-diF-2-furanyl

4-Me-2-furanyl

5-Bu-3-furanyl

4-$CF_3$-2-furanyl

5-MeS-2-furanyl

5-MeO-3-furanyl

4-BuO-2-furanyl

TABLE 21

Compounds of Formula If wherein A = NH and:

| n | $R^{43}$ | n | $R^{43}$ |
|---|---|---|---|
| 5 | Me | 6 | 4-F-PhO |
| 6 | Ph | 8 | 4-Et-PhO |
| 8 | 4-$CF_3$-Ph | 2 | 4-EtO-PhO |
| 9 | 3-$F_3$CO-Ph | 1 | 4-(4-F-PhO)PhO |
| 5 | 2,4-diCl-Ph | 2 | 4-(3-Et-PhO)PhO |
| 1 | 4-F-Ph | 3 | 4-(3-Bu-PhO)PhO |
| 2 | 4-Et-Ph | 3 | MeNH |
| 3 | 3-EtO-Ph | 4 | $Me_2N$ |
| 4 | 4-(4-F-PhO)Ph | 1 | PhNH |
| 3 | 4-(3-Et-PhO)Ph | 5 | PhN(Me) |
| 1 | 4-(3-BuO-PhO)Ph | 6 | 4-$CF_3$-PhNH |
| 5 | 3-(4-$CF_3$-PhO)Ph | 2 | 3-$F_3$CO-PhNH |
| 8 | MeO | 3 | 2,4-diCl-PhNH |
| 6 | PhO | 8 | 4-Et-PhNH |
| 2 | 4-$F_3$CO-PhO | 4 | 4-(4-F-PhO)PhNH |
| 4 | 3,5-diCl-PhO | 3 | 4-(4-$CF_3$-PhO)PhNH |

TABLE 22

Compounds of Formula Ig wherein A = NH and:

| n | T | M | n | T | M |
|---|---|---|---|---|---|
| 1 | O | H | 6 | O | 4-F-Ph |
| 4 | O | Et | 7 | O | 3,5-diMe-Ph |
| 2 | O | Bzl | 8 | O | 4-pyridyl |
| 3 | O | 4-Cl-Bzl | 1 | O | 2,6-diEt-4-pyridyl |
| 5 | O | Ph | 4 | O | MeC(=O) |
| 1 | O | 2,6-diF-Ph | 2 | O | PhC(=O) |

| n | T | W | n | T | W |
|---|---|---|---|---|---|
| 1 | O | 3,5-diF-PhC(=O) | 3 | NH | 3,5-diF-Ph |
| 3 | O | 3,5-MeO-PhC(=O) | 2 | NH | 4-pyridyl |
| 6 | O | 4-pyridyl-C(=O) | 1 | NH | EtC(=O) |
| 7 | O | 3,5-diCl-4-pyridyl-C(=O) | 4 | NH | PhC(=O) |
| 4 | O | BuOC(=O) | 5 | NH | (4-pyridyl)C(=O) |
| 2 | O | PhOC(=O) | 6 | NH | MeOC(=O) |
| 5 | O | (3,5-diF-Ph)OC(=O) | 3 | NH | PhOC(=O) |
| 3 | O | (4-EtO-Ph)OC(=O) | 1 | NH | (3-pyridyl)OC(=O) |
| 6 | O | (4-pyridyl)OC(=O) | 1 | NH | MeNHC(=O) |
| 2 | O | NHMeC(=O) | 2 | N-Me | Me |
| 5 | O | NHPhC(=O) | 3 | N-Me | Ph |
| 1 | O | NHpyridylC(=O) | 4 | N-Ph | Ph |
| 4 | NH | Me | 5 | --- | H |
| 1 | NH | Bzl | 1 | --- | Me |
| 8 | NH | Ph | 2 | --- | Ph |

TABLE 23

Compounds of Formula Ih wherein A = NH and:

| n | m | $R^{35}$ | n | m | $R^{35}$ |
|---|---|---|---|---|---|
| 2 | 1 | MeS | 3 | 1 | MeC(=O)O |
| 3 | 1 | $FCH_2CH_2O$ | 2 | 2 | PhOC(=O)O |
| 1 | 1 | *c*-hexyloxy | 1 | 3 | MeNHC(=O)O |
| 1 | 1 | EtOC(=O) | 2 | 1 | PhNH |
| 1 | 2 | OH | 2 | 3 | $(MeO)_2P(=O)O$ |

| n | m | $R^{35}$ | n | m | $R^{35}$ |
|---|---|---|---|---|---|
| 1 | 2 | $MeSO_3$ | 2 | 2 | PhO |
| 3 | 2 | 4-pyridyl | 2 | 6 | H |
| 1 | 3 | 4-pyridyloxy | 2 | 8 | H |
| 2 | 2 | 2-pyrimidyl | 1 | 7 | EtS |
| 2 | 1 | c-hexyl | 3 | 5 | $CF_3O$ |
| 1 | 2 | c-pentyl | 3 | 5 | c-hexyl |
| 1 | 2 | EtO | 1 | 8 | Ph |
| 1 | 1 | Ph | | | |

Formulations

Useful formulations of the compounds of Formula I can be prepared in conventional forms such as dusts, granules, pellets, solutions, suspensions, emulsions, wettable powders, emulsifiable concentrates and the like. Many of these can be applied directly. Sprayable formulations can be extended in suitable media and used at spray volumes of from a few liters to several hundred liters per hectare. High-strength composition are primarily used as intermediates for further formulation. The formulations, can contain from 0.1% to 99% by weight of active compound(s) and at least one of (a) 0.1% to 20% surfactant(s) and (b) 1% to 99.9% solid or liquid diluent(s). More specifically, they will contain these ingredients in the following approximate proportions:

| Formulation | Weight Percent* | | |
|---|---|---|---|
| | Compound | Diluent(s) | Surfactant(s) |
| Wettable Powders | 20-90 | 0-74 | 1-10 |
| Oil Suspensions, Emulsions, Solutions, (including Emulsifiable Concentrates) | 3-50 | 40-95 | 0-15 |
| Aqueous Suspension | 10-50 | 40-84 | 1-20 |
| Dusts | 1-25 | 70-99 | 0-5 |
| Granules and Pellets | 0.1-95 | 5-99.9 | 0-15 |
| High-Strength Compositions | 90-99 | 0-10 | 0-2 |

*Active compound plus at least one of a surfactant or a diluent equals 100 weight percent.

Lower or higher levels of active compound can, of course, be present depending on the intended use and the physical properties of the compound. Higher ratios of surfactant to active compound are sometimes desirable, and are achieved by incorporation into the formulation or by tank mixing.

Typical solid diluents are described in Watkins, et al., "Handbook of Insecticide Dust Diluents and Carriers", 2nd Ed., Dorland Books, Caldwell, New Jersey, but other solids, either mined or manufactured, can be used. The more absorptive diluents are preferred for wettable powders and the denser ones for dusts. Typical liquid diluents and solvents are described in Marsden, "Solvents Guide," 2nd Ed., Interscience, New York, 1950. Solubility under 0.1% is preferred for suspension concentrates; solution concentrates are preferably stable against phase separation at 0°C. "McCutcheon's Detergents and Emulsifiers Annual", MC Publishing Corp., Ridgewood, New Jersey, as well as Sisely and Wood, "Encyclopedia of Surface Active Agents", Chemical Publishing Co., Inc., New York, 1964, list surfactants and recommended uses. All formulations can contain minor amounts of additives to reduce foaming, caking, corrosion, microbiological growth, etc.

The methods of making such compositions are well known. Solutions are prepared by simply mixing the ingredients. Fine solid compositions are made by blending and, usually, grinding as in a hammer or fluid

energy mill. Suspensions are prepared by wet milling (see, for example, U.S. Patent 3,060,084). Granules and pellets can be made by spraying the active material upon preformed granular carriers or by agglomeration techniques. See J. E. Browning, "Agglomeration", *Chemical Engineering*, December 4, 1967, pp 147ff. and "Perry's Chemical Engineer's Handbook", 5th Ed., McGraw-Hill, New York, 1973, pp. 8-57ff.

For further information regarding the art of formulation, see for example:

U.S. Patent 3,235,361;

U.S. Patent 3,309,192;

U.S. Patent 2,891,855;

G. C. Klingman, "Weed Control as a Science", John Wiley and Sons, Inc., New York, 1961, pp 81-96; and

J. D. Fryer et al., "Weed Control Handbook", 5th Ed., Blackwell Scientific Publications, Oxford, 1968, pp 101-103.

In the following examples of formulations of the compounds of Formula I, all parts are by weight unless otherwise indicated.

Example A

| Wettable Powder | |
|---|---|
| 5-(2,4-diF-Ph)-5-Me-3-(phenylamino)-4-thioxooxazolidin-2-one | 80% |
| sodium alkylnaphthalenesulfonate | 2% |
| sodium ligninsulfonate | 2% |
| synthetic amorphous silica | 3% |
| kaolinite | 13% |

The ingredients are blended, hammer-milled until all the solids are essentially under 50 microns, reblended, and packaged.

Example B

| Wettable Powder | |
|---|---|
| 5-(2,4-diF-Ph)-5-Me-3-(phenylamino)-4-thioxooxazolidin-2-one | 50% |
| sodium alkylnaphthalenesulfonate | 2% |
| low viscosity Me cellulose | 2% |
| diatomaceous earth | 46% |

The ingredients are blended, coarsely hammer-milled and then air-milled to produce particles essentially all below 10 microns in diameter. The product is reblended before packaging.

Example C

| Granule | |
|---|---|
| Wettable Powder of Example A | 5% |
| attapulgite granules (U.S.S. 20-40 mesh; 0.84-0.42 mm) | 95% |

A slurry of wettable powder containing 25% solids is sprayed on the surface of attapulgite granules in a double-cone blender. The granules aredried and packaged.

Example D

| Extruded Pellet | |
|---|---|
| 5-(2,4-diF-Ph)-5-Me-3-(phenylamino)-4-thioxooxazolidin-2-one | 25% |
| anhydrous sodium sulfate | 10% |
| crude calcium ligninsulfonate | 5% |
| sodium alkylnaphthalenesulfonate | 1% |
| calcium/magnesium bentonite | 59% |

The ingredients are blended, hammer-milled and then moistened with about 12% water. The mixture is extruded as cylinders about 3 mm diameter which are cut to produce pellets about 3 mm long. These can be used directly after drying, or the dried pellets can be crushed to pass a U.S.S. No. 20 sieve (0.84 mm openings). The granules hold on s U.S.S. No. 40 sieve (0.42 mm openings) can be packaged for use and the fines recycled.

Example E

| Oil Suspension | |
|---|---|
| 5-(2,4-diF-Ph)-5-Me-3-(phenylamino)-4-thioxooxazolidin-2-one | 25% |
| polyoxyethylene sorbitol hexaoleate | 5% |
| highly aliphatic hydrocarbon oil | 70% |

The ingredients are ground together in a sand mill until the solid particles have been reduced to under about 5 microns. The resulting thick suspension can be applied directly, but preferably after being extended with oils or emulsified in water.

Example F

| Wettable Powder | |
|---|---|
| 5-(2,4-diF-Ph)-5-Me-3-(phenylamino)-4-thioxooxazolidin-2-one | 20% |
| sodium alkylnaphthalenesulfonate | 4% |
| sodium ligninsulfonate | 4% |
| low viscosity Me cellulose | 3% |
| attapulgite | 69% |

The ingredients are thoroughly blended. After grinding in a hammer-mill to produce particles essentially all below 100 microns, the material is reblended and sifted through a U.S.S. No. 50 sieve (0.3mm opening) and packaged.

Example G

| Low Strength Granule | |
|---|---|
| 5-(2,4-diF-Ph)-5-Me-3-(phenylamino)-4-thioxooxazolidin-2-one | 1% |
| N,N-dimethylformamide | 9% |
| attapulgite granules (U.S.S. 20-40 sieve) | 90% |

The active ingredient is dissolved in the solvent and the solution is sprayed upon dedusted granules in a double cone blender. After spraying of the solution has been completed, the blender is allowed to run for a short period and then the granules are packaged.

Example H

| Aqueous Suspension | |
|---|---|
| 5-(2,4-diF-Ph)-5-Me-3-(phenylamino)-4-thioxooxazolidin-2-one | 40% |
| polyacrylic acid thickener | 0.3% |
| dodecylphenol polyethylene glycol ether | 0.5% |
| disodium phosphate | 1% |
| monosodium phosphate | 0.5% |
| polyvinyl alcohol | 1.0% |
| water | 56.7% |

The ingredients are blended and ground together in a sand mill to produce particles essentially all under 5 microns in size.

Example I

| Low Strength Granule | |
|---|---|
| 5-(2,4-diF-Ph)-5-Me-3-(phenylamino)-4-thioxooxazolidin-2-one | 0.1% |
| attapulgite granules (U.S.S. 20-40 mesh) | 99.9% |

The active ingredient is dissolved in a solvent and the solution is sprayed upon dadusted granules in a double-cone blender. After spraying of the solution has been completed, the material is warmed to evaporate the solvent. The material is allowed to cool and then packaged.

Example J

| Granule | |
|---|---|
| 5-(2,4-diF-Ph)-5-Me-3-(phenylamino)-4-thioxooxazolidin-2-one | 80% |
| wetting agent | 1% |
| crude ligninsulfonate salt (containing 5-20% of the natural sugars) | 10% |
| attapulgite clay | 9% |

The ingredients are blended and milled to pass through a 100 mesh screen. This material is then added to a fluid bed granulator, the air flow is adjusted to gently fluidize the material, and a fine spray of water is sprayed onto the fluidized material. The fluidization and spraying are continued until granules of the desired size range are made. The spraying is stopped, but fluidization is continued, optionally with heat, until the water content is reduced to the desired level, generally less than 1%. The material is then discharged, screened to the desired size range, generally 14-100 mesh (1410-149 microns), and packagedfor use.

Example K

| High Strength Concentrate | |
|---|---|
| 5-(2,4-diF-Ph)-5-Me-3-(phenylamino)-4-thioxooxazolidin-2-one | 99% |
| silica aerogel | 0.5% |
| synthetic amorphous silica | 0.5% |

The ingredients are blended and ground in a hammer-mill to produce a material essentially all passing a U.S.S. No. 50 screen (0.3 mm opening). The concentrate can be formulated further if necessary.

Example L

| Wettable Powder | |
|---|---|
| 5-(2,4-diF-Ph)-5-Me-3-(phenylamino)-4-thioxooxazolidin-2-one | 90% |
| dioctyl sodium sulfosuccinate | 0.1% |
| synthetic fine silica | 9.9% |

The ingredients are blended and ground in a hammer-mill to produce particles essentially all below 100 microns. The material is sifted through a U.S.S. No. 50 screen and then packaged.

Example M

| Wettable Powder | |
|---|---|
| 5-(2,4-diF-Ph)-5-Me-3-(phenylamino)-4-thioxooxazolidin-2-one | 40% |
| sodium ligninsulfonate | 20% |
| montmorillonite clay | 40% |

The ingredients are thoroughly blended, coarsely hammer-milled and then air-milled to produce particles essentially all below 10 microns in size. The material is reblended and then packaged.

Example N

| Oil Suspension | |
|---|---|
| 5-(2,4-diF-Ph)-5-Me-3-(phenylamino)-4-thioxooxazolidin-2-one | 35% |
| blend of polyalcohol carboxylic esters and oil soluble petroleum sulfonates | 6% |
| xylene | 59% |

The ingredients are combined and ground together in a sand mill to produce particles essentially all below 5 microns. The product can be used directly, extended with oils, or emulsified in water.

Example O

| Emulsifiable Concentrate | |
|---|---|
| 5-(2,4-diF-Ph)-5-Me-3-(phenylamino)-4-thioxooxazolidin-2-one | 20% |
| chlorobenzene | 74% |
| sorbitan monostearate and polyoxyethylene condensates thereof | 6% |

The ingredients are combined and stirred to produce a solution which can be emulsified in water for application.

Example P

| Wettable Powder | |
|---|---|
| 4-imino-5-methyl-5-(4-phenoxyphenyl)-3-(phenylamino)-2-oxazolidinone hydrobromide | 50% |
| sodium alkylnaphthalenesulfonate | 2% |
| low viscosity Me cellulose | 2% |
| diatomaceous earth | 46% |

The ingredients are blended, coarsely hammer-milled and then air-milled to produce particles essentially all below 10 microns in diameter. The product is reblended before packaging.

Example O

| Emulsifiable Concentrate | |
|---|---|
| 4-imino-5-methyl-5-(4-phenoxyphenyl)-3-(phenylamino)-2-oxazolidinone hydrododecylbenzene sulfonate | 20% |
| chlorobenzene | 74% |
| sorbitan monostearate and polyoxyethylene condenates thereof | 6% |

The ingredients are combined and stirred to produce a solution which can be emulsified in water for application.

UTILITY

The compounds of this invention are useful as plant disease control agents. They provide control of diseases caused by a broad spectrum of fungal plant pathogens in the *Basidiomycete, Ascomycete* and *Oomycete* classes. They are effective in controlling a broad spectrum of plant diseases, particularly foliar pathogens of ornamental, vegetable, field, cereal, and fruit crops. These pathogens include, *Cercosporidium personatum, Cercospora arachidicola, Cercospora beticola, Pseudocercosporella herpotrichoides, Puccinia recondita, Puccinia gramminis, Hemileia vastatrix, Puccinia striiformis, Puccinia arachidis, Phytophthora infestans, Plasmopara viticola, Peronospora tabacina, Pseudoperonospora cubensis, Pythium aphanidermatum, Alternaria brassicae, Septoria nodorum,* and other species closely related to these pathorgens.

They also control seed pathogens.

The compounds of this invention can be mixed with various agents such as fungicides, bactericides, acaricides, nematicides, insecticides or other biologically active compounds in order to achieve desired results with a minimum of expenditure of time, effort and material. Suitable agents of this type are well-known to those skilled in the art. Some of these agents are listed below:

Fungicides

methyl 2-benzimidazolecarbamate (carbendazim)
tetramethylthiuram disulfide (thiuram)
*n*-dodecylguanidine acetate (dodine)
manganese ethylenebisdithiocarbamate (maneb)
1,4-dichloro-2,5-dimethoxybenzene (chloroneb)
methyl 1-(butylcarbamoyl)-2-benzimidazolecarbamate (benomyl)
2-cyano-*N*-ethylcarbamoyl-2-methoxyiminoacetamide (cymoxanil)
*N*-trichloromethylthiotetrahydrophthalamide (captan)
*N*-trichloromethylthiophthalimide (folpet)
dimethyl 4,4'-(*o*-phenylene) bis (3-thioallophanate) (thiophanate-methyl)
2-(thiazol-4-yl)benzimidazole (thiabendazole)
aluminum tris(*O*-ethylphosphonate)(phosethyl aluminum)
tetrachloroisophthalonitrile (chlorothalonil)
2,6-dichloro-4-nitroaniline (dichloran)
*N*-(2,6-dimethylphenyl)-*N*-(methoxyacetyl)alanine methyl ester (metalaxyl)
2-methoxy-*N*-(2-oxo-1,3-oxazolidin-3-yl)acet2',6'-xylidide (oxadixyl)
cis-*N*-[1,1,2,2-tetrachloroethyl)thio]cyclohex-4-ene-1,2-dicarbioximide (captafol)
3-(3,5-dichlorophenyl)-*N*-(1-methylethyl)-2,4-dioxo-1-imidazolidine carboxamide (iprodione)
3-(3,5-dichlorophenyl)-5-ethenyl-5-methyl-2,4-oxazolidinedione (vinclozolin)
kasugamycin
*O*-ethyl-*S*,*S*-diphenylphosphorodithioate (edifenphos)
4-(3-(4-(1,1-dimethylethyl)phenyl)-2-methyl)propyl-2,6-dimethylmorpholine (fenpropimorph)
4-(3-4(1,1-dimethylethyl)phenyl)-2-methyl)propylpiperidine (fenpropidine)
1-(4-chlorophenoxy)-3,3-dimethyl-1-1H-1,2,4-triazol-1-yl)butanone (triadimefon)

2-(4-chlorophenyl)-2-(1H-1,2,4-triazol-1-ylmethyl)hexanenitrile (myclobutanil)
1-[2-(4-chlorophenyl)ethyl]-1-(1,1-dimethylethyl)-1-(1H-1,2,4-triazole-1-yl)ethanol (tebuconazol)
3-chloro-4-[4-methyl-2-(1H-1,2,4-triazol)-1-ylmethyl)-1,3-dioxolan-2-yl]phenyl-4-chlorophenyl ether (difenoconazole)
1-[2-(2,4-dichlorophenyl)pentyl]1H-1,2,4-triazole (penconazole)
2,4'-difluoro-1-(1H-1,2,4-triazole-1-ylmethyl)benzhydryl alcohol (flutriafol)
1-[[[bis(4-fluorophenyl)]methylsilyl]methyl]-1H-1,2,4-triazole (flusilazole)
*N*-propyl-*N*-[2-(2,4,6-trichlorophenoxy)ethyl]imidazole-1-carboxamide (prochloraz)
1-[2-(2,4-dichlorophenyl)-4-propyl-1,3-dioxolan-2-ylmethyl]-1H-1,2,4-triazole (propiconazole)
1-(2-chlorophenyl)-1-(4-chlorophenyl)-1-(5-pyrimidin)methanol (fenarimol)
1-(4-chlorophenoxy)-3,3-dimethyl-1-(1H-1,2,4-triazole-1-yl)butan-2-ol (triadimenol)
1-(2,4-dichlorophenyl)-4,4-dimethyl-2-(1H-1,2,4-triazol-1-yl)pentan-3-ol (diclobutrazol)
copper oxychloride
methyl *N*-(2,6-dimethylphenyl)-*N*-(2-furnanylcarbonyl)-DL-alaninate (furalaxyl)

Bactericides

tribasic copper sulfate
streptomycin sulfate
oxytetracycline

Acaricides

senecioic acid, ester with 2-sec-butyl-4,6-dinitro-phenol (binapacryl)
6-methyl-1,3-dithiolo[2,3-B]quinonolin-2-one (oxythio-quinox)
2,2,2-trichloro-1,1-bis(4-chlorophenyl)ethanol (dicofol)
bis(pentachloro-2,4-cyclopentadien-1-yl)(dienochlor)
tricyclohexyltin hydroxide (cyhexatin)
hexakis(2-methyl-2-phenylpropyl)distannoxane (fenbutin oxide)

Nematicides

2-[diethoxyphosphinylimino]-1,3-diethietane (fosthietan)
*S*-methyl-1-(dimethylcarbamoyl)-*N*-(methylcarbamoyloxy)thioformimidate (oxamyl)
*S*-methyl-1-carbamoyl-*N*-(methylcarbamoyloxy)thioformimidate
*N*-isopropylphosphoramidic acid, *O*-ethyl-*O*'-[4-(methylthio)-*m*-tolyl]diester (fenamiphos)

Insecticides

3-hydroxy-*N*-methylcrotonamide(dimethylphosphate)ester (monocrotophos)
methylcarbamic acid, ester with 2,3-dihydro-2,2-dimethyl-7-benzofuranol (carbofuran)
*O*-[2,4,5-trichloro-a-(chloromethyl)benzyl]phosphoric acid, *O*',*O*'-dimethyl ester (tetrachlorvinphos)
2-mercaptosuccinic acid, diethyl ester, *S*-ester with thionophosphoric acid, dimethyl ester (malathion)
phosphorothioic acid, *O*,*O*-dimethyl, *O*-*p*-nitrophenyl ester (methyl parathion)
methylcarbamic acid, ester with α-naphthol (carbaryl)
methyl *N*-[[(methylamino)carbonyl]oxy]ethanimidothioate (methomyl)
*N*'-(4-chloro-o-tolyl)-*N*,*N*-dimethylformamidine (chlordimeform)
*O*,*O*-diethyl-*O*-(2-isopropyl-4-methyl-6-pyrimidyl)phosphorothioate (diazinon)
octachlorocamphene (toxaphene)
*O*-ethyl *O*-*p*-nitrophenyl phenylphosphonothioate (EPN)
cyano(3-phenoxyphenyl)-methyl 4-chloro-α-(1-methylethyl)benzeneacetete (fenvalerate)
(3-phenoxyphenyl)methyl 3-(2,2-dichloro-ethenyl)-2,2-dimethylcyclopropanecarboxylate (permethrin)
dimethyl *N*,*N*'-[thiobis(*N*-methylimmo)carbonyloxy]]-bis[ethanimidothioate] (thiodicarb)
phosphorothiolothionic acid, *O*-ethyl-*O*-[4-(methylthio)phenyl]-*S*-*n*-propyl ester (sulprofos)
alpha-cyano-3-phenoxybenzyl 3-(2,2-dichlorovinyl)-2,2-dimethylcyclopropanecarboxylate (cypermethrin)
cyano(3-phenoxyphenyl)methyl 4-(difluoromethoxy)-alpha-(methylethyl)benzeneacetate (flucythrinate)
*O*,*O*-diethyl-*O*-(3,5,6-trichloro-2-pyridyl)phosphorothioate (chlorpyrifos)
*O*,*O*-dimethyl-*S*-[(4-oxo-1,2,3-benzotriazin-3-(4H)-yl)methyl]phosphorodithioate (azinphos-methyl)

5,6-dimethyl-2-dimethylamino-4-pyrimidinyl dimethyl carbamate (pirimicarb)
*S*-(*N*-formyl-*N*-methylcarbamoylmethyl)-*O*,*O*-dimethyl phosphorodithioate (formothion)
*S*-2-(ethylthioethyl)-*O*,*O*-dimethyl phosphiorothioate (demeton-*S*-methyl)
(5)-α-cyano-3-phenoxybenzyl (1R,3R)-3-(2,2-dibromovinyl)-2,2-dimethylcyclopropanecarboxylate (deltamethrin)
cyano(3-phenoxyphenyl)methyl ester of *N*-(2-chloro-4-trifluoromethylphenyl)alanine (fluvalinate)

APPLICATION METHOD

Disease control is ordinarily accomplished by applying an effective amount of the compounds of the invention either pre-infection or post-infection to the portion of the plant to be protected such as the roots, stems, foliage, fruit, seeds, tubers or bulbs, or to the media (soil or sand) in which the plants to be protected are growing. The compound also may be applied to the seed, to protect the seed and seedling.

Rates of application for these compounds can be influenced by many factors of the environment and should be determined under actual use conditions. Foliage can normally be protected when treated at a rate of from less than 1 g/ha to 5000 g/ha of active ingredient. Plants growing in soil treated at a concentration from 0.1 to about 20 kg/ha can be protected from disease. Seed and seedlings can normally be protected when seed is treated at a rate of from 0.1 to 10 g per kilogram of seed. The efficacy of the compounds for disease control is evaluated according to Tests A-G below.

Test A

The test compounds are dissolved in acetone in an amount equal to 3% of the final volume and then suspended at a concentration of 200 ppm in purified water containing 250 ppm of the surfactant Trem 014 (polyhydric alcohol esters). This suspension is sprayed to the point of run-off on wheat seedlings. The following day the seedlings are inoculated with a spore dust of ***Erysiphe graminis*** f. sp. tritici, (the causal agent of wheat powdery mildew) and incubated in a growth chamber at 20 °C for 7 days, after which disease ratings are made.

Test B

The test compounds are dissolved in acetone in an amount equal to 3% of the final volume and then suspended at a concentration of 200 ppm in purified water containing 250 ppm of the surfactant Trem 014 (polyhydric alcohol esters). This suspension is sprayed to the point of run-off on wheat seedlings. The following day the seedlings are inoculated with a spore suspension of ***Puccinia recondita*** (the causal agent of wheat leaf rust) and incubated in a saturated atmosphere at 20 °C for 24 h, and then moved to a growth chamber at 20 °C for 6 days, after which disease ratings are made.

Test C

The test compounds are dissolved in acetone in an amount equal to 3% of the final volume and then suspended at a concentration of 200 ppm in purified water containing 250 ppm of the surfactant Trem 014 (polyhydric alcohol esters). This suspension is sprayed to the point of run-off on rice seedlings. The following day the seedlings are inoculated with a spore suspension of ***Pyricularis oryzae*** (the causal agent of rice blast) and incubated in a saturated atmosphere at 27 °C for 24 h, and then moved to a growth chamber at 30 °C for 5 days, after which disease ratings are made.

Test D

The test compounds are dissolved in acetone in an amount equal to 3% of the final volume and then suspended at a concentration of 200 ppm in purified water containing 250 ppm of the surfactant Trem 014 (polyhydric alcohol esters). This suspension is sprayed to the point of run-off on tomato seedlings. The following day the seedlings are inoculated with a spore suspension of ***Phytophthora infestans*** (the causal agent of potato and tomato late blight) and incubated in a saturated atmosphere at 20 °C for 24 h, and then moved to a growth chamber at 20 °C for 5 days, after which disease ratings are made.

Test E

The test compounds are dissolved in acetone in an amount equal to 3% of the final volume and then suspended at a concentration of 200 ppm in purified water containing 250 ppm of the surfactant Trem 014 (polyhydric alcohol esters). This suspension is sprayed to the point of run-off on potato seedlings. The following day the seedlings are inoculated with a spore suspension of *Phytophthora infestans* (the causal agent of potato late blight) and incubated in a high humidity atmosphere at 20° C for 24 h, and then moved to a greenhouse at 20 to 25° C for 7 days, after which disease ratings are made.

Test F

The test compounds are dissolved in acetone in an amount equal to 3% of the final volume and then suspended at a concentration of 200 ppm in purified water containing 250 ppm of the surfactant Trem 014 (polyhydric alcohol esters). This suspension is sprayed to the point of run-off on grape seedlings. The following day the seedlings are inoculated with a spore suspension of *Plasmopara viticola* (the causal agent of grape downy mildew) and incubated in a saturated atmosphere at 20° C for 24 h, moved to a growth chamber at 20° C for 6 days, and then incubated in a saturated atmosphere at 20° C for 24 h, after which disease ratings are made.

Test G

The tent compounds are dissolved in acetone in an amount equal to 3% of the final volume and then suspended at a concentration of 200 ppm in purified water containing 250 ppm of the surfactant Trem 014 (polyhydric alcohol esters). This suspension is sprayed to the point of run-off on cucumber seedlings. The following day the seedlings are inoculated with a spore suspension of *Botrytis cinerea* (the causal agent of gray mold on many crops) and incubated in a saturated atmosphere at 20° C for 48 h, and moved to a growth chamber at 20° C for 5 days, after which disease ratings are made.

Results for Test A to G are given in Table 1. In the table, a rating of 100 indicates 100% disease control and a rating of 0 indicated no disease control (relative to the carrier sprayed controls). NT indicates that no test was performed.

The Index Table below identifies the compounds evaluated in Tests A-G.

INDEX TABLE

Compounds of Formula I wherein

$R^1$ = Me, $R^3$ = Ph, $R^4$ = H

| Cmpd. No. | $R^2$ | A | m.p. (°C) |
|---|---|---|---|
| 1 | Me | $H_2N^+Br^-$ | 223 dec |
| 2 | n-octyl | $H_2N^+Br^-$ | 183-186 dec |
| 3 | 4-PhO-Ph | $H_2N^+Br^-$ | 161 dec |
| 4 | Me | PhNHC(=O)N | 157-158 dec |
| 5 | 4-PhO-Ph | $BrCH_2C(=O)N$ | 156-158 dec |
| 6 | 4-PhO-Ph | $Me_3C(=O)N$ | 152-155 dec |
| 7 | 2,4-diF-Ph | HN | 186-189 |
| 8 | 4-PhO-Ph | HN | 137-139 |
| 9 | 4-PhO-Ph | MeC(=O) | 160-162 dec |
| 10 | 4-PhO-Ph | MeOC(=O)N | 147-149 dec |
| 11 | 4-PhO-Ph | PhNHC(=O)N | 104-106 dec |
| 12 | 4-PhO-Ph | PhC(=O) | 100-104 dec |
| 13 | 4-PhO-Ph | $Me_2P(=O)$ | 172-180 |
| 14 | 2,4-diF-Ph | S | 124 |
| 15 | 4-PhO-Ph | S | 110-112 |
| 16 | n-octyl | S | oil[1] |
| 17 | 2,4-diF-Ph | $BrCH_2C(=O)N$ | 128-130 dec |
| 18 | 4-PhO-Ph | $H_2N^+$(dodecyl-benzenesulfonate)$^-$ | 67-75 |

[1]Anal. Calcd. for $C_{18}H_{26}N_2O_2S$: C 64.63; H 7.84; N 8.38; S 9.59; Found (average of 2): C 62.98; H 7.68; N 8.00; S 12.34; IR: 1807 $cm^{-1}$

TABLE I

| CMPD. NO. | TEST A | TEST B | TEST C | TEST D | TEST E | TEST F | TEST G |
|---|---|---|---|---|---|---|---|
| 1 | 0 | 84 | 14 | 0 | NT | 14 | 35 |
| 2 | 94 | 5 | 12 | 96 | 63* | 100 | 3 |
| 3 | 59 | 100 | 81 | 99 | 93 | 100 | 56 |
| 4 | 0 | 0 | 7 | 75 | 32 | 99 | 75 |
| 5 | 0 | 84 | 27 | 74 | 33* | 99 | 60 |
| 6 | 53 | 93 | 0 | 84 | 29* | 100 | 38 |
| 7 | 53 | 83 | 0 | 92 | 68* | 100 | 38 |
| 8 | 0 | 96 | 27 | 69 | NT | 100 | 8 |
| 9 | 0 | 80* | 0 | 30 | NT | 100 | 0 |
| 10 | 57 | 55 | 0 | 69 | NT | 100 | 0 |
| 11 | 0 | 0 | 0 | 30 | NT | 100 | 8 |
| 12 | 0 | 80 | 0 | 52 | NT | 100 | 0 |
| 13 | 57 | 91 | 27 | 82 | NT | 100 | 0 |
| 14 | 0 | 89 | 0 | 91 | NT | 100 | NT |
| 15 | 0 | 93 | 0 | 73 | 9* | 100 | 0 |
| 16 | 83 | 0 | 28 | 73 | 66* | 85 | 38 |
| 17 | 83 | 17 | 0 | 73 | NT | 100 | 0 |

* - Plants were sprayed at a concentration of 40 ppm.

From the foregoing description, one skilled in the art can easily ascertain the essential characteristics of this invention, and without departing from the spirit and scope thereof, can make various changes and modifications of the invention to adapt it to various usages and conditions.

**Claims**

**1.** A fungicidally active compound of Formula I comprising

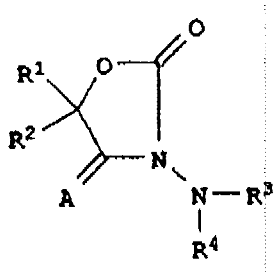

I

wherein:

| | |
|---|---|
| A | is S or N-J; |
| J | is $R^{15}$; $C(=O)R^{16}$; $C(=O)OR^{17}$; $C(=O)SR^{18}$; $C(=O)NR^{19}R^{20}$; $P(=O)(C_1$-$C_4$ alkyl$)_2$; or OG; |
| G | is H; $C_1$-$C_6$ alkyl; benzyl optionally substituted with $R^{34}$ on the phenyl ring; $C(=O)(C_1$-$C_4$ alkyl); $C(=O)(C_1$-$C_4$ alkoxy); or C-$(=O)NHR^{36}$; |
| $R^1$ | is $C_1$-$C_4$ alkyl; $C_1$-$C_4$ haloalkyl; $C_3$-$C_6$ cycloalkyl; $C_2$-$C_4$ alkenyl; $C_2$-$C_4$ alkoxycarbonyl; or phenylmethyl optionally substituted with $R^6$ on the phenyl ring and with $R^8$ on the benzylic carbon; |
| $R^2$ | is $C_1$-$C_{20}$ alkyl optionally substituted with $R^{22}$; $C_2$-$C_{20}$ alkoxyalkyl optionally substituted with $R^{35}$; $C_2$-$C_{20}$ alkenyl optionally |

subitituted with $R^{42}$; $C_2$-$C_{20}$ alkynyl optionally substituted with $R^{41}$; $(CH_2CH_2OCH_2CH_2)CH$-; $(CH_2CH_2SCH_2CH_2)CH$-; $(CH_2CH_2SO_2CH_2CH_2)CH$-; $C_5$-$C_7$ cycloalkyl; $C_5$-$C_7$ cycloalkenyl; phenyl optionally substituted with $R^5$ and $R^7$, 2-naphthalenyl; thienyl optionally substituted with $R^5$ and $R^7$; furyl optionally substituted with $R^7$; or pyridyl optionally substituted with $R^5$ and $R^7$; or

$R^1$ and $R^2$ can be taken together to form structures selected from the group consisting of $-CH_2(CH_2)_2CH_2-$, $-CH_2(CH_2)_3CH_2-$, $-CH_2(CH_2)_4CH_2-$, $-CH_2CH_2OCH_2CH_2-$, $-CH_2CH_2SCH_2CH_2-$,

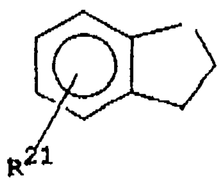 , 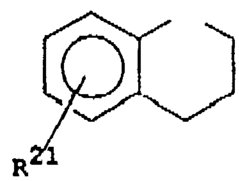 , and 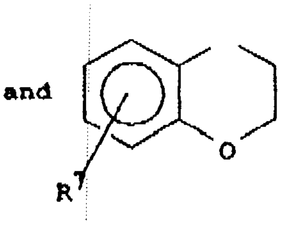 ;

$R^3$ is phenyl, pyridyl, or pyrimidinyl each optionally substituted with $R^{10}$ or phenylmethyl;

$R^4$ is H or methyl;

$R^5$ is halogen; nitro; cyano; $C_1$-$C_6$ alkyl; $C_5$-$C_6$ cycloalkyl; $C_1$-$C_6$ haloalkyl; $C_1$-$C_6$ alkylthio; $C_1$-$C_6$ haloalkylthio; $C_1$-$C_6$ alkoxy; $C_1$-$C_6$ haloalkoxy; $C_5$-$C_6$ cycloalkyloxy; $C_2$-$C_6$ alkoxyalkyl; $C_2$-$C_6$ alkoxyalkoxy; $C_3$-$C_6$ alkenyl; $C_3$-$C_6$ haloalkenyl; $C_3$-$C_6$ alkenyloxy; $C_3$-$C_6$ alkynyl; $C_3$-$C_6$ haloalkynyl; $C_3$-$C_6$ alkynyloxy; $C_1$-$C_6$ alkylsulfonyl; $C_1$-$C_6$ haloalkylsulfonyl; phenyl or phenylthio each optionally substituted with $R^{26}$; phenylmethyl, phenoxymethyl, phenethyl, or styryl each optionally substituted with $R^{26}$ on the phenyl ring; phenoxy optionally substituted with $R^{27}$; benzyloxy optionally substituted with $R^{30}$ on the phenyl ring; $-OC(=O)NHR^{28}$; $-C(=O)OR^{28}$; or $-OC(=O)R^{28}$;

$R^6$, $R^7$, $R^{12}$, $R^{13}$, $R^{24}$, $R^{26}$ and $R^{34}$ are independently 1-2 halogen; nitro; $C_1$-$C_4$ alkyl; trifluoromethyl; methylthio; or $C_1$-$C_4$ alkoxy;

$R^8$, $R^{14}$, $R^{20}$, $R^{40}$ and $R^{38}$ are independently H or $C_1$-$C_4$ alkyl;

$R^9$ is $C_1$-$C_{18}$ alkyl; or phenyl optionally substituted with $R^7$;

$R^{10}$, $R^{25}$ and $R^{33}$ are independently 1-2 substituents selected from the group consisting of halogen, nitro, cyano, $C_1$-$C_4$ alkyl, trifluoromethyl, $C_1$-$C_4$ alkylthio, $C_1$-$C_4$ alkoxy and trifluoromethoxy;

$R^{11}$ and $R^{36}$ are independently $C_1$-$C_6$ alkyl; or phenyl optionally substituted with $R^{12}$;

$R^{15}$ is H; $C_1$-$C_8$ alkyl optionally substituted with $C_1$-$C_2$ alkoxy; $C_3$-$C_6$ cycloalkyl; $C_3$-$C_8$ alkenyl; $C_3$-$C_8$ alkynyl; phenyl optionally substituted with $R^{13}$; benzyl optionally substituted with $R^{13}$ on the phenyl ring and with $R^{20}$ on the benzylic carbon; or pyridyl optionally substituted with $R^{13}$;

$R^{16}$ is H; $C_1$-$C_{17}$ alkyl optionally substituted with $R^{31}$; $C_2$-$C_{17}$ alkenyl optionally substituted with $R^{32}$; $C_2$-$C_7$ alkynyl; $C_3$-$C_8$ cycloalkyl; $C_5$-$C_6$ cycloalkenyl; $C_6$-$C_7$ alkylcycloalkyl; $C_4$-$C_8$ cycloalkylalkyl; phenyl optionally substituted with $R^{33}$; naphthalenyl, furanyl, thienyl, benzoyl, or pyridyl each optionally substituted with $R^{34}$; or $C_2$-$C_5$ alkoxycarbonyl;

$R^{17}$ and $R^{18}$ are independently $C_1$-$C_{18}$ alkyl optionally substituted with $R^{23}$; $C_2$-$C_{10}$ alkenyl optionally substituted with $R^{32}$; $C_3$-$C_8$ alkynyl; $C_3$-$C_{12}$ cycloalkyl; $C_5$-$C_6$ cycloalkenyl; $C_6$-$C_7$ alkylcycloalkyl;

$C_6$-$C_7$ cycloalkylalkyl; or phenyl, naphthalenyl, or thienyl each optionally substituted with $R^{34}$;

$R^{19}$ is H; $C_1$-$C_{10}$ alkyl; $C_5$-$C_6$ cycloalkyl; or phenyl optionally substituted with $R^{34}$; or

$R^{19}$ and $R^{20}$ can be taken together to form structures selected from the group consisting of $-CH_2(CH_2)_2CH_2-$, $-CH_2(CH_2)_3CH_2-$, $-CH_2(CH_2)_4CH_2-$, $-CH_2CH_2OCH_2CH_2-$, $-CH_2CH(Me)CH_2CH(Me)CH_2-$, and $-CH_2CH(Me)OCH(Me)CH_2-$;

$R^{21}$ is 1-2 halogen; nitro; $C_1$-$C_4$ alkyl; trifluoromethyl; methylthio; or $C_1$-$C_4$ alkoxy; or phenoxy optionally substituted with $R^{26}$;

$R^{22}$ is cyano; nitro; $C_1$-$C_{19}$ alkylthio; $C_1$-$C_{19}$ alkylsulfinyl; $C_1$-$C_{19}$ haloalkoxy; $C_5$-$C_6$ cycloalkyloxy; $C_3$-$C_{19}$ alkenyloxy; $C_3$-$C_{19}$ alkynyloxy; $C_1$-$C_{19}$ alkylsulfonyl; $C_2$-$C_{19}$ alkoxycarbonyl; hydroxyl; hydroxycarbonyl; $R^{28}C(=O)O$; $R^{28}OC(=O)O$; $R^{28}R^{40}NC(=O)O$; $R^{39}R^{40}N$; $(C_1$-$C_4$ alkoxy$)_2P(=E)O$; $R^{11}SO_3$; $R^{40}R^{14}R^{38}N^+$;phenyl, phenylthio, phenoxy, phenylsulfonyl, phenylsulfinyl, pyridyl or pyridyloxy each optionally substituted with $R^{30}$; thienyl, pyrimidinyl, furanyl, naphthalenyl, pyrimidinyloxy, naphthalenyloxy each optionally substituted with $R^7$; tetrahydropyranyl; $C_3$-$C_6$ cycloalkyl; 2-tetrahydropyranyloxy; or $C(=Q)R^{40}$;

E is O or S;

Q is O or N-T-W;

T is Q; $NR^{37}$; or a direct bond;

W is H; $C_1$-$C_8$ alkyl, $C_3$-$C_8$ alkenyl; phenylmethyl optionaliy substituted with $R^7$ on the phenyl ring and $R^{14}$ on the benzylic carbon; phenyl or pyridyl each optionally substituted with $R^7$; $C(=O)R^{28}$; $C(=O)OR^{28}$; or $C(=O)NR^{28}R^{14}$;

$R^{23}$ is 1-3 halogen; $C_1$-$C_{12}$ alkoxy; $C_1$-$C_{12}$ alkylthio; phenyl or naphthalenyl each optionally substituted with $R^{34}$; or phenoxymethyl optionally substituted with $R^{34}$ on the phenyl ring;

$R^{27}$ is 1-2 halogen; nitro; cyano; $C_1$-$C_6$ alkyl; $C_1$-$C_6$ haloalkyl; $C_1$-$C_6$ alkoxy; $C_1$-$C_6$ haloalkoxy; $C_1$-$C_4$ alkylsulfonyl; $C_2$-$C_6$ alkoxyalkyl; $C_1$-$C_4$ alkylthio; $C_5$-$C_6$ cycloalkyl; $C_5$-$C_6$ cycloalkyloxy; $C_2$-$C_6$ alkenyl; $C_2$-$C_6$ haloalkenyl; $C_2$-$C_6$ alkynyl; hydroxycarbonyl; $C_2$-$C_4$ alkoxycarbonyl; or phenoxy optionally substituted with $R^{24}$;

$R^{28}$ is $C_1$-$C_8$ alkyl; or phenyl or pyridyl each optionally substituted with $R^{30}$;

$R^{30}$ is 1-2 substituents selected from the group consisting of halogen, nitro, cyano $C_1$-$C_4$ alkyl, trifluoromethyl, $C_1$-$C_4$ alkoxy and trifluoromethoxy; or phenoxy optionally substituted with $R^{26}$;

$R^{31}$ is 1-3 halogen; $C_1$-$C_{18}$ alkoxy; allyloxy; $C_1$-$C_{18}$ alkylthio; phenyl, phenoxy, benzyloxy, or phenylthio each optionally substituted with $R^{34}$ on the phenyl ring; acetyl; or $C_2$-$C_5$ alkoxycarbonyl;

$R^{32}$ is 1-3 halogen; or $C_1$-$C_4$ alkoxy;

$R^{35}$ is cyano; nitro; $C_1$-$C_{17}$ alkylthio; $C_1$-$C_{17}$ alkylsulfinyl; $C_1$-$C_{17}$ haloalkoxy; $C_5$-$C_6$ cycloalkyloxy; $C_2$-$C_{17}$ haloalkenyl; $C_3$-$C_{17}$ alkenyloxy; $C_3$-$C_{17}$ haloalkynyl; $C_3$-$C_{17}$ alkynyloxy; $C_1$-$C_{17}$ alkylsulfonyl; $C_2$-$C_{17}$ alkoxycarbonyl; hydroxyl; hydroxycarbonyl; $R^{28}C(=O)O$; $R^{28}OC(=O)O$; $R^{28}R^{40}NC(=O)O$; $R^{40}R^{39}N$; $(C_1$-$C_4$ alkoxy$)_2P(=E)O$; $R^{11}SO_3$; $R^{40}R^{14}R^{38}N^+$;phenyl, phenoxy, phenylthio, phenylsulfonyl, phenylsulfinyl, pyridyl or pyridyloxy each optionally substituted with $R^{30}$; thienyl, pyrimidinyl, furanyl, naphthalenyl, pyrimidinyloxy, naph-

| | |
|---|---|
| | thalenyloxy each optionally substituted with $R^7$; tetrahydropyranyl; 2-tetrahydropyranyloxy; $C_1$-$C_{17}$ alkoxy; $C_2$-$C_{17}$ alkoxyalkoxy; $C_3$-$C_{17}$ alkynyl; $C_3$-$C_6$ cycloalkyl; or $C_2$-$C_{17}$ haloalkoxyalkoxy; |
| $R^{37}$ | is H; $C_1$-$C_6$ alkyl; or phenyl optionally substituted with $R^7$; |
| $R^{39}$ | is $C_1$-$C_{19}$ alkyl; $C_2$-$C_{19}$ alkylcarbonyl; $C_2$-$C_{19}$ alkoxycarbonyl, $(R^9R^{40}N)C=O$; phenyl optionally substituted with $R^{25}$; or phenoxycarbonyl optionally substituted with $R^7$; |
| $R^{41}$ | is cyano; nitro; $C_1$-$C_{17}$ alkylthio; $C_1$-$C_{17}$ alkylsulfinyl; $C_1$-$C_{17}$ haloalkoxy; $C_5$-$C_6$ cycloalkyloxy; $C_3$-$C_{17}$ alkenyloxy; $C_3$-$C_{17}$ alkynyloxy; $C_1$-$C_{17}$ alkylsulfonyl; $C_2$-$C_{17}$ alkoxycarbonyl; hydroxyl; hydroxycarbonyl; $R^{28}C(=O)O$; $R^{28}OC(=O)O$; $R^{28}R^{40}NC(=O)O$; $R^{40}R^{39}N$; $(C_1$-$C_4$ alkoxy$)_2P(=E)O$; $R^{11}SO_3$; $R^{40}R^{14}R^{38}N^+$;phenyl, phenoxy, phenylsulfonyl, phenylsulfinyl, pyridyl or pyridyloxy each optionally substituted with $R^{30}$; thienyl, pyrimidinyl, furanyl, naphthalenyl, pyrimidinyloxy, naphthalenyloxy each optionally substituted with $R^7$; tetrahydropyranyl; 2-tetrahydropyranyloxy; $C_1$-$C_{17}$ alkoxy; 1-3 halogen; $C_2$-$C_{17}$ alkoxyalkoxy; or $C_3$-$C_6$ cycloalkyl; |
| $R^{42}$ | is cyano; nitro; $C_1$-$C_{17}$ alkylthio; $C_1$-$C_{17}$ alkylsulfinyl; $C_1$-$C_{17}$ haloalkoxy; $C_5$-$C_6$ cycloalkyloxy; $C_3$-$C_{17}$ alkenyloxy; $C_3$-$C_{17}$ haloalkynyl; $C_3$-$C_{17}$ alkynyloxy; $C_1$-$C_{17}$ alkylsulfonyl; $C_2$-$C_{17}$ alkoxycarbonyl; hydroxyl; hydroxycarbonyl; $R^{28}C(=O)O$; $R^{28}OC(=O)O$; $R^{28}R^{40}NC(=O)O$; $R^{40}R^{39}N$; $(C_1$-$C_4$ alkoxy$)_2P(=E)O$; $R^{11}SO_3$; $R^{40}R^{14}R^{38}N^+$; phenyl, phenoxy, phenylthio, phenylsulfonyl, phenylsulfinyl, pyridyl or pyridyloxy each optionally substituted with $R^{30}$; thienyl, pyrimidinyl, furanyl, naphthalenyl, pyrimidinyloxy, naphthalenyloxy each optionally substituted with $R^7$; tetrahydropyranyl; 2-tetrahydropyranyloxy; $C_1$-$C_{17}$ alkoxy; 1-3 halogen; $C_2$-$C_{17}$ alkoxyalkoxy; $C_3$-$C_{17}$ alkynyl; or $C_3$-$C_6$ cycloalkyl; |

provided that

i) when $R^2$ is a substituted phenyl or heterocyclic ring, then only H or F can be substituted on the carbon atom(s) of the phenyl or heterocyclic ring adjacent to the carbon bearing the oxazolidinone ring;

ii) when $R^3$ is a phenyl or heterocyclic ring disubstituted with two alkyl or alkoxy groups, or one alkyl and one alkoxy group, then at least one of the alkyl and alkoxy groups is methyl or methoxy; and

iii) the total number of carbons in $R^2$, $R^{16}$, $R^{17}$, and $R^{18}$ is each less than or equal to 20.

**2.** A fungicidally active compound of Formula I comprising

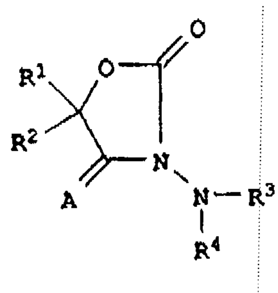

I

wherein:

| | |
|---|---|
| A | is S or N-OG; |
| G | is H; $C_1$-$C_6$ alkyl; benzyl optionally substituted with $R^{34}$ on the phenyl ring; $C(=O)(C_1$-$C_4$ alkyl); $C(=O)(C_1$-$C_4$ alkoxy); or $C(=O)NHR^{36}$; |

$R^1$ is $C_1$-$C_4$ alkyl; $C_1$-$C_4$ haloalkyl; $C_3$-$C_6$ cycloalkyl; $C_2$-$C_4$ alkenyl; $C_2$-$C_4$ alkoxycarbonyl; or phenylmethyl optionally substituted with $R^6$ on the phenyl ring and with $R^8$ on the benzylic carbon;

$R^2$ is $C_1$-$C_6$ alkyl; $C_5$-$C_7$ cycloalkyl; $C_2$-$C_6$ alkenyl; $C_5$-$C_7$ cycloalkenyl; $C_5$-$C_7$ cycloalkenyl; phenyl optionally substituted with $R^5$ and $R^7$; 2-naphthalenyl; thienyl optionally substituted with $R^5$ and $R^7$; furyl optionally substituted with $R^7$; or pyridyl optionally substituted with $R^7$; or phenoxy or phenylthio each optionally substituted with $R^7$; or

$R^1$ and $R^2$ can be taken together to form structures selected from the group consisting of
$-CH_2(CH_2)_2CH_2-$, $-CH_2(CH_2)_3CH_2-$, $-CH_2(CH_2)_4CH_2-$, $-CH_2CH_2OCH_2CH_2-$, $-CH_2CH_2SCH_2CH_2-$,

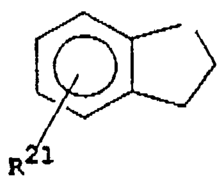
,
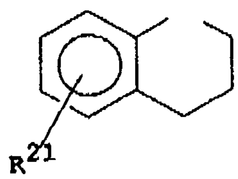
, and
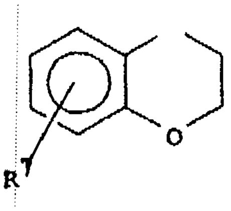
;

$R^3$ is phenyl, pyridyl, or pyrimidinyl each optionally substituted with $R^{10}$; or phenylmethyl;

$R^4$ is H or methyl;

$R^5$ is halogen; nitro; cyano; $C_1$-$C_6$ alkyl; $C_5$-$C_6$ cycloalkyl; $C_1$-$C_6$ haloalkyl; $C_1$-$C_6$ alkylthio; $C_1$-$C_6$ haloalkylthio; $C_1$-$C_6$ alkoxy; $C_1$-$C_6$ haloalkoxy; $C_5$-$C_6$ cycloalkyloxy; $C_2$-$C_6$ alkoxyalkyl; $C_2$-$C_6$ alkoxyalkoxy; $C_3$-$C_6$ alkenyl; $C_3$-$C_6$ haloalkenyl; $C_3$-$C_6$ alkenyloxy; $C_3$-$C_6$ alkynyl; $C_3$-$C_6$ haloalkynyl; $C_3$-$C_6$ alkynyloxy; $C_1$-$C_6$ alkylsulfonyl; $C_1$-$C_6$ haloalkylsulfonyl; phenyl or phenylthio each optionally substituted with $R^{24}$; phenylmethyl, phenoxymethyl, phenethyl, or styryl each optionally substituted with $R^{24}$ on the phenyl ring; phenoxy optionally substituted with $R^{27}$; and $C_2$-$C_6$ alkoxycarbonyl;

$R^6$, $R^7$, $R^{24}$, $R^{26}$ and $R^{34}$ are independently 1-2 halogen; nitro; $C_1$-$C_4$ alkyl; trifluoromethyl; methylthio; or $C_1$-$C_4$ alkoxy;

$R^8$ is H or $C_1$-$C_4$ alkyl;

$R^{10}$ is 1-2 substituents selected from the group consisting of halogen, nitro, cyano, $C_1$-$C_4$ alkyl, trifluoromethyl, $C_1$-$C_4$ alkylthio, $C_1$-$C_4$ alkoxy and trifluoromethoxy;

$R^{21}$ is halogen; nitro; $C_1$-$C_4$ alkyl; trifluoromethyl; methyltho; or $C_1$-$C_4$ alkoxy; or phenoxy optionally substituted with $R^{26}$;

$R^{27}$ is 1-2 halogen; nitro; cyano; $C_1$-$C_6$ alkyl; $C_1$-$C_6$ haloalkyl; $C_1$-$C_6$ alkoxy; $C_1$-$C_6$ haloalkoxy; $C_1$-$C_4$ alkylsulfonyl; $C_2$-$C_6$ alkoxyalkyl; $C_1$-$C_4$ alkylthio; $C_5$-$C_6$ cycloalkyl; $C_5$-$C_6$ cycloalkyloxy; $C_2$-$C_6$ alkenyl; $C_2$-$C_6$ haloalkenyl; $C_2$-$C_6$ alkynyl; $C_2$-$C_4$ alkoxycarbonyl; or phenoxy; and

$R^{36}$ is $C_1$-$C_6$ alkyl; or phenyl optionally substituted with $R^{12}$.

**3.** A fungicidally active compound according to Claim 1 wherein:

J is H; OH; $C(=O)R^{16}$; $C(=O)OR^{17}$; $C(=O)NHR^{19}$;

$R^1$ is $C_1$-$C_4$ alkyl; $C_1$-$C_4$ haloalkyl; or vinyl;

$R^2$ is $C_1$-$C_{20}$ alkyl; $C_2$-$C_{20}$ alkoxyalkyl; $C_2$-$C_{20}$ haloalkyl; $C_3$-$C_8$ alkyl substituted with phenoxy or phenylthio each optionally substituted with $R^{30}$; $C_5$-$C_7$ cycloalkyl; $C_2$-$C_{20}$ alkenyl; $C_5$-$C_7$ cycloalkenyl; phenyl optionally substituted with $R^5$ and $R^7$; 2-naphthalenyl; thienyl optionally substituted with $R^5$ and $R^7$; furyl optionally substituted with $R^7$; or pyridyl optionally substituted with $R^5$ and $R^7$;

$R^3$ is phenyl optionally substituted with $R^{10}$;

$R^{16}$ is H; $C_1$-$C_6$ alkyl; or phenyl optionally substituted with $R^{34}$;

$R^{17}$ is $C_1$-$C_6$ alkyl;

$R^{19}$ is $C_1$-$C_6$ alkyl; or phenyl optionally substituted with $R^{34}$;
provided that when $R^2$ in phenyl and $R^5$ is not F, then
$R^5$ is attached to the para-position relative to the oxazolidinone ring.

**4.** A fungicidally active compound according to Claim 3 wherein:

- J is H; OH or C(=O)$R^{16}$;
- $R^1$ is $C_1$-$C_2$ alkyl or vinyl;
- $R^2$ is $C_1$-$C_{12}$ alkyl; $C_5$-$C_7$ cycloalkyl; phenyl optionally substituted with $R^5$ and $R^7$; thienyl optionally substituted with $R^7$; or pyridyl optionally substituted with $R^5$ and $R^7$;
- $R^3$ is phenyl optionally substituted with F; Cl; or methyl;
- $R^4$ is H;
- $R^5$ is halogen; nitro; $C_1$-$C_6$ alxyl; $C_1$-$C_3$ haloalkyl;methylthio; $C_1$-$C_6$ alkoxy; $C_1$-$C_2$ haloalkoxy; $C_5$-$C_6$ cycloalkyloxy; phenoxy optionally substituted with $R^{27}$; phenylthio substituted with $R^{24}$; phenoxymethyl optionally substituted with $R^{24}$ on the phenyl ring; benzyloxy optionally substituted with $R^{30}$ on the phenyl ring or -OC(=O)NH$R^{28}$;
- $R^7$ ahd $R^{24}$ are independently F; $C_1$-$C_2$ alkyl; methylthio or $C_1$-$C_2$ alkoxy;
- $R^{16}$ is $C_1$-$C_6$ alkyl;
- $R^{19}$ is phenyl optionally substituted with $R^{34}$;
- $R^{27}$ is 1-2 halogen; cyano; $C_1$-$C_4$ alkyl; trifluoromethyl; $C_1$-$C_4$ alkoxy; $C_1$-$C_4$ haloalkoxy; $C_1$-$C_4$ alkylthio; $C_5$-$C_6$ cycloalkyloxy; or allyl;
- $R^{30}$ is 1-2 halogen, cyano, $C_1$-$C_4$ alkyl, trifluoromethyl, $C_1$-$C_4$ alkoxy or trifluoromethoxy; and
- $R^{34}$ is 1-2 halogen; nitro; $C_1$-$C_2$ alkyl; or $C_1$-$C_2$ alkoxy.

**5.** A fungicidally active compound according to Claim 4 wherein:

- J is H;
- $R^1$ is methyl;
- $R^2$ is $C_1$-$C_{12}$ alkyl; phenyl optionally substituted with $R^5$ and $R^7$; pyridyl optionally substituted with $R^5$ and $R^7$; and
- $R^5$ is F; Cl; Br; $C_1$-$C_6$ alkyl; trifluoromethyl; $C_1$-$C_6$ alkoxy; trifluoromethoxy; 2,2,2-trifluoroethoxy; $C_5$-$C_6$ cycloalkyloxy; methylthio; phenoxy optionally substituted with $R^{27}$; phenylthio optionally substituted with $R^{24}$; benzyloxy optionally substituted with $R^{30}$ on the phenyl ring; or -OC(=O)$R^{28}$.

**6.** A fungicidally active compound according to Claim 5 selected from the group consisting of

5-(2,4-Difluorophenyl)-5-methyl-3-(phenylamino)-4-thioxo-2-oxazolidinone;

4-Imino-5-methyl-5-(4-phenoxyphenyl)-3-(phenylamino)-2-oxazolidinone;

4-Imino-5-methyl-5-(4-phenoxyphenyl)-3-(phenylamino)-2-oxazolidinone hydrobromide; and

5-(2,4-Difluorophenyl)-4-imino-5-methyl-3-(phenylamino)-2-oxazolidinone.

**7.** A fungicidally active composition comprising the compounds of any one of Claims 1-6.

**8.** A method of controlling disease in plants comprising applying to said plants a fungicidally active compound of Formula I

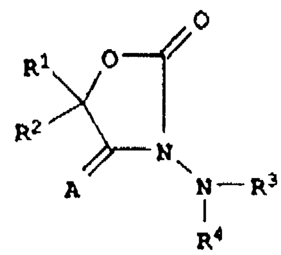

I

wherein:

A — is S or N-J;

J — is $R^{15}$; $C(=O)R^{16}$; $C(=O)OR^{17}$; $C(=O)SR^{18}$; $C(=O)NR^{19}R^{20}$; $P(=O)(C_1\text{-}C_4\ alkyl)_2$; or OG;

G — is H; $C_1$-$C_6$ alkyl; benzyl optionally substituted with $R^{34}$ on the phenyl ring; $C(=O)(C_1\text{-}C_4\ alkyl)$; $C(=O)(C_1\text{-}C_4\ alkoxy)$; or $C(=O)NHR^{36}$;

$R^1$ — is $C_1$-$C_4$ alkyl; $C_1$-$C_4$ haloalkyl; $C_3$-$C_6$ cycloalkyl; $C_2$-$C_4$ alkenyl; $C_2$-$C_4$ alkoxycarbonyl; or phenylmethyl optionally substituted with $R^6$ on the phenyl ring and with $R^8$ on the benzylic carbon;

$R^2$ — is $C_1$-$C_{20}$ alkyl optionally subatituted with $R^{22}$; $C_2$-$C_{20}$ alkoxyalkyl optionally substituted with $R^{35}$; $C_2$-$C_{20}$ alkenyl optionally substituted with $R^{42}$; $C_2$-$C_{20}$ alkynyl optionally substituted with $R^{41}$; $(CH_2CH_2OCH_2CH_2)CH$-; $(CH_2CH_2SCH_2CH_2)CH$-; $(CH_2CH_2SO_2CH_2CH_2)CH$-; $C_5$-$C_7$ cycloalkyl; $C_5$-$C_7$ cycloalkenyl; phenyl optionally substituted with $R^5$ and $R^7$; 2-naphthalenyl; thienyl optionally substituted with $R^5$ and $R^7$; furyl optionally substituted with $R^7$; or pyridyl optionally substituted with $R^5$ and $R^7$; or

$R^1$ and $R^2$ — can be taken together to form structures selected from the group consisting of $-CH_2(CH_2)_2CH_2-$, $-CH_2(CH_2)_3CH_2-$, $-CH_2(CH_2)_4CH_2-$, $-CH_2CH_2OCH_2CH_2-$, $-CH_2CH_2SCH_2CH_2-$,

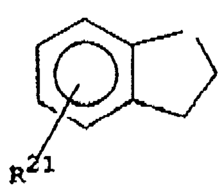 , 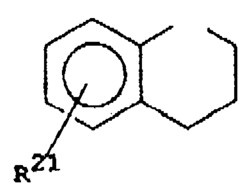 , and 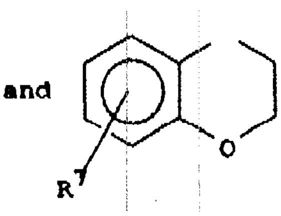 ;

$R^3$ — is phenyl, pyridyl, or pyrimidinyl each optionally substituted with $R^{10}$ or phenylmethyl;

$R^4$ — is H or methyl;

$R^5$ — is halogen; nitro; cyano; $C_1$-$C_6$ alkyl; $C_5$-$C_6$ cycloalkyl; $C_1$-$C_6$ haloalkyl; $C_1$-$C_6$ alkylthio; $C_1$-$C_6$ haloalkylthio; $C_1$-$C_6$ alkoxy; $C_1$-$C_6$ haloalkoxy; $C_5$-$C_6$ cycloalkyloxy; $C_2$-$C_6$ alkoxyalkyl; $C_2$-$C_6$ alkoxyalkoxy; $C_3$-$C_6$ alkenyl; $C_3$-$C_6$ haloalkenyl; $C_3$-$C_6$ alkenyloxy; $C_3$-$C_6$ alkynyl; $C_3$-$C_6$ haloalkynyl; $C_3$-$C_6$ alkynyloxy; $C_1$-$C_6$ alkylsulfonyl; $C_1$-$C_6$ haloalkylsulfonyl; phenyl or phenylthio each optionally substituted with $R^{26}$; phenylmethyl, phenoxymethyl, phenethyl, or styryl each optionally substituted with $R^{26}$ on the phenyl ring; phenoxy optionally

substituted with $R^{27}$; benzyloxy optionally substituted with $R^{30}$ on the phenyl ring; -OC(=O)NHR$^{28}$; - C(=O)OR$^{28}$; or -OC-(=O)R$^{28}$;

| | |
|---|---|
| $R^6$, $R^7$, $R^{12}$, $R^{13}$, $R^{24}$, $R^{26}$ and $R^{34}$ | are independently 1-2 halogen; nitro; $C_1$-$C_4$ alkyl; trifluoromethyl; methylthio; or $C_1$-$C_4$ alkoxy; |
| $R^8$, $R^{14}$, $R^{20}$, $R^{40}$ and $R^{38}$ | are independently H or $C_1$-$C_4$ alkyl; |
| $R^9$ | is $C_1$-$C_{18}$ alkyl; or phenyl optionally substituted with $R^7$; |
| $R^{10}$, $R^{25}$ and $R^{33}$ | are independently 1-2 substituents selected from the group consisting of halogen, nitro, cyano, $C_1$-$C_4$ alkyl, trifluoromethyl, $C_1$-$C_4$ alkylthio, $C_1$-$C_4$ alkoxy and trifluoromethoxy; |
| $R^{11}$ and $R^{36}$ | are independently $C_1$-$C_6$ alkyl; or phenyl optionally substituted with $R^{12}$; |
| $R^{15}$ | is H; $C_1$-$C_8$ alkyl optionally substituted with $C_1$-$C_2$ alkoxy; $C_3$-$C_6$ cycloalkyl; $C_3$-$C_8$ alkenyl; $C_3$-$C_8$ alkynyl; phenyl optionally substituted with $R^{13}$; benzyl optionally substituted with $R^{13}$ on the phenyl ring and with $R^{20}$ on the benzylic carbon; or pyridyl optionally substituted with $R^{13}$; |
| $R^{16}$ | is H; $C_1$-$C_{17}$ alkyl optionally substituted with $R^{31}$; $C_2$-$C_{17}$ alkenyl optionally substituted with $R^{32}$; $C_2$-$C_7$ alkynyl; $C_3$-$C_8$ cycloalkyl; $C_5$-$C_6$ cycloalkenyl; $C_6$-$C_7$ alkylcycloalkyl; $C_4$-$C_8$ cycloalkylalkyl; phenyl optionally substituted with $R^{33}$; naphthalenyl, furanyl, thienyl, benzoyl, or pyridyl each optionally substituted with $R^{34}$; or $C_2$-$C_5$ alkoxycarbonyl; |
| $R^{17}$ and $R^{18}$ | are independently $C_1$-$C_{18}$ alkyl optionally substituted with $R^{23}$; $C_2$-$C_{10}$ alkenyl optionally substituted with $R^{32}$; $C_3$-$C_8$ alkynyl; $C_3$-$C_{12}$ cycloalkyl; $C_5$-$C_6$ cycloalkenyl; $C_6$-$C_7$ alkylcycloalkyl; $C_6$-$C_7$ cycloalkylalkyl; or phenyl, naphthalenyl, or thienyl each optionally substituted with $R^{34}$; |
| $R^{19}$ | is H; $C_1$-$C_{10}$ alkyl; $C_5$-$C_6$ cycloalkyl; or phenyl optionally substituted with $R^{34}$; or |
| $R^{19}$ and $R^{20}$ | can be taken together to form structures selected from the group consisting of $-CH_2(CH_2)_2CH_2-$, $-CH_2(CH_2)_3CH_2-$, $-CH_2(CH_2)_4CH_2-$, $-CH_2CH_2OCH_2CH_2-$, $-CH_2CH(Me)CH_2CH(Me)CH_2-$, and $-CH_2CH(Me)OCH(Me)CH_2-$; |
| $R^{21}$ | is 1-2 halogen; nitro; $C_1$-$C_4$ alkyl; trifluoromethyl; methylthio; or $C_1$-$C_4$ alkoxy; or phenoxy optionally substituted with $R^{26}$; |
| $R^{22}$ | is cyano; nitro; $C_1$-$C_{19}$ alkylthio; $C_1$-$C_{19}$ alkylsulfinyl; $C_1$-$C_{19}$ haloalkoxy; $C_5$-$C_6$ cycloalkyloxy; $C_3$-$C_{19}$ alkenyloxy; $C_3$-$C_{19}$ alkynyloxy; $C_1$-$C_{19}$ alkylsulfonyl; $C_2$-$C_{19}$ alkoxycarbonyl; hydroxyl; hydroxycarbonyl; $R^{28}C(=O)O$; $R^{28}OC(=O)O$; $R^{28}R^{40}NC(=O)O$; $R^{39}R^{40}N$; $(C_1$-$C_4$ alkoxy$)_2P(=E)O$; $R^{11}SO_3$; $R^{40}R^{14}R^{38}N^+$;phenyl, phenylthio, phenoxy, phenylsulfonyl, phenylsulfinyl, pyridyl or pyridyloxy each optionally substituted with $R^{30}$; thienyl, pyrimidinyl, furanyl, naphthalenyl, pyrimidinyloxy, naphthalenyloxy each optionally substituted with $R^7$; tetrahydropyranyl; $C_3$-$C_6$ cycloalkyl; 2-tetrahydropyranyloxy; or $C(=Q)R^{40}$; |
| E | is O or S; |
| Q | is O or N-T-W; |
| T | is O; $NR^{37}$; or a direct bond; |
| W | is H; $C_1$-$C_8$ alkyl, $C_3$-$C_8$ alkenyl; phenylmethyl optionally substituted with $R^7$ on the phenyl ring and $R^{14}$ on the benzylic carbon; phenyl or pyridyl each optionally substituted with $R^7$; $C(=O)R^{28}$; $C(=O)OR^{28}$; or $C(=O)NR^{28}R^{14}$; |
| $R^{23}$ | is 1-3 halogen; $C_1$-$C_{12}$ alkoxy; $C_1$-$C_{12}$ alkylthio; phenyl or naphthalenyl each optionally substituted with $R^{34}$; or phenoxymethyl optionally substituted with $R^{34}$ on the phenyl ring; |

$R^{27}$ is 1-2 halogen; nitro; cyano; $C_1$-$C_6$ alkyl; $C_1$-$C_6$ haloalkyl; $C_1$-$C_6$ alkoxy; $C_1$-$C_6$ haloalkoxy; $C_1$-$C_4$ alkylsulfonyl; $C_2$-$C_6$ alkoxyalkyl; $C_1$-$C_4$ alkylthio; $C_5$-$C_6$ cycloalkyl; $C_5$-$C_6$ cycloalkyloxy; $C_2$-$C_6$ alkenyl; $C_2$-$C_6$ haloalkenyl; $C_2$-$C_6$ alkynyl; hydroxycarbonyl; $C_2$-$C_4$ alkoxycarbonyl; or phenoxy optionally substituted with $R^{24}$;

$R^{28}$ is $C_1$-$C_8$ alkyl; or phenyl or pyridyl each optionally substituted with $R^{30}$;

$R^{30}$ is 1-2 substituents selected from the group consisting of halogen, vitro, cyano, $C_1$-$C_4$ alkyl, trifluoromethyl, $C_1$-$C_4$ alkoxy and trifluoromethoxy; or phenoxy optionally substituted with $R^{26}$;

$R^{31}$ is 1-3 halogen; $C_1$-$C_{18}$ alkoxy; allyloxy; $C_1$-$C_{18}$ alkylthio; phenyl, phenoxy, benzyloxy, or phenylthio each optionally substituted with $R^{34}$ on the phenyl ring; acetyl; or $C_2$-$C_5$ alkoxycarbonyl;

$R^{32}$ is 1-3 halogen; or $C_1$-$C_4$ alkoxy;

$R^{35}$ is cyano; nitro; $C_1$-$C_{17}$ alkylthio; $C_1$-$C_{17}$ alkylsulfinyl; $C_1$-$C_{17}$ haloalkoxy; $C_5$-$C_6$ cycloalkyloxy; $C_2$-$C_{17}$ haloalkenyl; $C_3$-$C_{17}$ alkenyloxy; $C_3$-$C_{17}$ haloalkynyl; $C_3$-$C_{17}$ alkynyloxy; $C_1$-$C_{17}$ alkylsulfonyl; $C_2$-$C_{17}$ alkoxycarbonyl; hydroxyl; hydroxycarbonyl; $R^{28}C(=O)O$; $R^{28}OC(=O)O$; $R^{28}R^{40}NC(=O)O$; $R^{40}R^{39}N$; $(C_1$-$C_4$ alkoxy$)_2P(=E)O$; $R^{11}SO_3$; $R^{40}R^{14}R^{38}N^+$;phenyl, phenoxy, phenylthio, phenylsulfonyl, phenylsulfinyl, pyridyl or pyridyloxy each optionally substituted with $R^{30}$; thienyl, pyrimidinyl, furanyl, naphthalenyl, pyrimidinyloxy, naphthalenyloxy each optionally substituted with $R^7$; tetrahydropyranyl; 2-tetrahydropyranyloxy; $C_1$-$C_{17}$ alkoxy; $C_2$-$C_{17}$ alkoxyalkoxy; $C_3$-$C_{17}$ alkynyl; $C_3$-$C_6$ cycloalkyl; or $C_2$-$C_{17}$ haloalkoxyalkoxy;

$R^{37}$ is H; $C_1$-$C_6$ alkyl; or phenyl optionally substituted with $R^7$;

$R^{39}$ is $C_1$-$C_{19}$ alkyl; $C_2$-$C_{19}$ alkylcarbonyl; $C_2$-$C_{19}$ alkoxycarbonyl; $(R^9R^{40}N)C=O$; phenyl optionally substituted with $R^{25}$; or phenoxycarbonyl optionally substituted with $R^7$;

$R^{41}$ is cyano; nitro; $C_1$-$C_{17}$ alkylthio; $C_1$-$C_{17}$ alkylsulfinyl; $C_1$-$C_{17}$ haloalkoxy; $C_5$-$C_6$ cycloalkyloxy; $C_3$-$C_{17}$ alkenyloxy; $C_3$-$C_{17}$ alkynyloxy; $C_1$-$C_{17}$ alkylsulfonyl; $C_2$-$C_{17}$ alkoxycarbonyl; hydroxyl; hydroxycarbonyl; $R^{28}C(=O)O$; $R^{28}OC(=O)O$; $R^{28}R^{40}NC(=O)O$; $R^{40}R^{39}N$; $(C_1$-$C_4$ alkoxy$)_2P(=E)O$; $R^{11}SO_3$; $R^{40}R^{14}R^{38}N^+$;phenyl, phenoxy, phenylsulfonyl, phenylsulfinyl, pyridyl or pyridyloxy each optionally substituted with $R^{30}$; thienyl, pyrimidinyl, furanyl, naphthalenyl, pyrimidinyloxy, naphthalenyloxy each optionally substituted with $R^7$; tetrahydropyranyl; 2-tetrahydropyranyloxy; $C_1$-$C_{17}$ alkoxy; 1-3 halogen; $C_2$-$C_{17}$ alkoxyalkoxy; or $C_3$-$C_6$ cycloalkyl;

$R^{42}$ is cyano; nitro; $C_1$-$C_{17}$ alkylthio; $C_1$-$C_{17}$ alkylsulfinyl; $C_1$-$C_{17}$ haloalkoxy; $C_5$-$C_6$ cycloalkyloxy; $C_3$-$C_{17}$ alkenyloxy; $C_3$-$C_{17}$ haloalkynyl; $C_3$-$C_{17}$ alkynyloxy; $C_1$-$C_{17}$ alkylsulfonyl; $C_2$-$C_{17}$ alkoxycarbonyl; hydroxyl; hydroxycarbonyl; $R^{28}C(=O)O$; $R^{28}OC(=O)O$; $R^{28}R^{40}NC(=O)O$; $R^{40}R^{39}N$; $(C_1$-$C_4$ alkoxy$)_2P(=E)O$; $R^{11}SO_3$; $R^{40}R^{14}R^{38}N^+$; phenyl, phenoxy, phenylthio, phenylsulfonyl, phenylsulfinyl, pyridyl or pyridyloxy each optionally substituted with $R^{30}$; thienyl, pyrimidinyl, furanyl, naphthalenyl, pyrimidinyloxy, naphthalenyloxy each optionally substituted with $R^7$; tetrahydropyranyl; 2-tetrahydropyranyloxy; $C_1$-$C_{17}$ alkoxy; 1-3 halogen; $C_2$-$C_{17}$ alkoxyalkoxy; $C_3$-$C_{17}$ alkynyl; or $C_3$-$C_6$ cycloalkyl;

provided that

i) when $R^2$ is a substituted phenyl or heterocyclic ring, then only H or F can be substituted on the carbon atom(s) of the phenyl or heterocyclic ring adjacent to the carbon bearing the oxazolidinone ring;
ii) when $R^3$ is a phenyl or heterocyclic ring disubstituted with two alkyl or alkoxy groups, or one alkyl and one alkoxy group, then at least one of the alkyl and alkoxy groups is methyl or methoxy; and
iii) the total number of carbons in $R^2$, $R^{16}$, $R^{17}$, and $R^{18}$ is each less than or equal to 20.

**9.** A method of controlling disease in plants comprising applying to said plants a fungicidally active compound of Formula I

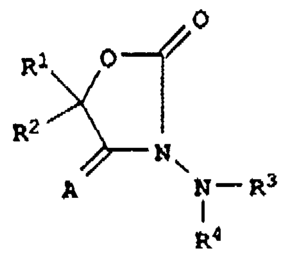

I

wherein:

| | |
|---|---|
| A | is S or N-OG; |
| G | is H; $C_1$-$C_6$ alkyl; benzyl optionally substituted with $R^{34}$ on the phenyl ring; C(=O)($C_1$-$C_4$ alkyl); C(=O)($C_1$-$C_4$ alkoxy); or C(=O)NH$R^{36}$; |
| $R^1$ | is $C_1$-$C_4$ alkyl; $C_1$-$C_4$ haloalkyl; $C_3$-$C_6$ cycloalkyl; $C_2$-$C_4$ alkenyl; $C_2$-$C_4$ alkoxycarbonyl; or phenylmethyl optionally substituted with $R^6$ on the phenyl ring and with $R^8$ on the benzylic carbon; |
| $R^2$ | is $C_1$-$C_6$ alkyl; $C_5$-$C_7$ cycloalkyl; $C_2$-$C_6$ alkenyl; $C_5$-$C_7$ cycloalkenyl; $C_5$-$C_7$ cycloalkenyl; phenyl optionally substituted with $R^5$ and $R^7$; 2-naphthalenyl; thienyl optionally substituted with $R^5$ and $R^7$; furyl optionally substituted with $R^7$; or pyridyl optionally substituted with $R^7$; or phenoxy or phenylthio each optionally substituted with $R^7$; or |
| $R^1$ and $R^2$ | can be taken together to form structures selected from the group consisting of $-CH_2(CH_2)_2CH_2-$, $-CH_2(CH_2)_3CH_2-$, $-CH_2(CH_2)_4CH_2-$, $-CH_2CH_2OCH_2CH_2$, $-CH_2CH_2SCH_2CH_2-$, |

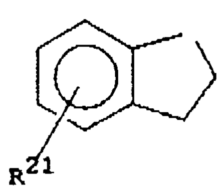 , 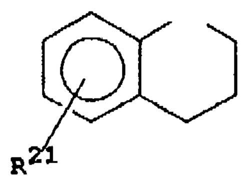 , and 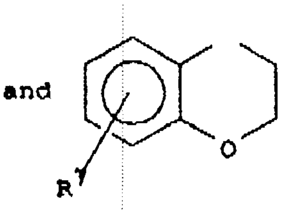 ;

| | |
|---|---|
| $R^3$ | is phenyl, pyridyl, or pyrimidinyl each optionally substituted with $R^{10}$; or phenylmethyl; |
| $R^4$ | is H or methyl; |
| $R^5$ | is halogen; nitro; cyano; $C_1$-$C_6$ alkyl; $C_5$-$C_6$ cycloalkyl; $C_1$-$C_6$ haloalkyl; $C_1$-$C_6$ alkylthio; $C_1$-$C_6$ haloalkylthio; $C_1$-$C_6$ alkoxy; $C_1$-$C_6$ haloalkoxy; $C_5$-$C_6$ cycloalkyloxy; $C_2$-$C_6$ alkoxyalkyl; $C_2$-$C_6$ alkoxyalkoxy; $C_3$-$C_6$ alkenyl; $C_3$-$C_6$ haloalkenyl; $C_3$-$C_6$ alkenyloxy; $C_3$-$C_6$ alkynyl; $C_3$-$C_6$ haloalkynyl; $C_3$-$C_6$ alkynyloxy; $C_1$-$C_6$ alkylsulfonyl; $C_1$-$C_6$ haloalkylsulfonyl; phenyl or phenylthio each optionally substituted with $R^{24}$; phenylmethyl, phenoxymethyl, phenethyl, or styryl each optionally sub- |

stituted with $R^{24}$ on the phenyl ring; phenoxy optionally substituted with $R^{27}$; and $C_2$-$C_6$ alkoxycarbonyl;

$R^6$, $R^7$, $R^{24}$, $R^{26}$ and $R^{34}$ are independently 1-2 halogen; nitro; $C_1$-$C_4$ alkyl; trifluoromethyl; methylthio; or $C_1$-$C_4$ alkoxy;

$R^8$ is H or $C_1$-$C_4$ alkyl;

$R^{10}$ is 1-2 substituents selected from the group consisting of halogen, nitro, cyano, $C_1$-$C_4$ alkyl, trifluoromethyl, $C_1$-$C_4$ alkylthio, $C_1$-$C_4$ alkoxy and trifluoromethoxy;

$R^{21}$ is halogen; nitro; $C_1$-$C_4$ alkyl; trifluoromethyl; methylthio; or $C_1$-$C_4$ alkoxy; or phenoxy optionally substituted with $R^{26}$;

$R^{27}$ is 1-2 halogen; nitro; cyano; $C_1$-$C_6$ alkyl; $C_1$-$C_6$ haloalkyl; $C_1$-$C_6$ alkoxy; $C_1$-$C_6$ haloalkoxy; $C_1$-$C_4$ alkylsulfonyl; $C_2$-$C_6$ alkoxyalkyl; $C_1$-$C_4$ alkylthio; $C_5$-$C_6$ cycloalkyl; $C_5$-$C_6$ cycloalkyloxy; $C_2$-$C_6$ alkenyl; $C_2$-$C_6$ haloalkenyl; $C_2$-$C_6$ alkynyl; $C_2$-$C_4$ alkoxycarbonyl; or phenoxy; and

$R^{36}$ is $C_1$-$C_6$ alkyl; or phenyl optionally substituted with $R^{12}$.

**10.** A method of Claim 8 wherein:

J is H; OH; C(=O)$R^{16}$; C(=O)O$R^{17}$ or C(=0)NH$R^{19}$;

$R^1$ is $C_1$-$C_4$ alkyl; $C_1$-$C_4$ haloalkyl; or vinyl;

$R^2$ is $C_1$-$C_{20}$ alkyl; $C_2$-$C_{20}$ alkoxyalkyl; $C_2$-$C_{20}$ haloalkyl; $C_3$-$C_8$ alkyl substituted with phenoxy or phenylthio each optionally substituted with $R^{30}$; $C_5$-$C_7$ cycloalkyl; $C_2$-$C_{20}$ alkenyl; $C_5$-$C_7$ cycloalkenyl; phenyl optionally substituted with $R^5$ and $R^7$; 2-naphthalenyl; thienyl optionally substituted with $R^5$ and $R^7$; furyl optionally substituted with $R^7$; or pyridyl optionally substituted with $R^5$ and $R^7$;

$R^3$ is phenyl optionally substituted with $R^{10}$;

$R^{16}$ is H; $C_1$-$C_6$ alkyl; or phenyl optionally substituted with $R^{34}$;

$R^{17}$ is $C_1$-$C_6$ alkyl;

$R^{19}$ is $C_1$-$C_6$ alkyl; or phenyl optionally substituted with $R^{34}$;

provided that when $R^2$ is phenyl and $R^5$ is not F, then

$R^5$ is attached to the para-position relative to the oxazolidinone ring.

**11.** The method of Claim 8 wherein:

J in H; OH; or C(=O)$R^{16}$;

$R^1$ is $C_1$-$C_2$ alkyl or vinyl;

$R^2$ is $C_1$-$C_{12}$ alkyl; $C_5$-$C_7$ cycloalkyl; phenyl optionally substituted with $R^5$ and $R^7$; thienyl optionally substituted with $R^7$; or pyridyl optionally substituted with $R^5$ and $R^7$;

$R^3$ is phenyl optionally substituted with F; Cl; or methyl;

$R^4$ is H;

$R^5$ is halogen; nitro; $C_1$-$C_6$ alkyl; $C_1$-$C_3$ haloalkyl; methylthio; $C_1$-$C_6$ alkoxy; $C_1$-$C_2$ haloalkoxy; $C_5$-$C_6$ cycloalkyloxy; phenoxy optionally substituted with $R^{27}$; phenylthio substituted with $R^{24}$; phenoxymethyl optionally substituted with $R^{24}$ on the phenyl ring; benzyloxy optionally substituted with $R^{30}$ on the phenyl ring; -OC(=O)NH$R^{28}$;

$R^7$ and $R^{24}$ are independently F; $C_1$-$C_2$ alkyl; methylthio; or $C_1$-$C_2$ alkoxy;

$R^{16}$ is $C_1$-$C_6$ alkyl;

$R^{19}$ is phenyl optionally substituted with $R^{34}$;

$R^{27}$ is 1-2 halogen; cyano; $C_1$-$C_4$ alkyl; trifluoromethyl; $C_1$-$C_4$ alkoxy; $C_1$-$C_4$ haloalkoxy; $C_1$-$C_4$ alkylthio; $C_5$-$C_6$ cycloalkyloxy; or allyl;

$R^{30}$ is 1-2 halogen, cyano, $C_1$-$C_4$ alkyl, trifluoromethyl, $C_1$-$C_4$ alkoxy or trifluoromethoxy; and

$R^{34}$ is 1-2 halogen; nitro; $C_1$-$C_2$ alkyl; or $C_1$-$C_2$ alkoxy.

**12.** A method of Claim 11 wherein:

J is H;

$R^1$ is methyl;

$R^2$ is $C_1$-$C_{12}$ alkyl; phenyl optionally substituted with $R^5$ and $R^7$; pyridyl optionally substituted with $R^5$ and $R^7$; and

$R^5$ is F; Cl; Br; $C_1$-$C_6$ alkyl; trifluoromethyl; $C_1$-$C_6$ alkoxy; trifluoromethoxy; 2,2,2-trifluoroethoxy;

$C_5$-$C_6$ cycloalkyloxy; methylthio; phenoxy optionally substituted with $R^{27}$; phenylthio optionally substituted with $R^{24}$; benzyloxy optionally substituted with $R^{30}$ on the phenyl ring; or -OC(=O)$R^{28}$.

**13.** A method of Claim 8 wherein said compound is selected from the group consisting of

5-(2,4-Difluorophenyl)-5-methyl-3-(phenylamino)-4-thioxo-2-oxazolidinone;

4-Imino-5-methyl-5-(4-phenoxyphenyl)-3-(phenylamino)-2-oxazolidinone;

4-Imino-5-methyl-5-(4-phenoxyphenyl)-3-(phenylamino)-2-oxazolidinone hydrobromide; and

5-(2,4-Difluorophenyl)-4-imino-5-methyl-3-(phenylamino)-2-oxazolidinone.

**14.** A method for controlling disease in plants comprising applying to said plants a composition comprising a fungicidally active compound of any one of Claims 1-6.

European Patent Office

# EUROPEAN SEARCH REPORT

Application Number

EP 92 30 1597

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| D,X | EP-A-0 393 911 (E.I. DU PONT DE NEMOURS AND COMPANY)<br>* claims *<br>--- | 1-14 | C07D263/46<br>C07D263/48<br>A01N43/76<br>C07F9/653<br>C07D263/52<br>C07D498/10<br>C07D403/04<br>C07D403/12 |
| A | DE-A-3 607 068 (CONSORTIUM FÜR ELEKTROCHEMISCHE INDUSTRIE GMBH)<br>* the whole document *<br>--- | 1-14 | |
| A | US-A-3 671 535 (MICHEL FALDUTTI ET AL)<br>* the whole document *<br>----- | 1-14 | |
| | | | TECHNICAL FIELDS SEARCHED (Int. Cl.5) |
| | | | C07D |

The present search report has been drawn up for all claims

| Place of search | Date of completion of the search | Examiner |
|---|---|---|
| THE HAGUE | 22 MAY 1992 | HENRY J.C. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document